(19) Europäisches Patentamt
European Patent Office
Office européen des brevets

(11) EP 2 611 912 B1

(12) **EUROPEAN PATENT SPECIFICATION**

(45) Date of publication and mention of the grant of the patent:
**02.11.2016 Bulletin 2016/44**

(21) Application number: **11749857.6**

(22) Date of filing: **02.09.2011**

(51) Int Cl.:
***C12N 9/02*** *(2006.01)*

(86) International application number:
**PCT/EP2011/065179**

(87) International publication number:
**WO 2012/028709 (08.03.2012 Gazette 2012/10)**

# (54) NOVEL MONOOXYGENASE VARIANTS

NEUE MONOOXYGENASE-VARIANTEN

NOUVELLES VARIANTES DE MONOOXYGÉNASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.09.2010 EP 10009185**

(43) Date of publication of application:
**10.07.2013 Bulletin 2013/28**

(73) Proprietor: **B.R.A.I.N.
Biotechnology Research and Information Network AG
64673 Zwingenberg (DE)**

(72) Inventors:
- **SCHWANEBERG, Ulrich
B-4728 Kelmis Hergenrath (BE)**
- **BLANUSA, Milan
07745 Jena (DE)**
- **NIEHAUS, Frank
64646 Heppenheim (DE)**
- **ECK, Jürgen
64625 Bensheim (DE)**

(74) Representative: **Vossius & Partner
Patentanwälte Rechtsanwälte mbB
Siebertstrasse 3
81675 München (DE)**

(56) References cited:
**WO-A2-01/07574 WO-A2-03/008563
WO-A2-2006/105082**

- **SCHWANEBERG U ET AL: "A continuous spectrophotometric assay for P450 BM-3, a fatty acid hydroxylating enzyme, and its mutant F87A", ANALYTICAL BIOCHEMISTRY, ACADEMIC PRESS INC, NEW YORK LNKD-DOI:10.1006/ABIO.1999.4047, vol. 269, no. 2, 1 May 1999 (1999-05-01), pages 359-366, XP002156030, ISSN: 0003-2697**
- **NAZOR JOVANA ET AL: "Laboratory evolution of P450BM3 for mediated electron transfer yielding an activity-improved and reductase-independent variant", PROTEIN ENGINEERING DESIGN & SELECTION, vol. 21, no. 1, January 2008 (2008-01), pages 29-35, XP002607558, ISSN: 1741-0126**
- **LI QING-SHAN ET AL: "Rational evolution of a medium chain-specific cytochrome P-450 BM-3 variant", BIOCHIMICA ET BIOPHYSICA ACTA, vol. 1545, no. 1-2, 9 February 2001 (2001-02-09), pages 114-121, XP002607559, ISSN: 0006-3002**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 611 912 B1

SEQUENCE LISTING

<110> B.R.A.I.N. Biotechnology Research and Information Network AG

<120> Novel Monooxygenase Variants

<130> R2728 PCT

<140> EP 10 00 9185.9
<141> 2010-09-03

<160> 18

<170> PatentIn version 3.4

<210> 1
<211> 577
<212> PRT
<213> Artificial sequence

<220>
<223> /note="Description of artificial sequence:P450 BM3 reductase domain"

<400> 1

```
Lys Lys Ala Glu Asn Ala His Asn Thr Pro Leu Leu Val Leu Tyr Gly
1               5                   10                  15

Ser Asn Met Gly Thr Ala Glu Gly Thr Ala Arg Asp Leu Ala Asp Ile
            20                  25                  30

Ala Met Ser Lys Gly Phe Ala Pro Gln Val Ala Thr Leu Asp Ser His
        35                  40                  45

Ala Gly Asn Leu Pro Arg Glu Gly Ala Val Leu Ile Val Thr Ala Ser
    50                  55                  60

Tyr Asn Gly His Pro Pro Asp Asn Ala Lys Gln Phe Val Asp Trp Leu
65                  70                  75                  80

Asp Gln Ala Ser Ala Asp Glu Val Lys Gly Val Arg Tyr Ser Val Phe
                85                  90                  95

Gly Cys Gly Asp Lys Asn Trp Ala Thr Thr Tyr Gln Lys Val Pro Ala
            100                 105                 110

Phe Ile Asp Glu Thr Leu Ala Ala Lys Gly Ala Glu Asn Ile Ala Asp
        115                 120                 125

Arg Gly Glu Ala Asp Ala Ser Asp Asp Phe Glu Gly Thr Tyr Glu Glu
    130                 135                 140

Trp Arg Glu His Met Trp Ser Asp Val Ala Ala Tyr Phe Asn Leu Asp
```

```
145                 150                 155                 160

Ile Glu Asn Ser Glu Asp Asn Lys Ser Thr Leu Ser Leu Gln Phe Val
                165                 170                 175

Asp Ser Ala Ala Asp Met Pro Leu Ala Lys Met His Gly Ala Phe Ser
            180                 185                 190

Thr Asn Val Val Ala Ser Lys Glu Leu Gln Gln Pro Gly Ser Ala Arg
        195                 200                 205

Ser Thr Arg His Leu Glu Ile Glu Leu Pro Lys Glu Ala Ser Tyr Gln
    210                 215                 220

Glu Gly Asp His Leu Gly Val Ile Pro Arg Asn Tyr Glu Gly Ile Val
225                 230                 235                 240

Asn Arg Val Thr Ala Arg Phe Gly Leu Asp Ala Ser Gln Gln Ile Arg
                245                 250                 255

Leu Glu Ala Glu Glu Glu Lys Leu Ala His Leu Pro Leu Ala Lys Thr
            260                 265                 270

Val Ser Val Glu Glu Leu Leu Gln Tyr Val Glu Leu Gln Asp Pro Val
        275                 280                 285

Thr Arg Thr Gln Leu Arg Ala Met Ala Ala Lys Thr Val Cys Pro Pro
    290                 295                 300

His Lys Val Glu Leu Glu Ala Leu Leu Glu Lys Gln Ala Tyr Lys Glu
305                 310                 315                 320

Gln Val Leu Ala Lys Arg Leu Thr Met Leu Glu Leu Leu Glu Lys Tyr
                325                 330                 335

Pro Ala Cys Glu Met Lys Phe Ser Glu Phe Ile Ala Leu Leu Pro Ser
            340                 345                 350

Ile Arg Pro Arg Tyr Tyr Ser Ile Ser Ser Ser Pro Arg Val Asp Glu
        355                 360                 365

Lys Gln Ala Ser Ile Thr Val Ser Val Val Ser Gly Glu Ala Trp Ser
    370                 375                 380

Gly Tyr Gly Glu Tyr Lys Gly Ile Ala Ser Asn Tyr Leu Ala Glu Leu
385                 390                 395                 400
```

```
Gln Glu Gly Asp Thr Ile Thr Cys Phe Ile Ser Thr Pro Gln Ser Glu
                405                 410                 415

Phe Thr Leu Pro Lys Asp Pro Glu Thr Pro Leu Ile Met Val Gly Pro
            420                 425                 430

Gly Thr Gly Val Ala Pro Phe Arg Gly Phe Val Gln Ala Arg Lys Gln
        435                 440                 445

Leu Lys Glu Gln Gly Gln Ser Leu Gly Glu Ala His Leu Tyr Phe Gly
    450                 455                 460

Cys Arg Ser Pro His Glu Asp Tyr Leu Tyr Gln Glu Glu Leu Glu Asn
465                 470                 475                 480

Ala Gln Ser Glu Gly Ile Ile Thr Leu His Thr Ala Phe Ser Arg Met
                485                 490                 495

Pro Asn Gln Pro Lys Thr Tyr Val Gln His Val Met Glu Gln Asp Gly
            500                 505                 510

Lys Lys Leu Ile Glu Leu Leu Asp Gln Gly Ala His Phe Tyr Ile Cys
        515                 520                 525

Gly Asp Gly Ser Gln Met Ala Pro Ala Val Glu Ala Thr Leu Met Lys
    530                 535                 540

Ser Tyr Ala Asp Val His Gln Val Ser Glu Ala Asp Ala Arg Leu Trp
545                 550                 555                 560

Leu Gln Gln Leu Glu Glu Lys Gly Arg Tyr Ala Lys Asp Val Trp Ala
                565                 570                 575

Gly
```

<210> 2
<211> 1048
<212> PRT
<213> Bacillus megaterium

<400> 2

```
Thr Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30
```

```
Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Val Arg Asp
65                  70                  75                  80

Phe Ala Gly Asp Gly Leu Phe Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285
```

```
Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540
```

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met

```
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035
```

Gly Arg Tyr Ala Lys Asp Val Trp Ala Gly
1040 1045

<210> 3
<211> 1731
<212> DNA
<213> Artificial sequence

<220>
<223> /note="Description of artificial sequence:P450 BM3 reductase domain coding sequence"

<400> 3

```
aaaaaggcag aaaacgctca taatacgccg ctgcttgtgc tatacggttc aaatatggga      60
acagctgaag gaacggcgcg tgatttagca gatattgcaa tgagcaaagg atttgcaccg     120
caggtcgcaa cgcttgattc acacgccgga aatcttccgc gcgaaggagc tgtattaatt     180
gtaacggcgt cttataacgg tcatccgcct gataacgcaa agcaatttgt cgactggtta     240
gaccaagcgt ctgctgatga agtaaaaggc gttcgctact ccgtatttgg atgcggcgat     300
aaaaactggg ctactacgta tcaaaaagtg cctgctttta tcgatgaaac gcttgccgct     360
aaaggggcag aaaacatcgc tgaccgcggt gaagcagatg caagcgacga ctttgaaggc     420
acatatgaag aatggcgtga acatatgtgg agtgacgtag cagcctactt taacctcgac     480
attgaaaaca gtgaagataa taaatctact ctttcacttc aatttgtcga cagcgccgcg     540
gatatgccgc ttgcgaaaat gcacggtgcg ttttcaacga acgtcgtagc aagcaaagaa     600
cttcaacagc caggcagtgc acgaagcacg cgacatcttg aaattgaact tccaaaagaa     660
gcttcttatc aagaaggaga tcatttaggt gttattcctc gcaactatga aggaatagta     720
aaccgtgtaa cagcaaggtt cggcctagat gcatcacagc aaatccgtct ggaagcagaa     780
gaagaaaaat tagctcattt gccactcgct aaaacagtat ccgtagaaga gcttctgcaa     840
tacgtggagc ttcaagatcc tgttacgcgc acgcagcttc gcgcaatggc tgctaaaacg     900
gtctgcccgc cgcataaagt agagcttgaa gccttgcttg aaaagcaagc ctacaaagaa     960
caagtgctgg caaaacgttt aacaatgctt gaactgcttg aaaaataccc ggcgtgtgaa    1020
atgaaattca gcgaatttat cgcccttctg ccaagcatac gcccgcgcta ttactcgatt    1080
tcttcatcac ctcgtgtcga tgaaaaacaa gcaagcatca cggtcagcgt tgtctcagga    1140
gaagcgtgga gcggatatgg agaatataaa ggaattgcgt cgaactatct tgccgagctg    1200
caagaaggag atacgattac gtgctttatt tccacaccgc agtcagaatt tacgctgcca    1260
aaagaccctg aaacgccgct tatcatggtc ggaccgggaa caggcgtcgc gccgtttaga    1320
ggctttgtgc aggcgcgcaa acagctaaaa gaacaaggac agtcacttgg agaagcacat    1380
ttatacttcg gctgccgttc acctcatgaa gactatctgt atcaagaaga gcttgaaaac    1440
gcccaaagcg aaggcatcat tacgcttcat accgcttttt ctcgcatgcc aaatcagccg    1500
aaaacatacg ttcagcacgt aatggaacaa gacggcaaga aattgattga acttcttgat    1560
caaggagcgc acttctatat ttgcggagac ggaagccaaa tggcacctgc cgttgaagca    1620
acgcttatga aaagctatgc tgacgttcac caagtgagtg aagcagacgc tcgcttatgg    1680
ctgcagcagc tagaagaaaa aggccgatac gcaaaagacg tgtgggctgg g             1731
```

<210> 4
<211> 1048
<212> PRT

<213> Artificial sequence

<220>
<223> /note="Description of artificial sequence:P450 BM3 variant F87A"

<400> 4

```
Thr Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Val Arg Asp
65                  70                  75                  80

Phe Ala Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175
```

```
Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430
```

Ile Lys Glu Thr Leu Thr Leu Lys Pro Glu Gly Phe Val Val Lys Ala
435 440 445

Lys Ser Lys Lys Ile Pro Leu Gly Gly Ile Pro Ser Pro Ser Thr Glu
450 455 460

Gln Ser Ala Lys Lys Val Cys Lys Lys Ala Glu Asn Ala His Asn Thr
465 470 475 480

Pro Leu Leu Val Leu Tyr Gly Ser Asn Met Gly Thr Ala Glu Gly Thr
485 490 495

Ala Arg Asp Leu Ala Asp Ile Ala Met Ser Lys Gly Phe Ala Pro Gln
500 505 510

Val Ala Thr Leu Asp Ser His Ala Gly Asn Leu Pro Arg Glu Gly Ala
515 520 525

Val Leu Ile Val Thr Ala Ser Tyr Asn Gly His Pro Pro Asp Asn Ala
530 535 540

Lys Gln Phe Val Asp Trp Leu Asp Gln Ala Ser Ala Asp Glu Val Lys
545 550 555 560

Gly Val Arg Tyr Ser Val Phe Gly Cys Gly Asp Lys Asn Trp Ala Thr
565 570 575

Thr Tyr Gln Lys Val Pro Ala Phe Ile Asp Glu Thr Leu Ala Ala Lys
580 585 590

Gly Ala Glu Asn Ile Ala Asp Arg Gly Glu Ala Asp Ala Ser Asp Asp
595 600 605

Phe Glu Gly Thr Tyr Glu Glu Trp Arg Glu His Met Trp Ser Asp Val
610 615 620

Ala Ala Tyr Phe Asn Leu Asp Ile Glu Asn Ser Glu Asp Asn Lys Ser
625 630 635 640

Thr Leu Ser Leu Gln Phe Val Asp Ser Ala Ala Asp Met Pro Leu Ala
645 650 655

Lys Met His Gly Ala Phe Ser Thr Asn Val Val Ala Ser Lys Glu Leu
660 665 670

Gln Gln Pro Gly Ser Ala Arg Ser Thr Arg His Leu Glu Ile Glu Leu

```
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925
```

```
Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045
```

<210> 5
<211> 1048
<212> PRT
<213> Artificial sequence

<220>
<223> /note="Description of artificial sequence:P450 BM3 variant A3"

<400> 5

```
Thr Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Val Arg Asp
65                  70                  75                  80

Phe Ala Gly Asp Gly Leu Ala Thr Ser Trp Thr His Glu Lys Asn Trp
```

85 90 95

Lys Lys Ala His Asn Ile Leu Leu Pro Ser Phe Ser Gln Gln Ala Met
100 105 110

Lys Gly Tyr His Ala Met Met Val Asp Ile Ala Val Gln Leu Val Gln
115 120 125

Lys Trp Glu Arg Leu Asn Ala Asp Glu His Ile Glu Val Pro Glu Asp
130 135 140

Met Thr Arg Leu Thr Leu Asp Thr Ile Gly Leu Cys Gly Phe Asn Tyr
145 150 155 160

Arg Phe Asn Ser Phe Tyr Arg Asp Gln Pro His Pro Phe Ile Thr Ser
165 170 175

Met Val Arg Ala Leu Asp Glu Ala Met Asn Lys Leu Gln Arg Ala Asn
180 185 190

Pro Asp Asp Pro Ala Tyr Asp Glu Asn Lys Arg Gln Phe Gln Glu Asp
195 200 205

Ile Lys Val Met Asn Asp Leu Val Asp Lys Ile Ile Ala Asp Arg Lys
210 215 220

Ala Ser Gly Glu Gln Ser Asp Asp Leu Leu Thr His Met Leu Asn Gly
225 230 235 240

Lys Asp Pro Glu Thr Gly Glu Pro Leu Asp Asp Glu Asn Ile Arg Tyr
245 250 255

Gln Ile Ile Thr Phe Leu Ile Ala Gly His Glu Thr Thr Ser Gly Leu
260 265 270

Leu Ser Phe Ala Leu Tyr Phe Leu Val Lys Asn Pro His Val Leu Gln
275 280 285

Lys Ala Ala Glu Glu Ala Ala Arg Val Leu Val Asp Pro Val Pro Ser
290 295 300

Tyr Lys Gln Val Lys Gln Leu Lys Tyr Val Gly Met Val Leu Asn Glu
305 310 315 320

Ala Leu Arg Leu Trp Pro Thr Ala Pro Ala Phe Ser Leu Tyr Ala Lys
325 330 335

Glu Asp Thr Val Leu Gly Gly Glu Tyr Pro Leu Glu Lys Gly Asp Glu
340 345 350

Leu Met Val Leu Ile Pro Gln Leu His Arg Asp Lys Thr Ile Trp Gly
355 360 365

Asp Asp Val Glu Glu Phe Arg Pro Glu Arg Phe Glu Asn Pro Ser Ala
370 375 380

Ile Pro Gln His Ala Phe Lys Pro Phe Gly Asn Gly Gln Arg Ala Cys
385 390 395 400

Ile Gly Gln Gln Phe Ala Leu His Glu Ala Thr Leu Val Leu Gly Met
405 410 415

Met Leu Lys His Phe Asp Phe Glu Asp His Thr Asn Tyr Glu Leu Asp
420 425 430

Ile Lys Glu Thr Leu Thr Leu Lys Pro Glu Gly Phe Val Val Lys Ala
435 440 445

Lys Ser Lys Lys Ile Pro Leu Gly Gly Ile Pro Ser Pro Ser Thr Glu
450 455 460

Gln Ser Ala Lys Lys Val Cys Lys Lys Ala Glu Asn Ala His Asn Thr
465 470 475 480

Pro Leu Leu Val Leu Tyr Gly Ser Asn Met Gly Thr Ala Glu Gly Thr
485 490 495

Ala Arg Asp Leu Ala Asp Ile Ala Met Ser Lys Gly Phe Ala Pro Gln
500 505 510

Val Ala Thr Leu Asp Ser His Ala Gly Asn Leu Pro Arg Glu Gly Ala
515 520 525

Val Leu Ile Val Thr Ala Ser Tyr Asn Gly His Pro Pro Asp Asn Ala
530 535 540

Lys Gln Phe Val Asp Trp Leu Asp Gln Ala Ser Ala Asp Glu Val Lys
545 550 555 560

Gly Val Arg Tyr Ser Val Phe Gly Cys Gly Asp Lys Asn Trp Ala Thr
565 570 575

Thr Tyr Gln Lys Val Pro Ala Phe Ile Asp Glu Thr Leu Ala Ala Lys
580 585 590

```
Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Thr Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845
```

```
Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045
```

<210> 6
<211> 1048
<212> PRT
<213> Artificial sequence

<220>
<223> /note="Description of artificial sequence:P450 BM3 variant Dm1"

<400> 6

```
Thr Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Phe Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Val Arg Asp
65                  70                  75                  80

Phe Ala Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255
```

```
Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Cys Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
```

500 505 510

Val Ala Thr Leu Asp Ser His Ala Gly Asn Leu Pro Arg Glu Gly Ala
515 520 525

Val Leu Ile Val Thr Ala Ser Tyr Asn Gly His Pro Pro Asp Asn Ala
530 535 540

Lys Gln Phe Val Asp Trp Leu Asp Gln Ala Ser Ala Asp Glu Val Lys
545 550 555 560

Gly Val Arg Tyr Ser Val Phe Gly Cys Gly Asp Lys Asn Trp Ala Thr
565 570 575

Thr Tyr Gln Lys Val Pro Ala Phe Ile Asp Glu Thr Leu Ala Ala Lys
580 585 590

Gly Ala Glu Asn Ile Ala Asp Arg Gly Glu Ala Asp Ala Ser Asp Asp
595 600 605

Phe Glu Gly Thr Tyr Glu Glu Trp Arg Glu His Met Trp Ser Asp Val
610 615 620

Ala Ala Tyr Phe Asn Leu Asp Ile Glu Asn Ser Glu Asp Asn Lys Ser
625 630 635 640

Thr Leu Ser Leu Gln Phe Val Asp Ser Ala Ala Asp Met Pro Leu Ala
645 650 655

Lys Met His Gly Ala Phe Ser Thr Asn Val Val Ala Ser Lys Glu Leu
660 665 670

Gln Gln Pro Gly Ser Ala Arg Ser Thr Arg His Leu Glu Ile Glu Leu
675 680 685

Pro Lys Glu Ala Ser Tyr Gln Glu Gly Asp His Leu Gly Val Ile Pro
690 695 700

Arg Asn Tyr Glu Gly Ile Val Asn Arg Val Thr Ala Arg Phe Gly Leu
705 710 715 720

Asp Ala Ser Gln Gln Ile Arg Leu Glu Ala Glu Glu Glu Lys Leu Ala
725 730 735

His Leu Pro Leu Ala Lys Thr Val Ser Val Glu Glu Leu Leu Gln Tyr
740 745 750

```
Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005
```

Val Glu Ala Thr Leu Met Lys Ser Tyr Ala Asp Val His Gln Val
1010 1015 1020

Ser Glu Ala Asp Ala Arg Leu Trp Leu Gln Gln Leu Glu Glu Lys
1025 1030 1035

Gly Arg Tyr Ala Lys Asp Val Trp Ala Gly
1040 1045

<210> 7
<211> 1048
<212> PRT
<213> Artificial sequence

<220>
<223> /note="Description of artificial sequence:P450 BM3 variant E1"

<400> 7

Thr Ile Lys Glu Met Pro Gln Pro Lys Thr Phe Gly Glu Leu Lys Asn
1 5 10 15

Leu Pro Leu Leu Asn Thr Asp Lys Pro Val Gln Ala Leu Met Lys Ile
20 25 30

Ala Asp Glu Leu Gly Glu Ile Phe Lys Phe Glu Ala Pro Gly Phe Val
35 40 45

Thr Arg Tyr Leu Ser Ser Gln Arg Leu Ile Lys Glu Ala Cys Asp Glu
50 55 60

Ser Arg Phe Asp Lys Asn Leu Ser Gln Ala Leu Lys Phe Val Arg Asp
65 70 75 80

Phe Ala Gly Asp Gly Leu Ala Thr Ser Trp Thr His Glu Lys Asn Trp
85 90 95

Lys Lys Ala His Asn Ile Leu Leu Pro Ser Phe Ser Gln Gln Ala Met
100 105 110

Lys Gly Tyr His Ala Met Met Val Asp Ile Ala Val Gln Leu Val Gln
115 120 125

Lys Trp Glu Arg Leu Asn Ala Asp Glu His Ile Glu Val Pro Glu Asp
130 135 140

Met Thr Arg Leu Thr Leu Asp Thr Ile Gly Leu Cys Gly Phe Asn Tyr
145 150 155 160

```
Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415
```

Met Leu Lys His Phe Asp Phe Glu Asp His Thr Asn Tyr Glu Leu Asp
420 425 430

Ile Lys Glu Thr Leu Thr Leu Lys Pro Glu Gly Phe Val Val Lys Ala
435 440 445

Lys Ser Lys Lys Ile Pro Leu Gly Gly Ile Pro Ser Pro Ser Thr Glu
450 455 460

Gln Ser Ala Lys Lys Val Cys Lys Lys Ala Glu Asn Ala His Asn Thr
465 470 475 480

Pro Leu Leu Val Leu Tyr Gly Ser Asn Met Gly Thr Ala Glu Gly Thr
485 490 495

Ala Arg Asp Leu Ala Asp Ile Ala Met Ser Lys Gly Phe Ala Pro Gln
500 505 510

Val Ala Thr Leu Asp Ser His Ala Gly Asn Leu Pro Arg Glu Gly Ala
515 520 525

Val Leu Ile Val Thr Ala Ser Tyr Asn Gly His Pro Pro Asp Asn Ala
530 535 540

Lys Gln Phe Val Asp Trp Leu Asp Gln Ala Ser Ala Asp Glu Val Lys
545 550 555 560

Gly Val Arg Tyr Ser Val Phe Gly Cys Gly Asp Lys Asn Trp Ala Thr
565 570 575

Thr Tyr Gln Lys Val Pro Ala Phe Ile Asp Glu Thr Leu Ala Ala Lys
580 585 590

Gly Ala Glu Asn Ile Ala Asp Arg Gly Glu Ala Asp Ala Ser Asp Asp
595 600 605

Phe Glu Gly Thr Tyr Glu Glu Trp Arg Glu His Met Trp Ser Asp Val
610 615 620

Ala Ala Tyr Phe Asn Leu Asp Ile Glu Asn Ser Glu Asp Asn Lys Ser
625 630 635 640

Thr Leu Ser Leu Gln Phe Val Asp Ser Ala Ala Asp Met Pro Leu Ala
645 650 655

Lys Met His Gly Ala Phe Ser Thr Asn Val Val Ala Ser Lys Glu Leu
660 665 670

```
Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Val Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
```

915 920 925

Glu Ala His Leu Tyr Phe Gly Cys Arg Ser Pro His Glu Asp Tyr Leu
930 935 940

Tyr Gln Glu Glu Leu Glu Asn Ala Gln Ser Glu Gly Ile Ile Thr Leu
945 950 955 960

His Thr Ala Phe Ser Arg Met Pro Asn Gln Pro Lys Thr Tyr Val Gln
965 970 975

His Val Met Glu Gln Asp Gly Lys Lys Leu Ile Glu Leu Leu Asp Gln
980 985 990

Gly Ala His Phe Tyr Ile Cys Gly Asp Gly Ser Gln Met Ala Pro Ala
995 1000 1005

Val Glu Ala Thr Leu Met Lys Ser Tyr Ala Asp Val His Gln Val
1010 1015 1020

Ser Glu Ala Asp Ala Arg Leu Trp Leu Gln Gln Leu Glu Glu Lys
1025 1030 1035

Gly Arg Tyr Ala Lys Asp Val Trp Ala Gly
1040 1045

<210> 8
<211> 1048
<212> PRT
<213> Artificial sequence

<220>
<223> /note="Description of artificial sequence:P450 BM3 variant E9"

<400> 8

```
Thr Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Phe Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Val Arg Asp
65                  70                  75                  80
```

Phe Ala Gly Asp Gly Leu Ala Thr Ser Trp Thr His Glu Lys Asn Trp
85 90 95

Lys Lys Ala His Asn Ile Leu Leu Pro Ser Phe Ser Gln Gln Ala Met
100 105 110

Lys Gly Tyr His Ala Met Met Val Asp Ile Ala Val Gln Leu Val Gln
115 120 125

Lys Trp Glu Arg Leu Asn Ala Asp Glu His Ile Glu Val Pro Glu Asp
130 135 140

Met Thr Arg Leu Thr Leu Asp Thr Ile Gly Leu Cys Gly Phe Asn Tyr
145 150 155 160

Arg Phe Asn Ser Phe Tyr Arg Asp Gln Pro His Pro Phe Ile Thr Ser
165 170 175

Met Val Arg Ala Leu Asp Glu Ala Met Asn Lys Leu Gln Arg Ala Asn
180 185 190

Pro Asp Asp Pro Ala Tyr Asp Glu Asn Lys Arg Gln Phe Gln Glu Asp
195 200 205

Ile Lys Val Met Asn Asp Leu Val Asp Lys Ile Ile Ala Asp Arg Lys
210 215 220

Ala Ser Gly Glu Gln Ser Asp Asp Leu Leu Thr His Met Leu Asn Gly
225 230 235 240

Lys Asp Pro Glu Thr Gly Glu Pro Leu Asp Asp Glu Asn Ile Arg Tyr
245 250 255

Gln Ile Ile Thr Phe Leu Ile Ala Gly His Glu Thr Thr Ser Gly Leu
260 265 270

Leu Ser Phe Ala Leu Tyr Phe Leu Val Lys Asn Pro His Val Leu Gln
275 280 285

Lys Ala Ala Glu Glu Ala Ala Arg Val Leu Val Asp Pro Val Pro Ser
290 295 300

Tyr Lys Gln Val Lys Gln Leu Lys Tyr Val Gly Met Val Leu Asn Glu
305 310 315 320

Ala Leu Arg Leu Trp Pro Thr Ala Pro Ala Phe Ser Leu Tyr Ala Lys

325 330 335

Glu Asp Thr Val Leu Gly Gly Glu Tyr Pro Leu Glu Lys Gly Asp Glu
340 345 350

Leu Met Val Leu Ile Pro Gln Leu His Arg Asp Lys Thr Ile Trp Gly
355 360 365

Asp Asp Val Glu Glu Phe Arg Pro Glu Arg Phe Glu Asn Pro Ser Ala
370 375 380

Ile Pro Gln His Ala Phe Lys Pro Phe Gly Asn Gly Gln Arg Ala Cys
385 390 395 400

Ile Gly Gln Gln Phe Ala Leu His Glu Ala Thr Leu Val Leu Gly Met
405 410 415

Met Leu Lys His Phe Asp Phe Glu Asp His Thr Asn Tyr Glu Leu Asp
420 425 430

Ile Lys Glu Thr Leu Thr Leu Lys Pro Glu Gly Phe Val Val Lys Ala
435 440 445

Lys Ser Lys Lys Ile Pro Leu Gly Gly Ile Pro Ser Pro Ser Thr Glu
450 455 460

Gln Ser Ala Lys Lys Val Cys Lys Lys Ala Glu Asn Ala His Asn Thr
465 470 475 480

Pro Leu Leu Val Leu Tyr Gly Ser Asn Met Gly Thr Ala Glu Gly Thr
485 490 495

Ala Arg Asp Leu Ala Asp Ile Ala Met Ser Lys Gly Phe Ala Pro Gln
500 505 510

Val Ala Thr Leu Asp Ser His Ala Gly Asn Leu Pro Arg Glu Gly Ala
515 520 525

Val Leu Ile Val Thr Ala Ser Tyr Asn Gly His Pro Pro Asp Asn Ala
530 535 540

Lys Gln Phe Val Asp Trp Leu Asp Gln Ala Ser Ala Asp Glu Val Lys
545 550 555 560

Gly Val Arg Tyr Ser Val Phe Gly Cys Gly Asp Lys Asn Trp Ala Thr
565 570 575

Thr Tyr Gln Lys Val Pro Ala Phe Ile Asp Glu Thr Leu Ala Ala Lys
580 585 590

Gly Ala Glu Asn Ile Ala Asp Arg Gly Glu Ala Asp Ala Ser Asp Asp
595 600 605

Phe Glu Gly Thr Tyr Glu Glu Trp Arg Glu His Met Trp Ser Asp Val
610 615 620

Ala Ala Tyr Phe Asn Leu Asp Ile Glu Asn Ser Glu Asp Asn Lys Ser
625 630 635 640

Thr Leu Ser Leu Gln Phe Val Gly Ser Ala Ala Asp Met Pro Leu Ala
645 650 655

Lys Met His Gly Ala Phe Ser Thr Asn Val Val Ala Ser Lys Glu Leu
660 665 670

Gln Gln Pro Gly Ser Ala Arg Ser Thr Arg His Leu Glu Ile Glu Leu
675 680 685

Pro Lys Glu Ala Ser Tyr Gln Glu Gly Asp His Leu Gly Val Ile Pro
690 695 700

Arg Asn Tyr Glu Gly Ile Val Asn Arg Val Thr Ala Arg Phe Gly Leu
705 710 715 720

Asp Ala Ser Gln Gln Ile Arg Leu Glu Ala Glu Glu Glu Lys Leu Ala
725 730 735

His Leu Pro Leu Ala Lys Thr Val Ser Val Glu Glu Leu Leu Gln Tyr
740 745 750

Val Glu Leu Gln Asp Pro Val Thr Arg Thr Gln Leu Arg Ala Met Ala
755 760 765

Ala Lys Thr Val Cys Pro Pro His Lys Val Glu Leu Glu Ala Leu Leu
770 775 780

Glu Lys Gln Ala Tyr Lys Glu Gln Val Leu Ala Lys Arg Leu Thr Met
785 790 795 800

Leu Glu Leu Leu Glu Lys Tyr Pro Ala Cys Glu Met Lys Phe Ser Glu
805 810 815

Phe Ile Ala Leu Leu Pro Ser Ile Arg Pro Arg Tyr Tyr Ser Ile Ser
820 825 830

```
Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                 1015                1020

Ser Glu  Ala Asp Ala Arg Leu  Trp Leu Gln Gln Leu  Glu Glu Lys
    1025                 1030                1035

Gly Arg  Tyr Ala Lys Asp Val  Trp Ala Gly
    1040                 1045
```

<210> 9
<211> 1048
<212> PRT
<213> Artificial sequence

<220>

<223> /note="Description of artificial sequence:P450 BM3 variant H3"

<400> 9

```
Thr Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Phe Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Val Arg Asp
65                  70                  75                  80

Phe Ala Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240
```

```
Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Cys Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495
```

```
Ala Arg Asp Leu Ala Asp Ile Ala Met Ser Lys Gly Phe Ala Pro Arg
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
```

740 745 750

Val Glu Leu Gln Asp Pro Val Thr Arg Thr Gln Leu Arg Ala Met Ala
755 760 765

Ala Lys Thr Val Cys Pro Pro His Lys Val Glu Leu Glu Ala Leu Leu
770 775 780

Glu Lys Gln Ala Tyr Lys Glu Gln Val Leu Ala Lys Arg Leu Thr Met
785 790 795 800

Leu Glu Leu Leu Glu Lys Tyr Pro Ala Cys Glu Met Lys Phe Ser Glu
805 810 815

Phe Ile Ala Leu Leu Pro Ser Ile Arg Pro Arg Tyr Tyr Ser Ile Ser
820 825 830

Ser Ser Pro Arg Val Asp Glu Lys Gln Ala Ser Ile Thr Val Ser Val
835 840 845

Val Ser Gly Glu Ala Trp Ser Gly Tyr Gly Glu Tyr Lys Gly Ile Ala
850 855 860

Ser Asn Tyr Leu Ala Glu Leu Gln Glu Gly Asp Thr Ile Thr Cys Phe
865 870 875 880

Ile Ser Thr Pro Gln Ser Glu Phe Thr Leu Pro Lys Asp Pro Glu Thr
885 890 895

Pro Leu Ile Met Val Gly Pro Gly Thr Gly Val Ala Pro Phe Arg Gly
900 905 910

Phe Val Gln Ala Arg Lys Gln Leu Lys Glu Gln Gly Gln Ser Leu Gly
915 920 925

Glu Ala His Leu Tyr Phe Gly Cys Arg Ser Pro His Glu Asp Tyr Leu
930 935 940

Tyr Gln Glu Glu Leu Glu Asn Ala Gln Ser Glu Gly Ile Ile Thr Leu
945 950 955 960

His Thr Ala Phe Ser Arg Met Pro Asn Gln Pro Lys Thr Tyr Val Gln
965 970 975

His Val Met Glu Gln Asp Gly Lys Lys Leu Ile Glu Leu Leu Asp Gln
980 985 990

```
Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045
```

<210> 10
<211> 1048
<212> PRT
<213> Artificial sequence

<220>
<223> /note="Description of artificial sequence:P450 BM3 variant M1"

<400> 10

```
Thr Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Ser Met Lys Ile
            20                  25                  30

Ala Asp Glu Leu Gly Glu Ile Phe Lys Phe Glu Ala Pro Gly Tyr Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Val Arg Asp
65                  70                  75                  80

Phe Ala Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
```

```
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Arg Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400
```

```
Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Cys Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Arg
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655
```

```
Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Asn Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910
```

Phe Val Gln Ala Arg Lys Gln Leu Lys Glu Gln Gly Gln Ser Leu Gly
915 920 925

Glu Ala His Leu Tyr Phe Gly Cys Arg Ser Pro His Glu Asp Tyr Leu
930 935 940

Tyr Gln Glu Glu Leu Glu Asn Ala Gln Ser Glu Gly Ile Ile Thr Leu
945 950 955 960

His Thr Ala Phe Ser Arg Met Pro Asn Gln Pro Lys Thr Tyr Val Gln
965 970 975

His Val Met Glu Gln Asp Gly Lys Lys Leu Ile Glu Leu Leu Asp Gln
980 985 990

Gly Ala His Phe Tyr Ile Cys Gly Asp Gly Ser Gln Met Ala Pro Ala
995 1000 1005

Val Glu Ala Thr Leu Met Lys Ser Tyr Ala Asp Val His Gln Val
1010 1015 1020

Ser Glu Ala Asp Ala Arg Leu Trp Leu Gln Gln Leu Glu Glu Lys
1025 1030 1035

Gly Arg Tyr Ala Lys Asp Val Trp Ala Gly
1040 1045

<210> 11
<211> 1048
<212> PRT
<213> Artificial sequence

<220>
<223> /note="Description of artificial sequence:P450 BM3 variant M2"

<400> 11

Thr Ile Lys Glu Met Pro Gln Pro Lys Thr Phe Gly Gly Leu Lys Asn
1 5 10 15

Leu Pro Leu Leu Asn Thr Asp Lys Pro Val Gln Ala Leu Met Lys Ile
20 25 30

Ala Asp Glu Leu Gly Glu Ile Phe Lys Phe Glu Ala Pro Gly Leu Val
35 40 45

Thr Arg Tyr Leu Ser Ser Gln Arg Leu Ile Lys Glu Ala Cys Asp Glu
50 55 60

```
Ser Arg Phe Asp Lys Asn Leu Ser Gln Ala Leu Lys Phe Val Arg Asp
65                  70                  75                  80

Phe Ala Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320
```

Ala Leu Arg Leu Trp Pro Thr Ala Pro Ala Phe Ser Leu Tyr Ala Lys
325 330 335

Glu Asp Thr Val Leu Gly Gly Glu Tyr Pro Leu Glu Lys Gly Asp Glu
340 345 350

Leu Met Val Leu Ile Pro Gln Leu His Arg Asp Lys Thr Ile Trp Gly
355 360 365

Asp Asp Val Glu Glu Phe Arg Pro Glu Arg Phe Glu Asn Pro Ser Ala
370 375 380

Ile Pro Gln His Ala Phe Lys Pro Phe Gly Asn Gly Gln Arg Ala Cys
385 390 395 400

Ile Gly Gln Gln Phe Ala Leu His Glu Ala Thr Leu Val Leu Gly Met
405 410 415

Met Leu Lys His Phe Asp Phe Glu Asp His Thr Asn Tyr Glu Leu Asp
420 425 430

Ile Lys Glu Thr Leu Thr Leu Lys Pro Glu Gly Phe Val Val Lys Ala
435 440 445

Lys Ser Lys Lys Ile Pro Leu Gly Gly Ile Pro Ser Pro Ser Thr Glu
450 455 460

Gln Ser Ala Lys Lys Val Cys Lys Lys Ala Glu Asn Ala His Asn Thr
465 470 475 480

Pro Leu Leu Val Leu Tyr Gly Ser Asn Met Gly Thr Ala Glu Gly Thr
485 490 495

Ala Arg Asp Leu Ala Asp Ile Ala Met Ser Lys Gly Phe Ala Pro Gln
500 505 510

Val Ala Thr Leu Asp Ser His Ala Gly Asn Leu Pro Arg Glu Gly Ala
515 520 525

Val Leu Ile Val Thr Ala Ser Tyr Asn Gly His Pro Pro Asp Asn Ala
530 535 540

Lys Gln Phe Val Asp Trp Leu Asp Gln Ala Ser Ala Asp Glu Val Lys
545 550 555 560

Gly Val Arg Tyr Ser Val Phe Gly Cys Gly Asp Lys Asn Trp Ala Thr

565 570 575

Thr Tyr Gln Lys Val Pro Ala Phe Ile Asp Glu Thr Leu Ala Ala Lys
580 585 590

Gly Ala Glu Asn Ile Ala Asp Arg Gly Glu Ala Asp Ala Ser Asp Asp
595 600 605

Phe Glu Gly Thr Tyr Glu Glu Trp Arg Glu His Met Trp Ser Asp Val
610 615 620

Ala Ala Tyr Phe Asn Leu Asp Ile Glu Asn Ser Glu Asp Asn Lys Ser
625 630 635 640

Thr Leu Ser Leu Gln Phe Val Asp Ser Ala Ala Asp Met Pro Leu Ala
645 650 655

Lys Met His Gly Ala Phe Ser Thr Asn Val Val Ala Ser Lys Glu Leu
660 665 670

Gln Gln Pro Gly Ser Ala Arg Ser Thr Arg His Leu Glu Ile Glu Leu
675 680 685

Pro Lys Glu Ala Ser Tyr Gln Glu Gly Asp His Leu Gly Val Ile Pro
690 695 700

Arg Asn Tyr Glu Gly Ile Val Asn Arg Val Thr Ala Arg Phe Gly Leu
705 710 715 720

Asp Ala Ser Gln Gln Ile Arg Leu Glu Ala Glu Glu Glu Lys Leu Ala
725 730 735

His Leu Pro Leu Ala Lys Thr Val Ser Val Glu Glu Leu Leu Gln Tyr
740 745 750

Val Glu Leu Gln Asp Pro Val Thr Arg Thr Gln Leu Arg Ala Met Ala
755 760 765

Ala Lys Thr Val Cys Pro Pro His Lys Val Glu Leu Glu Ala Leu Leu
770 775 780

Glu Lys Gln Ala Tyr Lys Glu Gln Val Leu Ala Lys Arg Leu Thr Met
785 790 795 800

Leu Glu Leu Leu Glu Lys Tyr Pro Ala Cys Glu Met Lys Phe Ser Glu
805 810 815

```
Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Ser  Trp Leu Gln Gln Leu  Glu Glu Lys
    1025                1030                1035

Gly Arg  Tyr Ala Lys Asp Val  Trp Ala Gly
    1040                1045
```

<210> 12
<211> 1048

<212> PRT
<213> Artificial sequence

<220>
<223> /note="Description of artificial sequence:P450 BM3 variant Dm2#4"

<400> 12

```
Thr Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Val Arg Asp
65                  70                  75                  80

Phe Ala Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220
```

Ala Ser Gly Glu Gln Ser Asp Asp Leu Leu Thr His Met Leu Asn Gly
225 230 235 240

Lys Asp Pro Glu Thr Gly Glu Pro Leu Asp Asp Glu Asn Ile Arg Tyr
245 250 255

Gln Ile Ile Thr Phe Leu Ile Ala Gly His Glu Thr Thr Ser Gly Leu
260 265 270

Leu Ser Phe Ala Leu Tyr Phe Leu Val Lys Asn Pro His Val Leu Gln
275 280 285

Lys Ala Ala Glu Glu Ala Ala Arg Val Leu Val Asp Pro Val Pro Ser
290 295 300

Tyr Lys Gln Val Lys Gln Leu Lys Tyr Val Gly Met Val Leu Asn Glu
305 310 315 320

Ala Leu Arg Leu Trp Pro Thr Ala Pro Ala Phe Ser Leu Tyr Ala Lys
325 330 335

Glu Asp Thr Val Leu Gly Gly Glu Tyr Pro Leu Glu Lys Gly Asp Glu
340 345 350

Leu Ser Val Leu Ile Pro Gln Leu His Arg Asp Lys Thr Ile Trp Gly
355 360 365

Asp Asp Val Glu Glu Phe Arg Pro Glu Arg Phe Glu Asn Pro Ser Ala
370 375 380

Ile Pro Gln His Ala Phe Lys Pro Phe Gly Asn Gly Gln Arg Ala Cys
385 390 395 400

Ile Gly Gln Gln Phe Ala Leu His Glu Ala Thr Leu Val Leu Gly Met
405 410 415

Met Leu Lys His Phe Asp Phe Glu Asp His Thr Asn Tyr Glu Leu Asp
420 425 430

Ile Lys Glu Thr Leu Thr Leu Lys Pro Glu Gly Phe Val Val Lys Ala
435 440 445

Lys Ser Lys Lys Ile Pro Leu Gly Gly Ile Pro Ser Pro Ser Thr Glu
450 455 460

Gln Ser Ala Lys Lys Val Cys Lys Lys Ala Glu Asn Ala His Asn Thr
465 470 475 480

```
Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735
```

His Leu Pro Leu Ala Lys Thr Val Ser Val Glu Glu Leu Leu Gln Tyr
740 745 750

Val Glu Leu Gln Asp Pro Val Thr Arg Thr Gln Leu Arg Ala Met Ala
755 760 765

Ala Lys Thr Val Cys Pro Pro His Lys Val Glu Leu Glu Ala Leu Leu
770 775 780

Glu Lys Gln Ala Tyr Lys Glu Gln Val Leu Ala Lys Arg Leu Thr Met
785 790 795 800

Leu Glu Leu Leu Glu Lys Tyr Pro Ala Cys Glu Met Lys Phe Ser Glu
805 810 815

Phe Ile Ala Leu Leu Pro Ser Ile Arg Pro Arg Tyr Tyr Ser Ile Ser
820 825 830

Ser Ser Pro Arg Val Asp Glu Lys Gln Ala Ser Ile Thr Val Ser Val
835 840 845

Val Ser Gly Glu Ala Trp Ser Gly Tyr Gly Glu Tyr Lys Gly Ile Ala
850 855 860

Ser Asn Tyr Leu Ala Glu Leu Gln Glu Gly Asp Thr Ile Thr Cys Phe
865 870 875 880

Ile Ser Thr Pro Gln Ser Glu Phe Thr Leu Pro Lys Asp Pro Glu Thr
885 890 895

Pro Leu Ile Met Val Gly Pro Gly Thr Gly Val Ala Pro Phe Arg Gly
900 905 910

Phe Val Gln Ala Arg Lys Gln Leu Lys Glu Gln Gly Gln Ser Leu Gly
915 920 925

Glu Ala His Leu Tyr Phe Gly Cys Arg Ser Pro His Glu Asp Tyr Leu
930 935 940

Tyr Gln Glu Glu Leu Glu Asn Ala Gln Ser Glu Gly Ile Ile Thr Leu
945 950 955 960

His Thr Ala Phe Ser Arg Met Pro Asn Gln Pro Lys Thr Tyr Val Gln
965 970 975

His Val Met Glu Gln Asp Gly Lys Lys Leu Ile Glu Leu Leu Asp Gln

```
980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045
```

<210> 13
<211> 1048
<212> PRT
<213> Artificial sequence

<220>
<223> /note="Description of artificial sequence:P450 BM3 variant D7"

<400> 13

```
Thr Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Val Arg Asp
65                  70                  75                  80

Phe Ala Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140
```

```
Met Thr Arg Leu Thr Leu Asp Thr Ile Gly Leu Cys Gly Phe Asn Tyr
145                 150                 155                 160

Arg Leu Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Ser Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
```

385 390 395 400

Ile Gly Gln Gln Phe Ala Leu His Glu Ala Thr Leu Val Leu Gly Met
405 410 415

Met Leu Lys His Phe Asp Phe Glu Asp His Thr Asn Cys Glu Leu Asp
420 425 430

Ile Lys Glu Thr Leu Thr Leu Lys Pro Glu Gly Phe Val Val Lys Ala
435 440 445

Lys Ser Lys Lys Ile Pro Leu Gly Gly Ile Pro Ser Pro Ser Thr Glu
450 455 460

Gln Ser Ala Lys Lys Val Cys Lys Lys Ala Glu Asn Ala His Asn Thr
465 470 475 480

Pro Leu Leu Val Leu Tyr Gly Ser Asn Met Gly Thr Ala Glu Gly Thr
485 490 495

Ala Arg Asp Leu Ala Asp Ile Ala Met Ser Lys Gly Phe Ala Pro Gln
500 505 510

Val Ala Thr Leu Asp Ser His Ala Gly Asn Leu Pro Arg Glu Gly Ala
515 520 525

Val Leu Ile Val Thr Ala Ser Tyr Asn Gly His Pro Pro Asp Asn Ala
530 535 540

Lys Gln Phe Val Asp Trp Leu Asp Gln Ala Ser Ala Asp Glu Val Lys
545 550 555 560

Gly Val Arg Tyr Ser Val Phe Gly Cys Gly Asp Lys Asn Trp Ala Thr
565 570 575

Thr Tyr Gln Lys Val Pro Ala Phe Ile Asp Glu Thr Leu Ala Ala Lys
580 585 590

Gly Ala Glu Asn Ile Ala Asp Arg Gly Glu Ala Asp Ala Ser Asp Asp
595 600 605

Phe Glu Gly Thr Tyr Glu Glu Trp Arg Glu His Met Trp Ser Asp Val
610 615 620

Ala Ala Tyr Phe Asn Leu Asp Ile Glu Asn Ser Glu Asp Asn Lys Ser
625 630 635 640

```
Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895
```

```
Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045
```

<210> 14
<211> 1048
<212> PRT
<213> Artificial sequence

<220>
<223> /note="Description of artificial sequence:P450 BM3 variant E5"

<400> 14

```
Thr Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45
```

```
Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Val Arg Asp
65                  70                  75                  80

Phe Ala Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Leu Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300
```

```
Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Ser Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Cys Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560
```

```
Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Ser Thr Met
785                 790                 795                 800

Leu Glu Leu Leu Glu Lys Tyr Pro Ala Cys Glu Met Lys Phe Ser Glu
```

805 810 815

Phe Ile Ala Leu Leu Pro Ser Ile Arg Pro Arg Tyr Tyr Ser Ile Ser
820 825 830

Ser Ser Pro Arg Val Asp Glu Lys Gln Ala Ser Ile Thr Val Ser Val
835 840 845

Val Ser Gly Glu Ala Trp Ser Gly Tyr Gly Glu Tyr Lys Gly Ile Ala
850 855 860

Ser Asn Tyr Leu Ala Glu Leu Gln Glu Gly Asp Thr Ile Thr Cys Phe
865 870 875 880

Ile Ser Thr Pro Gln Ser Glu Phe Thr Leu Pro Lys Asp Pro Glu Thr
885 890 895

Pro Leu Ile Met Val Gly Pro Gly Thr Gly Val Ala Pro Phe Arg Gly
900 905 910

Phe Val Gln Ala Arg Lys Gln Leu Lys Glu Gln Gly Gln Ser Leu Gly
915 920 925

Glu Ala His Leu Tyr Phe Gly Cys Arg Ser Pro His Glu Asp Tyr Leu
930 935 940

Tyr Gln Glu Glu Leu Glu Asn Ala Gln Ser Glu Gly Ile Ile Thr Leu
945 950 955 960

His Thr Ala Phe Ser Arg Met Pro Asn Gln Pro Lys Thr Tyr Val Gln
965 970 975

His Val Met Glu Gln Asp Gly Lys Lys Leu Ile Glu Leu Leu Asp Gln
980 985 990

Gly Ala His Phe Tyr Ile Cys Gly Asp Gly Ser Gln Met Ala Pro Ala
995 1000 1005

Val Glu Ala Thr Leu Met Lys Ser Tyr Ala Asp Val His Gln Val
1010 1015 1020

Ser Glu Ala Asp Ala Arg Leu Trp Leu Gln Gln Leu Glu Glu Lys
1025 1030 1035

Gly Arg Tyr Ala Lys Asp Val Trp Ala Gly
1040 1045

<210> 15
<211> 1048
<212> PRT
<213> Artificial sequence

<220>
<223> /note="Description of artificial sequence:P450 BM3 variant M3"

<400> 15

Thr Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Gly
    50                  55                  60

Ser Arg Phe Asp Lys Asn Leu Ser Gln Ala Leu Lys Phe Val Arg Asp
65                  70                  75                  80

Phe Ala Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp His Lys

210 215 220

Ala Ser Gly Glu Gln Ser Asp Asp Leu Leu Thr His Met Leu Asn Gly
225 230 235 240

Lys Asp Pro Glu Thr Gly Glu Pro Leu Asp Asp Glu Asn Ile Arg Tyr
245 250 255

Gln Ile Ile Thr Phe Leu Ile Ala Gly His Glu Thr Thr Ser Gly Leu
260 265 270

Leu Ser Phe Ala Leu Tyr Phe Leu Val Lys Asn Pro His Val Leu Gln
275 280 285

Lys Ala Ala Glu Glu Ala Ala Arg Val Leu Val Asp Pro Val Pro Ser
290 295 300

Tyr Lys Gln Val Lys Gln Leu Lys Tyr Val Gly Met Val Leu Asn Glu
305 310 315 320

Ala Leu Arg Leu Trp Pro Thr Ala Pro Ala Phe Ser Leu Tyr Ala Lys
325 330 335

Glu Asp Thr Val Leu Gly Gly Glu Tyr Pro Leu Glu Lys Gly Asp Glu
340 345 350

Leu Ser Val Leu Ile Pro Gln Leu His Arg Asp Lys Thr Ile Trp Gly
355 360 365

Asp Asp Val Glu Glu Phe Arg Pro Glu Arg Phe Glu Asn Pro Ser Ala
370 375 380

Ile Pro Gln His Ala Phe Lys Pro Phe Gly Asn Gly Gln Arg Ala Cys
385 390 395 400

Ile Gly Gln Gln Phe Ala Leu His Glu Ala Thr Leu Val Leu Gly Met
405 410 415

Met Leu Lys His Phe Asp Phe Glu Asp His Thr Asn Tyr Glu Leu Asp
420 425 430

Ile Lys Glu Thr Leu Thr Leu Lys Pro Glu Gly Phe Val Val Lys Ala
435 440 445

Lys Ser Lys Lys Ile Pro Leu Gly Gly Ile Pro Ser Pro Ser Thr Glu
450 455 460

```
Gln Ser Ala Lys Lys Val Cys Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720
```

Asp Ala Ser Gln Gln Ile Arg Leu Glu Ala Glu Glu Glu Lys Leu Ala
725 730 735

His Leu Pro Leu Ala Lys Thr Val Ser Val Glu Glu Leu Leu Gln Tyr
740 745 750

Val Glu Leu Gln Asp Pro Val Thr Arg Thr Gln Leu Arg Ala Met Ala
755 760 765

Ala Lys Thr Val Cys Pro Pro His Lys Val Glu Leu Glu Ala Leu Leu
770 775 780

Glu Lys Gln Ala Tyr Lys Glu Gln Val Leu Ala Lys Arg Leu Thr Met
785 790 795 800

Leu Glu Leu Leu Glu Lys Tyr Pro Ala Cys Glu Met Lys Phe Ser Glu
805 810 815

Phe Ile Ala Leu Leu Pro Ser Ile Arg Pro Arg Tyr Tyr Ser Ile Ser
820 825 830

Ser Ser Pro Arg Val Asp Glu Lys Gln Ala Ser Ile Thr Val Ser Val
835 840 845

Val Ser Gly Glu Ala Trp Ser Gly Tyr Gly Glu Tyr Lys Gly Ile Ala
850 855 860

Ser Asn Tyr Leu Ala Glu Leu Gln Glu Gly Asp Thr Ile Thr Cys Phe
865 870 875 880

Ile Ser His Arg Gln Ser Glu Phe Thr Leu Pro Lys Asp Pro Glu Thr
885 890 895

Pro Leu Ile Met Val Gly Pro Gly Thr Gly Val Ala Pro Phe Arg Gly
900 905 910

Phe Val Gln Ala Arg Lys Gln Leu Lys Glu Gln Gly Gln Ser Leu Gly
915 920 925

Glu Ala His Leu Tyr Phe Gly Cys Arg Ser Pro His Glu Asp Tyr Leu
930 935 940

Tyr Gln Glu Glu Leu Glu Asp Ala Gln Ser Glu Gly Ile Ile Thr Leu
945 950 955 960

His Thr Ala Phe Ser Arg Met Pro Asn Gln Pro Lys Thr Tyr Val Gln
965 970 975

His Val Met Glu Gln Asp Gly Lys Lys Leu Ile Glu Leu Leu Asp Gln
980 985 990

Gly Ala His Phe Tyr Ile Cys Gly Asp Gly Ser Gln Met Ala Pro Ala
995 1000 1005

Val Glu Ala Thr Leu Met Lys Ser Tyr Ala Asp Val His Gln Val
1010 1015 1020

Ser Glu Ala Asp Ala Arg Leu Trp Leu Gln Gln Leu Glu Glu Lys
1025 1030 1035

Gly Arg Tyr Ala Lys Asp Val Trp Ala Gly
1040 1045

<210> 16
<211> 3147
<212> DNA
<213> Bacillus megaterium

<400> 16

atgacaatta aagaaatgcc tcagccaaaa acgtttggag agcttaaaaa tttaccgtta 60

ttaaacacag ataaaccggt tcaagctttg atgaaaattg cggatgaatt aggagaaatc 120

tttaaattcg aggcgcctgg tcgtgtaacg cgctacttat caagtcagcg tctaattaaa 180

gaagcatgcg atgaatcacg ctttgataaa aacttaagtc aagcgcttaa atttgtacgt 240

gattttgcag gagacgggtt atttacaagc tggacgcatg aaaaaaattg gaaaaaagcg 300

cataatatct tacttccaag cttcagtcag caggcaatga aaggctatca tgcgatgatg 360

gtcgatatcg ccgtgcagct tgttcaaaag tgggagcgtc taaatgcaga tgagcatatt 420

gaagtaccgg aagacatgac acgtttaacg cttgatacaa ttggtctttg cggctttaac 480

tatcgcttta acagctttta ccgagatcag cctcatccat ttattacaag tatggtccgt 540

gcactggatg aagcaatgaa caagctgcag cgagcaaatc cagacgaccc agcttatgat 600

gaaaacaagc gccagtttca agaagatatc aaggtgatga acgacctagt agataaaatt 660

attgcagatc gcaaagcaag cggtgaacaa agcgatgatt tattaacgca tatgctaaac 720

ggaaaagatc cagaaacggg tgagccgctt gatgacgaga acattcgcta tcaaattatt 780

acattcttaa ttgcgggaca cgaaacaaca agtggtcttt tatcatttgc gctgtatttc 840

ttagtgaaaa atccacatgt attacaaaaa gcagcagaag aagcagcacg agttctagta 900

gatcctgttc caagctacaa acaagtcaaa cagcttaaat atgtcggcat ggtcttaaac 960

gaagcgctgc gcttatggcc aactgctcct gcgttttccc tatatgcaaa agaagatacg 1020

gtgcttggag gagaatatcc tttagaaaaa ggcgacgaac taatggttct gattcctcag 1080

cttcaccgtg ataaaacaat ttggggagac gatgtggaag agttccgtcc agagcgtttt 1140
gaaaatccaa gtgcgattcc gcagcatgcg tttaaaccgt ttggaaacgg tcagcgtgcg 1200
tgtatcggtc agcagttcgc tcttcatgaa gcaacgctgg tacttggtat gatgctaaaa 1260
cactttgact ttgaagatca tacaaactac gagctggata ttaaagaaac tttaacgtta 1320
aaacctgaag gctttgtggt aaaagcaaaa tcgaaaaaaa ttccgcttgg cggtattcct 1380
tcacctagca ctgaacagtc tgctaaaaaa gtacgcaaaa aggcagaaaa cgctcataat 1440
acgccgctgc ttgtgctata cggttcaaat atgggaacag ctgaaggaac ggcgcgtgat 1500
ttagcagata ttgcaatgag caaaggattt gcaccgcagg tcgcaacgct tgattcacac 1560
gccggaaatc ttccgcgcga aggagctgta ttaattgtaa cggcgtctta taacggtcat 1620
ccgcctgata acgcaaagca atttgtcgac tggttagacc aagcgtctgc tgatgaagta 1680
aaaggcgttc gctactccgt atttggatgc ggcgataaaa actgggctac tacgtatcaa 1740
aaagtgcctg cttttatcga tgaaacgctt gccgctaaag ggcagaaaa catcgctgac 1800
cgcggtgaag cagatgcaag cgacgacttt gaaggcacat atgaagaatg gcgtgaacat 1860
atgtggagtg acgtagcagc ctactttaac ctcgacattg aaaacagtga agataataaa 1920
tctactcttt cacttcaatt tgtcgacagc gccgcggata tgccgcttgc gaaaatgcac 1980
ggtgcgtttt caacgaacgt cgtagcaagc aaagaacttc aacagccagg cagtgcacga 2040
agcacgcgac atcttgaaat tgaacttcca aaagaagctt cttatcaaga aggagatcat 2100
ttaggtgtta ttcctcgcaa ctatgaagga atagtaaacc gtgtaacagc aaggttcggc 2160
ctagatgcat cacagcaaat ccgtctggaa gcagaagaag aaaaattagc tcatttgcca 2220
ctcgctaaaa cagtatccgt agaagagctt ctgcaatacg tggagcttca agatcctgtt 2280
acgcgcacgc agcttcgcgc aatggctgct aaaacggtct gcccgccgca taaagtagag 2340
cttgaagcct tgcttgaaaa gcaagcctac aaagaacaag tgctggcaaa acgtttaaca 2400
atgcttgaac tgcttgaaaa atacccggcg tgtgaaatga aattcagcga atttatcgcc 2460
cttctgccaa gcatacgccc gcgctattac tcgatttctt catcacctcg tgtcgatgaa 2520
aaacaagcaa gcatcacggt cagcgttgtc tcaggagaag cgtggagcgg atatggagaa 2580
tataaaggaa ttgcgtcgaa ctatcttgcc gagctgcaag aaggagatac gattacgtgc 2640
tttatttcca caccgcagtc agaatttacg ctgccaaaag accctgaaac gccgcttatc 2700
atggtcggac cgggaacagg cgtcgcgccg tttagaggct ttgtgcaggc gcgcaaacag 2760
ctaaaagaac aaggacagtc acttggagaa gcacatttat acttcggctg ccgttcacct 2820
catgaagact atctgtatca agaagagctt gaaaacgccc aaagcgaagg catcattacg 2880
cttcataccg ctttttctcg catgccaaat cagccgaaaa catacgttca gcacgtaatg 2940

gaacaagacg gcaagaaatt gattgaactt cttgatcaag gagcgcactt ctatatttgc 3000
ggagacggaa gccaaatggc acctgccgtt gaagcaacgc ttatgaaaag ctatgctgac 3060
gttcaccaag tgagtgaagc agacgctcgc ttatggctgc agcagctaga agaaaaaggc 3120
cgatacgcaa aagacgtgtg ggctggg 3147

<210> 17
<211> 45
<212> DNA
<213> Artificial sequence

<220>
<223> /note="Description of artificial sequence:Forward primer"
"

<400> 17
accatgggca gcatgacaat taaagaaatg cctcagccaa aaacg 45

<210> 18
<211> 41
<212> DNA
<213> Artificial sequence

<220>
<223> /note="Description of artificial sequence:Reverse primer"

<400> 18
ggctttgtta gcttacccag cccacacgtc ttttgcgtat c 41

## Description

The present invention relates to a nucleic acid molecule encoding a polypeptide having cytochrome P450 monooxygenase activity, wherein said polypeptide comprises a reductase domain that deviates by at least one mutation and less than 70 mutations from (a) the reductase domain of cytochrome P450 BM3, wherein the reductase domain of cytochrome P450 BM3 is represented by SEQ ID NO:1; or (b) a reductase domain having at least 95% sequence identity to SEQ ID NO:1; and wherein said mutation(s) result(s) in an increased cytochrome P450 monooxygenase activity as compared to a polypeptide comprising the reductase domain of SEQ ID NO:1, as characterized in the appended claims. The present invention also relates to a vector comprising the nucleic acid molecule of the invention and a host transformed with and comprising the vector. Furthermore, the invention relates to a method of producing a polypeptide comprising culturing the host of the invention as well as to a polypeptide encoded by the nucleic acid molecule of the invention or produced by the method of the invention. The present invention further relates to the use of the polypeptide of the invention in biotransformation or fine chemical synthesis as well as to a composition.

Cytochrome P450 enzymes are a group of enzymes involved in the oxidative metabolism of drugs and carcinogens as well as a high number of natural compounds such as steroids, fatty acids, prostaglandins, and leukotrienes (Porter, T.D. and Coon, M.J. (1991) Cytochrome P450: multiplicity of isoforms, substrates, and catalytic and regulatory mechanisms, J. Biol. Chem.266: 13469-13472). They are found throughout the phylogenetic spectrum and have been classified into about forty different families. P450 enzymes are heme-proteins with a conserved cysteine residue. Many bacteria utilize P450 to grow on particular carbon sources or in the biosynthesis of secondary metabolites.

The monooxygenase P450 BM-3 is a cytochrome P450 enzyme originating from *Bacillus megaterium* -the product of the CYP 102 gene- and shows a high sequence homology with P450 enzymes from mammals (Boddupalli, S. S. et al., 1990, Fatty acid monooxygenation by cytochrome P-450 BM-3, J. Biol. Chem. 265, 4233-4239). Due to this similarity, P450 BM-3 represents a suitable model for mammalian P450 enzymes. P450 BM-3 is a 119 kDa watersoluble enzyme and its catalytic activity is one of the highest among all known P450 enzymes. Furthermore, cytochrome P-450 BM-3 is a catalytically self-sufficient fatty acid hydroxylase which monooxygenates saturated and unsaturated fatty acids, alcohols, and amides. Conversion of long chain alcohols and amines has also been reported as a natural activity of P450 BM-3. The enzyme converts lauric, myristic, and palmitic acids to omega-1, omega-2, and omega-3 hydroxy analogues. The catalytic activity is dependent on the chain length of the fatty acid, with an optimum chain length of 14 to 16 carbon atoms.

The P450 BM-3 protein consists of 1048 amino acids and has two domains: a first domain which contains heme and is P-450-oxygenase-like (471 aa; also referred to herein as oxygenase domain) and a second domain which contains FAD and FMN and is P-450 reductase-like (577 aa; also referred to herein as reductase domain). Both domains are located on a single polypeptide chain, separated by a trypsin cleavage site (Ruettinger et al. (1989), Coding nucleotide, 5'-Regulatory, and Deduced Amino Acid Sequences of P-450 BM-3, a Single Peptide Cytochrome P-450:NADPH-P-450 reductase from Bacillus megaterium, J. Biol. Chem. 264, 19: 10987-10995). Attempts to crystallize the full length protein were unsuccessful, most likely due to the presence of a highly flexible "hinge" region connecting the heme and the reductase domains of the protein. However, the structure of the heme- as well as the heme/FMN-binding complex have been solved. These structures enabled the rational investigation of the structure/function relationship in P450 BM3 by identifying key residues involved in substrate binding and catalysis as well as electron transfer from NADPH (Ravichandran, K.G., Boddupalli, S.S., Hasermann, C.A., Peterson, J.A. and Deisenhofer, J. (1993), Crystal structure of hemoprotein domain of P450 BM-3, a prototype for microsomal P450's. Science 261, 731-736; Sevrioukova, I.F., Li, H., Zhang, H., Peterson, J.A. and Poulos, T.L. (1999) Structure of a cytochrome P450-redox partner electron-transfer complex. Proc Natl Acad Sci USA 96: 1863-1868).

In the literature a number of variants and mutations of P450 BM3 have been described that aim at changing the substrate specificity and the products of hydroxylation. Notably, however, these mutations were predominantly located in the heme domain of the enzyme, whereas the reductase domain was mostly wild type. The heme domain of P450 BM3 consists of $\alpha$ and $\beta$ sub-domains. The heme in the active site is positioned within a long hydrophobic substrate binding channel formed predominantly by $\beta$ sub-domains. The heme porphyrin ring is bound to the rest of the polypeptide chain through the Cys400 residue, which is well conserved among other P450s. So far, a number of different residues have been identified as having a possible effect on substrate-binding or catalysis. For example, Arg47 is thought to interact with carboxyl groups of fatty acids, thus stabilizing the negative charge of the substrate through ionic interaction while Phe87 is thought to have an important role in substrate binding and region-selectivity of oxidation. Further, Arg 471 was found to improve the stability of P450 BM3 toward co-solvents such as DMSO (Wong, T.S., Arnold, F.H., and Schwaneberg, U., 2004, Laboratory evolution of cytochrome P450 BM-3 monooxygenase for organic co-solvents. Biotechnology and Bioengineering 85, 3: 351-358). Amongst others, these residues have an important role on substrate recognition and conversion (Noble, M.A., Miles, C.S., Chapman, S.K., Lysek, D.A., MacKay, A.C., Reid, G.A., Hanzlik, R.P. and Munro, A.W. (1999) Roles of key active-site residues in flavocytochrome P450 BM3. Biochem J. 339: 371-379).

Further publications that have described mutations in the heme domain are e.g. Whitehouse et al. (2009), A

highly active single-mutation variant of P450 BM3 (CYP102A1), Chem. Biochem., 10(10): 1654-6 and Girvan, HM. (2009), Novel haem co-ordination variants of flavocytochrome P450 BM3, Biochem. J. 417(1): 65-76. Furthermore, also a number of patents/patent applications disclose mutations in the heme domain and their effect on enzyme characteristics (see e.g. US 7691616, US 776768, US 7704715, EP 1196603 B1, EP 1196545 B1, EP 1196605 B1, US 7531335, US 7524664, US 7226768, US 2009/0264311 A1, WO 2003/008563 A2).

One example of engineering both the heme and the reductase domains of cytochrome P450 monooxygenase is described in WO 2008/115844 A2, which discloses chimeric P450 holoenzymes obtained by site-directed recombination. Chimeric enzymes comprising rearranged segments of the heme- and the reductase domains were recombinantly expressed in host cells and the enzymes investigated for improved monooxygenase activity as well as different substrate specificity of the chimeric polypeptides as compared to the wild- type polypeptide, showing that a whole set of novel enzymes were generated by this procedure.

Mutations in the reductase domain - or in both the heme and the reductase domain - are rarely described in the art. The reductase domain was described to be mutated in two positions - amino acids 494 and 1024 - in US 7226768 and WO 2003/008563 A2. With regard to these mutations in the reductase domain, the effect disclosed is a higher organic solvent resistance, while mutations in the heme domain are discussed in US 7226768 and WO 2003/008563 A2 as resulting in a higher alkane/alkene-oxidation capability. Although some of the mutants disclosed in US 7226768 and WO 2003/008563 A2 are shown to have an increased activity also in the absence of co-solvents, these results were obtained for enzymes having mutations in both the heme and the reductase domain. The inventors of US 7226768 and WO 2003/008563 ascribe the improvement to the mutations in the heme domain and show a further improvement in the presence of co-solvents, which is ascribed to the mutations in the reductase domain which are believed to increase the enzyme's resistance to these solvents.

Patent applications WO 2007/129050 and US 2009/0186415 A1 disclose beneficial effects of a truncated reductase domain on the co-expressed cytochrome P450 with regard to expression and activity of cytochrome P450.

WO 03/014341 describes a chimeric protein comprising the P450 BM3 reductase domain and the heme domain from a mammalian or plant cytochrome P450 useful for analysis of substrate specificities.

Despite the above described advances in the development of P450 enzymes with altered enzyme characteristics, there is still the need to provide P450 enzymes having increased enzymatic activity.

This need is addressed by the provision of the embodiments characterised in the claims.

Accordingly, the present invention relates to a nucleic acid molecule encoding a polypeptide having cytochrome P450 monooxygenase activity, wherein said polypeptide comprises a reductase domain that deviates by at least one mutation and less than 70 mutations from (a) the reductase domain of cytochrome P450 BM3, wherein the reductase domain of cytochrome P450 BM3 is represented by SEQ ID NO:1; or (b) a reductase domain having at least 95% sequence identity to SEQ ID NO:1; and wherein at least one of said at least one mutations in the reductase domain occurs at a position in SEQ ID NO:1 corresponding to position 812 in SEQ ID NO:2 and wherein said mutation results in an increased cytochrome P450 monooxygenase activity as compared to a polypeptide comprising the reductase domain of SEQ ID NO:1, wherein said cytochrome P450 monooxygenase activity is selected from the group consisting of monooxygenation of saturated and/or unsaturated fatty acids, alcohols, and/or amides; conversion of long chain alcohols and/or amines, conversion of lauric, myristic, and/or palmitic acids to omega-1, omega-2, and/or omega-3 hydroxy analogues and hydroxylation of coumarine based substrates.

The term "nucleic acid molecule" in accordance with the present invention includes DNA, such as cDNA or genomic DNA, and RNA. In this regard, "DNA" (deoxyribonucleic acid) means any chain or sequence of the chemical building blocks adenine (A), guanine (G), cytosine (C) and thymine (T), called nucleotide bases, that are linked together on a deoxyribose sugar backbone. DNA can have one strand of nucleotide bases, or two complementary strands which may form a double helix structure. "RNA" (ribonucleic acid) means any chain or sequence of the chemical building blocks adenine (A), guanine (G), cytosine (C) and uracil (U), called nucleotide bases, that are linked together on a ribose sugar backbone. RNA typically has one strand of nucleotide bases. Included are also single- and double-stranded hybrid molecules, i.e., DNA-DNA, DNA-RNA and RNA-RNA, wherein one of the strands encodes the mentioned polypeptide. The nucleic acid molecule may also be modified by means known in the art. Non-limiting examples of such modifications include methylation, "caps", substitution of one or more of the naturally occurring nucleotides with an analogue, and inter-nucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.). Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), peptide nucleic acid (PNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. Also included are nucleic acids containing modified bases, for example thio-uracil, thio-guanine and fluorouracil. A nucleic acid molecule typically carries genetic

information, including the information used by the cellular machinery to make polypeptides. The nucleic acid molecule of the invention may additionally comprise promoters, enhancers, response elements, signal sequences, polyadenylation sequences, introns, 5'- and 3'- non-coding regions, and the like.

The term "polypeptide" as used herein interchangeably with the term "protein" describes linear molecular chains of amino acids, including single chain proteins or their fragments, containing more than 30 amino acids. The term "fragment of the protein" in accordance with the present invention refers to a portion of the protein comprising at least the amino acid residues necessary to maintain the biological activity of the protein. Polypeptides may further form oligomers consisting of at least two identical or different molecules. The corresponding higher order structures of such multimers are, correspondingly, termed homo- or heterodimers, homo- or heterotrimers etc. Homo- or heterodimers etc. also fall under the definition of the term "polypeptide". Furthermore, peptidomimetics of such polypeptides where amino acid(s) and/or peptide bond(s) have been replaced by functional analogues are also encompassed by the invention. Such functional analogues include all known amino acids other than the 20 gene-encoded amino acids, such as selenocysteine. The term "polypeptide" also refers to naturally modified polypeptides where the modification is effected e.g. by glycosylation, acetylation, phosphorylation and similar modifications which are well known in the art.

In accordance with the present invention, the term "having cytochrome P450 monooxygenase activity" requires that the polypeptide encoded by the nucleic acid molecule of the invention has the biological activity of said enzyme, as characterized in the appended claims. Cytochrome P450 monooxygenase activity preferably refers to the biological activity of enzymes of class E.C. 1.14.-.-, more preferably of the P450 family CYP102. Such activity includes, without being limiting, at least one of monooxygenation of saturated and/or unsaturated fatty acids, alcohols, and/or amides; conversion of long chain alcohols and/or amines as well as conversion of lauric, myristic, and/or palmitic acids to omega-1, omega-2, and/or omega-3 hydroxy analogues. Methods of determining said activity are well known to the person skilled in the art and include, for example, measuring the conversion of p-nitrophenoxydodecanoic acid (p-NCA) as described previously (Schwaneberg, U., Schmidt-Dannert, C., Schmitt, J., Schmid, R.D. 1999, A continuous spectrophotometric assay for P450 BM-3, a fatty acid hydroxylating enzyme and its mutant F87A. Anal. Biochem 269: 359-366). Furthermore, the hydroxylation of BCC, BCC acid or DBCC as shown in the present invention (see examples) may also be used to assess cytochrome P450 monooxygenase activity.

The term "reductase domain", as used herein, refers to an amino acid sequence that functions as an electron donor. In particular, it serves as an electron donor for the oxygenase portion of a P450 enzyme, i.e. the heme domain. In cytochrome P450 BM3, represented by SEQ ID NO:2, the reductase domain starts at position 472 in SEQ ID NO:2 and ends at position 1048 in SEQ ID NO:2.

The term "mutation" in accordance with the present invention refers to changes in the amino acid sequence of a polypeptide and includes substitution, insertion, addition, inversion or deletion of one or more amino acids in the polypeptide.

In accordance with the present invention, the polypeptide comprising a reductase domain that deviates by at least one mutation and less than 70 mutations from the reductase domain of cytochrome P450 BM3, wherein the reductase domain of cytochrome P450 BM3 is represented by SEQ ID NO:1; or (b) a reductase domain having at least 95% sequence identity to SEQ ID NO:1, is also referred to herein as the "mutant polypeptide" or the "variant polypeptide".

The term "substitution" in accordance with the present invention, refers to (a) point mutation(s) resulting in an amino acid exchange as compared to the parent sequence. A substitution is the preferred mutation, in accordance with this invention.

The term "insertion" in accordance with the present invention refers to the addition of one or more amino acids to the parent sequence, wherein the addition is not to the N- or C-terminal end of the polypeptide.

The term "addition" in accordance with the present invention refers to the addition of one or more amino acids to the parent sequence, to the N- or C-terminal end of the polypeptide. In this case, the resulting mutant polypeptide may also be a fusion protein including for example amino acid sequence which confer desired properties such as modified/enhanced stability or solubility or tags for improved purification, such as for example a V5- or poly-His-tag or a tap-tag.

The term "deletion" as used in accordance with the present invention refers to the loss of one or more amino acids from the parent sequence; wherein the term does not include N-terminal or C-terminal truncations of the respective amino acid sequence.

The parent sequence, in accordance with the present invention, is a polypeptide comprising a reductase domain having either the sequence of SEQ ID NO:1 or at least 95% sequence identity to SEQ ID NO:1. The parent sequence can for example be cytochrome P450 BM3 as represented by SEQ ID NO:2, which comprises an oxygenase (i.e. heme) domain and a reductase domain, wherein the reductase domain spans from amino acids 472 to 1048 of SEQ ID NO:2 and corresponds to SEQ ID NO:1 (see Figure 4 for an alignment of SEQ ID NOs: 1 and 2). The coding sequence of cytochrome P450 BM3 is represented by SEQ ID NO:16.

In a preferred embodiment, the mutant polypeptide of the invention is not a polypeptide resulting solely from a deletion of amino acids as compared to the parent sequence. In a more preferred embodiment, the mutant polypeptide

of the invention deviates from the parent sequence in at least one amino acid substitution. In a further more preferred embodiment, the mutant polypeptide of the invention deviates from the parent sequence in that each of the at least one mutations in the reductase domain are amino acid substitutions.

The term "deviates by at least one mutation" refers to any deviation by one or more mutations, such as for example at least two mutations, such as at least three mutations, such as at least four mutations, such as at least five mutations, such as at least six mutations, such as at least seven mutations, such as at least eight mutations, such as at least nine mutations, such as at least ten mutations, such as at least 12 mutations, such as at least 15 mutations, such as at least 20 mutations, such as at least 25 mutations, such as at least 30 mutations or at least 35 mutations. In accordance with the present invention, the mutant polypeptides of the invention deviate from the parent sequence by less than 70 mutations, such as for example less than 50 mutations, such as less than 40 mutations. Any numerical values not explicitly mentioned above but falling within the above recited preferred ranges are also envisaged by the term "at least one mutation".

Methods for introducing mutations into the amino acid sequence of enzymes are well known in the art and include, without being limiting, directed evolution (e.g. Li, Q.S., Schwaneberg, U., Fischer, P., Schmid, R.D. 2000, Directed evolution of the fatty-acid hydroxylase P450 BM-3 into an indole-hydroxylating catalyst. Chemistry 6: 1531-1536), saturation and sequence saturation mutagenesis (Miyazaki, K., Arnold, F.H. 1999, Exploring nonnatural evolutionary pathways by saturation mutagenesis: rapid improvement of protein function. J. Mol. Evol. 49: 716-720; Wong TS, Tee KL, Hauer B, Schwaneberg U 2004, Sequence saturation mutagenesis (SeSaM): a novel method for directed evolution, Nucleic Acids Res. 10, 32(3) e26.), random mutagenesis (e.g. Chen, K., Arnold, F.H. 1993, Tuning the activity of an enzyme for unusual environments: sequential ramdom mutagenesis of subtilisin E for catalysis in dimethylformamide. Proc. Natl. Acad. Sci. USA 90: 5618-5622; McCullum E.O., Williams B.A., Zhang J., Chaput J.C. 2010, Random mutagenesis by error-prone PCR, Methods Mol Biol. 634:103-9) and others. Directed evolution has been described previously as a suitable way to introduce mutations into enzymes to generated variants with novel enzymatic properties (e.g. Sachis et al. (2008), Improved PCR method for the creation of saturation mutagenesis libraries in directed evolution: application to difficult-to-amplify templates. Appl. Microbiol. Biotechnol. 81(2): 387-97; Stemmer, W. (1994), Rapid evolution of a protein in vitro by DNA shuffling, Nature 370, 6488: 389-391; Tee KL, Schwaneberg U. (2007) Directed evolution of oxygenases: screening systems, success stories and challenges, Comb Chem High Throughput Screen 10: 197-217. Review). Also WO 2001/042455 describes this method to engineer biosynthetic pathways by directed evolution techniques, while directed evolution of oxygenase enzymes, amongst them P450 oxygenase, was further disclosed in WO 01/77368 A1.

Directed evolution offers many advantages over the rational design approach, especially in the cases where a crystal structure of the enzyme is not available. Microtiter plate and solid phase screening systems, with throughput of $10^2$-$10^6$, are commonly applied screening formats for improving enzyme properties when it comes to oxygenases. They are usually laborious, cost ineffective and even under optimal conditions allowing only small number of variants to be screened. This limits one to use libraries generated in low mutagenesis conditions i.e. 1-2 amino acid changes per gene. Recently, new techniques with ultra high throughput (>$10^7$), based on fluorescent detection and flow cytometry have been published (Miller, O.J. et al. (2006), Directed evolution by in vitro compartmentalization, Nat Methods 3(7), 561-70). Ultra high throughput methods are either based on surface display technology or *in vitro* compartmentalization (IVC) within double emulsions. The latter approach enabled development of flow cytometry based screening systems which, compared to display techniques, can be extended to intracellular/excreted enzymes with a soluble reaction product. Most important is that IVC enables physical connection between the gene (encoding active variant of enzyme) and the reaction product (fluorescent probe). In case when gene library (DNA) is entrapped, enzyme variants are directly expressed within the droplet using cell-free expression system. This approach has been successfully applied for directed evolution of DNA polymerases, phosphotriesterases, methyltransferases, endonucleases and galactosidases.

Mutant polypeptides of the invention can for example be obtained by codon exchange within SEQ ID NO: 3, which represents a nucleic acid molecule encoding the reductase domain of SEQ ID NO:1. Thus, in accordance with the present invention, "a nucleic acid molecule encoding a polypeptide comprising a reductase domain that deviates by at least one mutation and less than 70 mutations from the reductase domain of SEQ ID NO: 1" preferably is a nucleic acid molecule comprising a sequence that deviates from the sequence of SEQ ID NO:3 by at least one nucleic acid mutation resulting in a mutation of the reductase domain represented by SEQ ID NO:1 as defined above.

The present invention further encompasses nucleic acid molecules which additionally comprise silent mutations, i.e. mutations that do not lead to an amino acid mutation. Further comprised are nucleic acid molecules which are additionally altered in accordance with the codon usage of a specific origin or host organism, as well as nucleic acid molecules comprising a sequence that further deviates from the nucleic acid molecule of SEQ ID NO:3 due to the degeneracy of the genetic code (i.e. without altering the corresponding amino acid sequence) or that encode a polypeptide with additional conservative nucleotide substitution (i.e. the corresponding amino acid is replaced by another amino acid with the same charge, size, polarity and/or solubility and which encode a polypeptide according to the invention with an "increased cytochrome P450 monooxygenase activity"), and the corresponding complementary sequences.

In accordance with the present invention the polypeptide of the invention may also comprise a reductase domain that deviates in at least one mutation from a "reductase domain having at least 95% sequence identity to SEQ ID NO:1". More preferably, said reductase domain has at least 96%, even more preferably at least 97% sequence identity to the reductase domain of SEQ ID NO:1. Even more preferably the reductase domain has at least 98% sequence identity to the reductase domain of SEQ ID NO:1 and most preferably at least 99% sequence identity to the reductase domain of SEQ ID NO:1.

In accordance with the present invention, the term "% sequence identity" describes the number of matches ("hits") of identical nucleotides/amino acids of two or more aligned nucleic acid or amino acid sequences as compared to the number of nucleotides or amino acid residues making up the overall length of the nucleic acid or amino acid sequences (or the overall compared part thereof). In other terms, using an alignment, for two or more sequences or subsequences the percentage of amino acid residues or nucleotides that are the same (e.g., 80% or 85% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of a test sequence. Preferred nucleic acid molecules/polypeptides in accordance with the invention are those where the described identity exists over a region that is at least about 15 to 25 amino acids or nucleotides in length, more preferably, over a region that is at least about 50 to 100 amino acids or nucleotides in length. More preferred nucleic acid molecules/polypeptides in accordance with the present invention are those having the described sequence identity over the entire length of the nucleic acid molecule or polypeptide. Those having skill in the art will know how to determine percent sequence identity between/among sequences using, for example, algorithms such as those based on the NCBI BLAST algorithm (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402), CLUSTALW computer program (Thompson Nucl. Acids Res. 2 (1994), 4673-4680) or FASTA (Pearson and Lipman, Proc. Natl. Acad. Sci., 1988, 85; 2444), as known in the art.

The NCBI BLAST algorithm is preferably employed in accordance with this invention. The BLASTN program for nucleic acid sequences uses as default a word length (W) of 11, an expectation (E) of 10, M=5, N=4, and a comparison of both strands. For amino acid sequences, the BLASTP program uses as default a word length (W) of 3, and an expectation (E) of 10. The BLOSUM62 scoring matrix (Henikoff, Proc. Natl. Acad. Sci., 1989, 89:10915) uses alignments (B) of 50, expectation (E) of 10, M=5, N=4, and a comparison of both strands. Accordingly, all the nucleic acid molecules having the prescribed function and further having a sequence identity of at least 95% as determined with the NCBI BLAST program fall under the scope of the invention.

In accordance with the present invention, the at least one mutation in the reductase domain is a "functional mutation". Thus, the altered amino acid sequence results in an increased cytochrome P450 monooxygenase activity as compared to a polypeptide comprising a reductase domain represented by SEQ ID NO:1. Such an increased cytochrome P450 monooxygenase activity results in a higher and/or faster substrate turn-over rate and may be due to, for example, an altered reactivity pattern or an altered substrate profile.

"Increased cytochrome P450 monooxygenase activity" includes, for the purposes of the present invention, an improved reactivity such as an increase in specific activity (for example expressed as nmol reacted carboxylic acid/minute/nmol P450-enzyme) and/or an improvement in at least one kinetic parameter selected from amongst Kcat, Km and Kcat/Km. Preferably, the improvement is at least 1.5-fold, such as, for example, at least 2-fold, at least 5-fold, at least 10-fold, at least 50-fold, at least 100-fold, at least 150-fold, at least 250-fold, at least 500-fold or at least 1000-fold, as compared to the parent sequence.

More preferably, where the mutant polypeptide of the invention comprises additional mutations, for example in the heme domain, previously known in the art to improve the enzymatic activity of polypeptides comprising the reductase domain represented by SEQ ID NO:1, then the mutant polypeptide of the invention preferably also possesses an improved reactivity (such as e.g. an increase in specific activity expressed for example as nmol reacted carboxylic acid/minute/nmol P450-enzyme and/or an improvement in at least one kinetic parameter selected from amongst Kcat, Km and Kcat/Km) as compared to such previously mutated polypeptides, such as for example the cytochrome P450 BM3 as represented by SEQ ID NO:4. The above recited amounts of improvement apply *inter alia.*

Methods to investigate the kinetic properties of an enzyme are well known in the art and include, without being limiting, the determination of Michaelis-Menten parameters. The Michaelis-Menten equation, as shown below, is the basis for the majority of single-substrate enzyme kinetics:

$$v_0 = \frac{V_{\mathbf{max}}[\mathrm{S}]}{K_M + [\mathrm{S}]}$$

The Michaelis constant $K_M$ is defined as the substrate concentration at which the rate of the enzyme reaction is half of the maximum reaction rate $V_{max}$, i.e. $V_{max}/2$.

There is typically one rate-determining enzymatic step that allows this reaction to be modelled as a single catalytic step with an apparent unimolecular rate constant $k_{cat}$. The apparent unimolecular rate constant $k_{cat}$ is defined by the following reaction:

$$ES \xrightarrow{k_{cat}} E + P$$

The rate-limiting step in an enzymatic reaction is generally considered as one with a rate equal to $K_{cat}$. However, it has been found for the P450 enzyme that more than one step may have an influence on Kcat or $K_{cat}/K_m$. The efficiency of oxidation of a substrate by the monooxygenase is dependent on the electron transfer steps which are accomplished by the reductase domain of the P450 holoenzyme. The electron transfer from the substrate to P450 has been identified as a major rate-limiting step of the oxidation reaction (Guengerich, F.P. rate-limiting steps in Cytochrome P450 catalysis, Biol. Chem., 383, 1553-1564). Therefore, we set out to improve the enzymatic properties of the reductase domain in order to overcome the bottleneck in the catalytic cycle of P450.

In accordance with the present invention, novel P450 enzymes with hitherto not described mutations in the reductase domain of the enzyme were identified. These novel variants provide an improved cytochrome P450 monooxygenase activity as compared to the parent enzyme and are, therefore, particularly useful in chemical and biotechnological applications of P450 BM3, e.g. for organic synthesis, oxidation of fatty acids and industrial biotransformation of pharmaceuticals.

The role of the reductase domain as an electron donor for the monooxygenase domain is crucial for P450 enzyme activity and, consequently, for its biotechnological application. Electron transfer from the reductase to the hydroxylase domain was identified as one of the rate limiting steps within the P450 catalytic cycle (Guengerich, F.P. (2002) rate-limiting steps in cytochrome P450 catalysis, Biol. Chem._383: 1553-1564). Nevertheless, the potential of improving the electron transfer within the P450 system has so far been widely underestimated (Berhardt, R. (2006) Cytochromes P450 as versatile biocatalysts, J. Biotechnol. 124: 128-145).

As shown in the examples below, modified P450 monooxygenases are provided that are based on BM3 parent enzymes that carry mutations in the heme domain of the enzyme that have previously been described in the literature. After mutagenesis of the sequence of P450 BM3 applying an error prone PCR method, recombinant clones were screened in enzyme assays with different substrates. Those enzymes with improved kinetic properties were sequenced in order to determine their respective primary structure on the nucleic acid level. The identified novel variants are characterized by the surprising fact that they carry mutations in the reductase domain of the holoenzyme and show altered enzymatic properties in that they exhibit significantly improved Michaelis-Menten parameters such as $K_m$ and $K_{cat}$, expressed as enzymatic efficiency ($K_{cat}/K_m$), as compared to the wild type and the parent mutant.

In a preferred embodiment, the mutant polypeptide having cytochrome P450 monooxygenase activity is derived from cytochrome P450 BM3, as represented by SEQ ID NO:2. In accordance with this embodiment, the mutant polypeptide of the invention deviates from the polypeptide of SEQ ID NO:2 by at least one mutation in the reductase domain of cytochrome P450 BM3, i.e. SEQ ID NO:1.

In accordance with the present invention, at least one of said at least one mutations in the reductase domain occurs at a position in SEQ ID NO:1 corresponding to position 812 in SEQ ID NO:2. Also described herein is that at least one of said at least one mutations in the reductase domain occurs at a position in SEQ ID NO:1 corresponding to position 730, 648, 512, 857, 1030, 798, 883, 884 and/or 951 in SEQ ID NO:2

The term "at least one of said at least one mutations in the reductase domain" as used herein refers to the fact that at least one of the mutations in the reductase domain has to occur at the recited position, as characterized in the appended claims. For example, if the mutant polypeptide of the invention comprises e.g. six mutations in the reductase domain, at least one of said six mutations has to occur at the recited position. Thus, further mutations may occur in the reductase domain at positions other then the recited position as long as at least one mutation occurs at the recited position and provided that the preferred amount of overall mutations is as defined above with regard to the main embodiment. It is understood that a mutant polypeptide of the invention may also comprise, for example, mutations in two, three, four, five, six, seven, eight, nine or ten of the above recited positions.

The position of the respective mutations within SEQ ID NO:1 are provided by reference to the corresponding position in SEQ ID NO:2, which represents the full length cytochrome P450 BM3 polypeptide including the reductase domain represented by SEQ ID NO:1. The sequence of SEQ ID NO:1 corresponds to the sequence spanning from position 472 to position 1048 of SEQ ID NO:2, as shown in the sequence alignment provided in Figure 4. In other words, position 472 of SEQ ID NO:2 corresponds to position 1 of SEQ ID NO:1. Furthermore, the preferred positions 812, 730, 648, 512, 857, 1030, 798, 883, 884 and/or 951 in SEQ ID NO:2 correspond to positions 341, 259, 177, 41, 386, 559,

327, 412, 413 and 481, respectively, in SEQ ID NO:1.

In a more preferred embodiment of the nucleic acid molecule of the invention, met at a position in SEQ ID NO:1 corresponding to position 812 in SEQ ID NO:2 is replaced by thr, ser, cys, asn, gln, asp or glu.

Also described herein is that ala at a position in SEQ ID NO:1 corresponding to position 730 in SEQ ID NO:2 is replaced by val, gly, leu, ile, met, phe, trp or pro; asp at a position in SEQ ID NO:1 corresponding to position 648 in SEQ ID NO:2 is replaced by gly, val, ala, leu, ile, met, phe, trp or pro; gln at a position in SEQ ID NO:1 corresponding to position 512 in SEQ ID NO:2 is replaced by arg, lys or his; tyr at a position in SEQ ID NO:1 corresponding to position 857 in SEQ ID NO:2 is replaced by asn, gln, thr, ser, cys, asp or glu; leu at a position in SEQ ID NO:1 corresponding to position 1030 in SEQ ID NO:2 is replaced by ser, tyr, asn, glu, tyr, thr, cys, asp or glu; leu at a position in SEQ ID NO:1 corresponding to position 798 in SEQ ID NO:2 is replaced by ser, tyr, asn, gln, tyr, thr, cys, asp or glu; thr at a position in SEQ ID NO:1 corresponding to position 883 in SEQ ID NO:2 is replaced by his, lys, arg, phe, tyr or trp; pro at a position in SEQ ID NO:1 corresponding to position 884 in SEQ ID NO:2 is replaced by arg, lys or his; and/or asn at a position in SEQ ID NO:1 corresponding to position 951 in SEQ ID NO:2 is replaced by asp, glu, tyr, ser, cys, thr, asn or gln.

The amino acids referred to herein are abbreviated in accordance with the established nomenclature employed in the art which is well known to the skilled person.

In an even more preferred embodiment, met at a position in SEQ ID NO:1 corresponding to position 812 in SEQ ID NO:2 is replaced by thr.

Also described herein is that ala at a position in SEQ ID NO:1 corresponding to position 730 in SEQ ID NO:2 is replaced by val; asp at a position in SEQ ID NO:1 corresponding to position 648 in SEQ ID NO:2 is replaced by gly; gln at a position in SEQ ID NO:1 corresponding to position 512 in SEQ ID NO:2 is replaced by arg; tyr at a position in SEQ ID NO:1 corresponding to position 857 in SEQ ID NO:2 is replaced by asn; leu at a position in SEQ ID NO:1 corresponding to position 1030 in SEQ ID NO:2 is replaced by ser; leu at a position in SEQ ID N0:1 corresponding to position 798 in SEQ ID NO:2 is replaced by ser; thr at a position in SEQ ID NO:1 corresponding to position 883 in SEQ ID NO:2 is replaced by his; pro at a position in SEQ ID NO:1 corresponding to position 884 in SEQ ID NO:2 is replaced by arg; and/or asn at a position in SEQ ID NO:1 corresponding to position 951 in SEQ ID NO:2 is replaced by asp.

In accordance with this preferred embodiment, a group of mutants is derived from *Bacillus megaterium* cytochrome P450 monooxygenase BM-3, which has the amino acid sequence as shown in SEQ ID NO: 2. These mutants deviate from the sequence of SEQ ID NO:2 as shown in table 1 below, wherein at least one of said mutations occurs in one of the following amino acid sequence positions: M812T, A730V, D648G, Q512R, Y857N, L1030S, L798S, T883H, P884R, N951D.

In some embodiments, the mutations in question are shown in the one-letter amino acid code. The original amino acid is shown before the number which indicates the sequence position of the mutation, while the modified amino acid is shown after the number.

In another preferred embodiment of the nucleic acid molecule of the invention, the polypeptide having cytochrome P450 monooxygenase activity deviates by at least one further mutation from cytochrome P450 BM3, wherein cytochrome P450 BM3 is represented by SEQ ID NO:2.

The term "at least one further mutation" as used herein relates to at least one mutation in addition to the above recited mutation(s) in the reductase domain, wherein the further mutation is in a part of the polypeptide other than the reductase domain, such as for example in the heme domain of the polypeptide. The definitions provided above with regard to mutations apply mutatis mutandis also to this/these further mutation(s). Thus, in accordance with the present invention, the resulting mutant polypeptide has an increased cytochrome P450 monooxygenase activity as compared to the polypeptide of SEQ ID NO:2. In a preferred embodiment, a mutant polypeptide of the invention deviating by at least one further mutation from the polypeptide of SEQ ID NO:2 possesses a more increased cytochrome P450 monooxygenase activity as compared to a mutant polypeptide only deviating by at least one mutation in the reductase domain of SEQ ID NO:1 or a reductase domain having at least 95% sequence identity to the reductase domain of SEQ ID NO:1. In this regard, a "more increased cytochrome P450 monooxygenase activity" refers to an at least 1.5-fold higher increases in cytochrome P450 monooxygenase activity, such as for example an at least 2-fold higher increase, such as an at least 3-fold higher increase, such as an at least 5-fold higher increase, such as an at least 10-fold higher increase, such as an at least 50-fold higher increase, such as an at least 100-fold higher increase, such as an at least 500-fold higher increase or such as an at least 1000-fold higher increase.

In a more preferred embodiment, at least one of said at least one further mutations occurs at a position corresponding to position 87, 471, 47, 29, 189, 13, 162, 354, 429, 64 and/or 223 in SEQ ID NO:2.

Mutations at the above recited positions in the heme domain of P450 monooxygenase are well known in the art. For example, Arg47 is thought to interact with carboxyl groups of fatty acids, thus stabilizing the negative charge of the substrate through ionic interaction. Amongst others, these residues have an important role on substrate recognition and conversion (Noble, M.A., Miles, C.S., Chapman, S.K., Lysek, D.A., MacKay, A.C., Reid, G.A., Hanzlik, R.P. and Munro, A.W. (1999) Roles of key active-site residues in flavocytochrome P450 BM3. Biochem J. 339: 371-379). These previously described mutations in the heme domain of P450 BM3 may for example be comprised in the parent enzymes

used as starting proteins for generating improved variants in accordance with the present invention.

In a particularly preferred embodiment of the nucleic acid molecule encoding a polypeptide having cytochrome P450 monooxygenase activity deviating by at least one further mutation from cytochrome P450 BM3, the further mutations are a mutation at a position corresponding to position 87 in SEQ ID NO:2 and a mutation at a position corresponding to position 471 in SEQ ID NO:2.

In a further more preferred embodiment of the nucleic acid molecule of the invention, phe at a position corresponding to position 87 in SEQ ID NO:2 is replaced by ala, gly, val, leu, ile, met, trp or pro; arg at a position corresponding to position 471 in SEQ ID NO:2 is replaced by cys, ser, tyr, thr, asn, gln, asp or glu; arg at a position corresponding to position 47 in SEQ ID NO:2 is replaced by phe, tyr, leu, val, gly, ala, ile, met, trp or pro; leu at a position corresponding to position 29 in SEQ ID NO:2 is replaced by ser, thr, cys, asn, gln, asp or glu; gln at a position corresponding to position 189 in SEQ ID NO:2 is replaced by arg, lys or his; glu at a position corresponding to position 13 in SEQ ID NO:2 is replaced by gly, val, ala, leu, ile, met, phe, trp or pro; phe at a position corresponding to position 162 in SEQ ID NO:2 is replaced by leu, val, gly, ala, ile, met, phe, trp or pro; met at a position corresponding to position 354 in SEQ ID NO:2 is replaced by ser, asn, gln, tyr, thr, cys, asp or glu; tyr at a position corresponding to position 429 in SEQ ID NO:2 is replaced by cys, ser, asn, gln, tyr, thr, asp or glu; glu at a position corresponding to position 64 in SEQ ID NO:2 is replaced by gly, val, ala, leu, ile, met, phe, trp or pro; and/or arg at a position corresponding to position 223 in SEQ ID NO:2 is replaced by his, lys, phe, tyr or trp.

In another more preferred embodiment of the nucleic acid molecule of the invention, phe at a position corresponding to position 87 in SEQ ID NO:2 is replaced by ala; arg at a position corresponding to position 471 in SEQ ID NO:2 is replaced by cys; arg at a position corresponding to position 47 in SEQ ID NO:2 is replaced by phe, tyr or leu; leu at a position corresponding to position 29 in SEQ ID NO:2 is replaced by ser; gln at a position corresponding to position 189 in SEQ ID NO:2 is replaced by arg; glu at a position corresponding to position 13 in SEQ ID NO:2 is replaced by gly; phe at a position corresponding to position 162 in SEQ ID NO:2 is replaced by leu; met at a position corresponding to position 354 in SEQ ID NO:2 is replaced by ser; tyr at a position corresponding to position 429 in SEQ ID NO:2 is replaced by cys; glu at a position corresponding to position 64 in SEQ ID NO:2 is replaced by gly; and/or arg at a position corresponding to position 223 in SEQ ID NO:2 is replaced by his.

In a more preferred embodiment, the combination of further mutations present in the mutant polypeptide of the invention are selected from the group consisting of: (a) F87A and R471C; (b) F87A, R471C and R47F; (c) F87A, R471C, R47Y and L29S; (d) F87A, R471C, R47L and E13G; (e) F87A, R471C, F162L and M354S; or (f) F87A, R471C, E64G, R223H and M354S.

**Table 1:** List of mutants of P450 BM3 employed herein as well as starting sequences (highlighted in gray)

| Variant | Mutations | SEQ ID NO |
|---|---|---|
| WT Reductase domain | - | SEQ ID NO: 1 |
| WT Holoenzyme | - | SEQ ID NO: 2 |
| F87A | F87A/R471C | SEQ ID NO: 4 |
| A3 | F87A/R471 C/**M812T** | SEQ ID NO: 5 |
| Dm1 | R47F/F87A/R471C | SEQ ID NO: 6 |
| E1 | R47F/F87A/R471C/**A730V** | SEQ ID NO: 7 |
| E9 | R47F/F87A/R471C/**D648G** | SEQ ID NO: 8 |
| H3 | R47F/F87A/R471C/**Q512R** | SEQ ID NO: 9 |
| M1 | L29S/R47Y/F87A/Q189R/R471C/**Y857N** | SEQ ID NO: 10 |
| M2 | E13G/R47L/F87A/R471C/**L1030S** | SEQ ID NO: 11 |
| Dm2#4 | F87A/M354S/R471C | SEQ ID NO: 12 |
| D7 | F87A/F162L/M354S/Y429C/R471C | SEQ ID NO: 13 |
| E5 | F87A/F162L/M354S/R471C/**L798S** | SEQ ID NO: 14 |
| M3 | E64G/F87A/R223H/M354S/R471C/**T883H/P884R/N9 51D** | SEQ ID NO: 15 |

In a further more preferred embodiment of the nucleic acid molecule of the invention, the polypeptide deviates from the polypeptide of SEQ ID NO:2 by the amino acid substitution pattern: (a) F87A, R471C and/or M812T. Also

described herein is a nucleic acid molecule encoding a polypeptide that deviates from the polypeptide of SEQ ID NO:2 by an amino acid substitution pattern selected from the group consisting of: (b) F87A, R471C, R47F and/or A730V; (c) F87A, R471C, R47F and/or D648G; (d) F87A, R471C, R47F and/or Q512R; (e) F87A, R471C, R47Y, L29S, Q189R and/or Y857N; (f) F87A, R471C, R47L, E13G and/or L1030S; (g) F87A, R471C, F162L, M354S and/or L798S; and (h) F87A, R471C, E64G, R223H, M354S, T883H, P884R and/or N951D.

The invention furthermore relates to expression constructs comprising a nucleic acid sequence encoding a mutant according to the invention under the genetic control of regulatory nucleic acid sequences.

Preferably, the constructs according to the invention encompass a promoter 5'-upstream and a terminator sequence 3'-downstream of the encoding sequence in question, and, if appropriate, other customary regulatory elements, in each case operatively linked with the encoding sequence. Operative linkage is to be understood as meaning the sequential arrangement of promoter, encoding sequence, terminator and, if appropriate, other regulatory elements in such a manner that each of the regulatory elements can fulfil its intended function on expression of the encoding sequence. Non-limiting examples of operatively linkable sequences are targeting sequences, or else translation enhancers, polyadenylation signals, selection markers, amplification signals, replication origins and the like.

In addition to the artificial regulatory sequences, the natural regulatory sequence can still be present upstream of the actual structural gene. If desired, this natural regulation may also be switched off by genetic alteration, and the expression of the genes may be enhanced or lowered. However, the gene construct may also be simpler in construction, i.e. no additional regulatory signals are inserted upstream of the structural gene and the natural promoter with its regulation is not removed. Instead, the natural regulatory sequence is mutated in such a way that regulation no longer takes place and the gene expression is increased or reduced. One or more copies of the nucleic acid sequences may be present in the gene construct. Non-limiting examples of promoters are: cos, tac, trp, tet, trp-tet, Ipp, lac, lpp-lac, laclq, T7, T5, T3, gal, trc, ara, SP6, I-PR or I-PL promoter, all of which are advantageously employed in gram-negative bacteria; as well as the gram-positive promoters amy and SPO2, the yeast promoters ADC1, MFa, AC, P-60, CYC1, GAPDH or the plant promoters CaMV/35S, SSU, OCS, lib4, usp, STLS1, B33, nos, or the ubiquitin or phaseolin promoter.

In principle, all natural promoters with their regulatory sequences can be used. In addition, synthetic promoters may also be used in an advantageous fashion.

The abovementioned regulatory sequences are intended to allow the directed expression of the nucleic acid sequences. Depending on the host organism, this may mean, for example, that the gene is expressed or over-expressed only after induction has taken placed, or that the gene is expressed and/or over-expressed immediately, i.e. without induction.

The regulatory sequences or factors can preferably have a positive effect on expression and in this manner increase or reduce the latter. Thus, an enhancement of the regulatory elements may advantageously take place at the transcriptional level by using strong transcription signals such as promoters and/or "enhancers". In addition, translation may also be enhanced by improving, for example, mRNA stability.

An expression cassette can be generated by fusing a suitable promoter with a suitable monooxygenase nucleotide sequence and a terminator signal or polyadenylation signal. The coding sequences can e.g. be synthesized by standard methods, or isolated from natural sources. For generating the expression cassette, customary recombination and cloning techniques are used as described, for example, by T. Maniatis, E. F. Fritsch and J. Sambrook, Molecular Cloning: A Laboratory Manual, Cold Spring Harbor Laboratory, Cold Spring Harbor, N.Y. (1989); by T. J. Silhavy, M. L. Berman and L. W. Enquist, Experiments with Gene Fusions, Cold Spring Harbor Laboratory, Cold Spring Harbor, N.Y. (1984) and by Ausubel, F. M. et al., Current Protocols in Molecular Biology, Greene Publishing Assoc. and Wiley Interscience (1987).

The present invention further relates to a vector comprising the nucleic acid molecule or the expression construct of the invention.

For expression in a suitable host organism, the recombinant nucleic acid construct or gene construct is advantageously inserted into a host-specific vector which allows optimal gene expression in said host. Vectors are well known to the skilled worker and can be found, for example, in "Cloning Vectors" (Pouwels P. H. et al., Ed., Elsevier, Amsterdam-N.Y.-Oxford, 1985). Vectors are to be understood as meaning not only plasmids, but all other vectors known to the skilled worker such as, for example, phages, viruses such as SV40, CMV, baculovirus and adenovirus, transposons, IS elements, phasmids, cosmids, and linear or circular DNA. These vectors can be replicated autonomously in the host organism or chromosomally.

Non-limiting examples of vectors include prokaryotic plasmid vectors, such as the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pAO815, pPIC9K and pPIC3.5K (all Invitrogen).

Furthermore, it is preferred that the vector of the invention comprises a selectable marker. Examples of selectable markers include neomycin, ampicillin, and hygromycin resistance and the like. Specifically-designed vectors allow the shuttling of DNA between different hosts, such as between bacteria and fungal cells or between bacteria and animal cells.

The vectors according to the invention allow the generation of recombinant microorganisms which can be transformed, for example, with at least one vector according to the invention and which can be employed for producing the mutants. The above-described recombinant constructs according to the invention can advantageously be introduced into a suitable host system and expressed. It is preferred to use usual cloning and transfection methods known to the skilled worker, for example co-precipitation, protoplast fusion, electroporation, retroviral transfection and the like, in order to bring about expression of the abovementioned nucleic acids in the expression system in question. Suitable systems are described, for example, in Current Protocols in Molecular Biology, F. Ausubel et al., Ed., Wiley Interscience, New York 1997.

Thus, the present invention also relates to a host transformed with and comprising the vector of the invention, wherein the host is not a human and not a human embryo.

Suitable host organisms are, in principle, all organisms which allow expression of the nucleic acids according to the invention, their allelic variants, and their functional equivalents or derivatives. Host organisms are to be understood as meaning bacteria, fungi, yeast, as well as plant or animal cells. Preferred organisms are bacteria such as those of the genera *Escherichia* such as, for example, *Escherichia coli* but also bacteria such as for example *Streptomyces, Bacillus* or *Pseudomonas,* eukaryotic microorganisms such as *Saccharomyces cerevisiae, Aspergillus,* and higher eukaryotic cells from animals or plants, for example Sf9 or CHO cells.

In addition, expression of the gene product may also be brought about in transgenic organisms such as transgenic animals such as, in particular, mice, sheep, or transgenic plants. The transgenic organisms may also be knock-out animals or plants in which the corresponding endogenous gene has been eliminated, such as, for example, by mutation or partial or complete deletion. Such transgenic animals may be suitable as model organisms for example for testing potential pharmaceutical compositions *in vivo.*

Successfully transformed organisms may be selected by means of marker genes which are also contained in the vector or in the expression cassette. Examples of such marker genes are genes for resistance to antibiotics and genes for enzymes which catalyze a colour reaction causing the transformed cell to be coloured. They may then be selected by means of automatic cell sorting. Microorganisms which have been transformed successfully with a vector and which carry a suitable gene for resistance to antibiotics (for example G418 or hygromycin) may be selected by suitable liquid or solid media containing antibiotics. Marker proteins presented on the cell surface may be exploited for selection by means of affinity chromatography.

The combination of the host organisms and the vectors which match the organisms, such as plasmids, viruses or phages, for example plasmids with the RNA polymerase/promoter system, the phages $\lambda$, $\mu$ or other temperate phages or transposons and/or further advantageous regulatory sequences, forms an expression system. The term "expression system" is to be understood as meaning, for example, the combination of mammalian cells, such as CHO cells, and vectors, such as pcDNA3neo vector, which are suitable for mammalian cells.

As described above, the gene product may advantageously also be expressed in transgenic animals, for example mice, sheep, or transgenic plants. Equally, it is possible to program cell-free translation systems with the RNA derived from the nucleic acid.

Furthermore, the present invention also relates to a method of producing a polypeptide comprising culturing the host of the invention under suitable conditions and isolating the polypeptide produced.

Thus, for example a monooxygenase-producing microorganism is cultured, monooxygenase expression is induced, if appropriate, and the monooxygenase is isolated from the culture. In this manner, the monooxygenase according to the invention may also be produced on an industrial scale, if this is desired.

The microorganism can be cultured and fermented by known methods. Bacteria can, for example, be multiplied in TB or LB medium at a temperature of 20 to 40° C and a pH of 6 to 9. Suitable culture conditions are described in detail for example by T. Maniatis, E. F. Fritsch and J. Sambrook, Molecular Cloning: A Laboratory Manual, Cold Spring Harbor Laboratory, Cold Spring Harbor, N.Y. (1989).

Unless the monooxygenase is secreted into the culture medium, the cells have to be disrupted, and the monooxygenase is then obtained from the lysate by known protein isolation methods. The cells may be disrupted as desired, for example by high-frequency ultrasound, by high pressure, such as, for example, in a French pressure cell, by osmolysis, by the action of detergents, lytic enzymes or organic solvents, by homogenizers, or by combining a plurality of the methods listed.

The monooxygenase may be purified by known methods of chromatography, such as molecular sieve chromatography (gel filtration), such as chromatography on Q-Sepharose, ion-exchange chromatography and hydrophobic chromatography, and by other customary methods such as ultra-filtration, crystallization, salting out, dialysis and native gel electrophoresis. Suitable methods are described, for example, by Cooper, F. G., Biochemische Arbeitsmethoden [Methods in Biochemistry], Verlag Walter de Gruyter, Berlin, New York or by Scopes, R., Protein Purification, Springer

Verlag, New York, Heidelberg, Berlin.

To isolate the recombinant protein, it is especially advantageous to use vector systems or oligonucleotides which extend the cDNA by certain nucleotide sequences and thus encode altered polypeptides or fusion proteins for the purposes of simpler purification. Such modifications which are suitable are, for example, tags which act as anchors, for example the modification known as hexa-histidine anchor or epitopes which can be recognized as antigens of antibodies (described, for example, by Harlow, E. and Lane, D., 1988, Antibodies: A Laboratory Manual. Cold Spring Harbor (N.Y.) Press). These anchors can serve to attach the proteins to a solid support such as, for example, a polymer matrix, which may be filled into a chromatography column or used on a microtiter plate or on any other carrier, for example.

The present invention further relates to a polypeptide encoded by the nucleic acid molecule of the invention or produced by the method of the invention.

This polypeptide is also referred to herein as the mutant polypeptide of the invention or the variant polypeptide of the invention.

Further, the present invention relates to the use of the polypeptide of the invention in synthetic chemical transformation, biotransformation or fine chemical synthesis.

The term "synthetic chemical transformation" in accordance with the present invention relates to chemical alteration of synthetic chemicals by the use of enzymes such as monooxygenases to obtain transformation products with altered properties, e.g increased biodegradability in the environment or desired chemical or biochemical activity.

The term "biotransformation" means alteration of compounds such as (but not limited to) nutrients, amino acids, toxins, or drugs either in the body or -by enzymatic biotransformation-in vitro. One major application is the production of chiral compounds as building blocks for more complex molecules such as pharmaceuticals or catalysts by oxidative reactions (e.g. redox enzymes) or asymmetric sythesis (e.g. dehydrogenases). Cytochromes are principal enzymes involved in oxidative metabolism of drugs and other xenobiotics. From the bioindustrial point of view these enzymes can be involved in diverse synthetic transformations such as hydroxylation of the alkanes and aromatic hydrocarbons, epoxidation of carbon-carbon double bonds and heteroatom oxygenation. Non-limiting examples include the transformation of steroids and prostaglandins.

Fine chemicals are pure, single chemical substances that are commercially produced with chemical reactions into highly specialized applications. Fine chemicals produced can be categorized into active pharmaceutical ingredients and their intermediates, biocides, and specialty chemicals for technical applications. In chemical technology, a distinction is made between bulk chemicals, which are produced in massive quantities by standardized reactions, and fine chemicals, which are custom-produced in smaller quantities for special uses. For the synthesis of fine chemicals enzymes are required which catalyze chemical reactions with a high degree of specificity, especially entio- and stereoselectivity. Thus, the term "fine chemical synthesis", as used herein, refers to procedures for the regio- and stereo-controlled transformation of compounds involving oxidation or reduction reactions. This is accomplished by a wide range of catalysts, including organometallic systems, biocatalysts and biomimetics. Reactions in which the mutant polypeptides of the present invention may be employed include, without being limiting, hydroxylation reactions; oxidation of alcohols to aldehydes, ketones and carboxylic acids; reduction of ketones; and reduction of alkenes including $\alpha$, $\beta$-unsaturated carbonyl compounds. Further non-limiting exemplary reactions include Baeyer-Villiger oxidations and epoxidation reactions. Furthermore, the enantioselective transformation of synthetic chemical or natural product-derived compound collections can be utilized for compound library synthesis, e.g. for screening purposes in drug discovery.

Also described herein is an oligo- or polynucleotide comprising or consisting of at least 10 contiguous nucleotides in length which specifically hybridize(s) to a portion of the nucleic acid molecule of the invention, wherein said portion comprises at least one of the mutations in the reductase domain in accordance with the present invention and preferably one of the mutations specifically mentioned herein above by reference to a nucleotide or amino acid position. In other terms, at least one nucleotide of the oligo-or polynucleotide pairs by hybridisation with a nucleotide of the nucleic acid molecule encoding the monooxygenase of the invention which constitutes a mutated nucleotide.

In the context of the present invention the term "oligo- or polynucleotide" refers to nucleic acid molecules of different length: an oligonucleotide is a nucleic acid molecule consisting of up to 30 bp, a polynucleotide is a nucleic acid molecule consisting of more than 30 bp. The oligo- or polynucleotides specifically hybridize(s) to the nucleic acid molecule of the invention. In other words, they only hybridise to the nucleic acid molecule of the invention but do not cross-hybridise to the parent enzymes, such as for example the nucleic acid molecule encoding the polypeptide of SEQ ID NO:1 (i.e. SEQ ID NO:3) or the wild type P450 monooxygenase BM3 (SEQ ID NO:16). In order to specifically hybridise, the oligo- or polynucleotides hybridise to at least one sequence comprising a mutation in accordance with the invention. Also described herein is a set of oligo- or polynucleotides, such as for example a set of at least two oligo- or polynucleotides, such as at least three oligo- or polynucleotides, such as at least four oligo- or polynucleotides, such as at least five oligo- or polynucleotides or such as at least 10 oligo- or polynucleotides.

The term "hybridises/hybridising" as used herein refers to a pairing of an oligo- or polynucleotide to a complementary strand of this oligo- or polynucleotide, which thereby form a hybrid.

It is well known in the art how to perform hybridisation experiments with nucleic acid molecules. Correspondingly,

the person skilled in the art knows what hybridisation conditions she/he has to use to allow for a successful hybridization. The establishment of suitable hybridisation conditions is referred to in standard text books such as Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985).

"Stringent conditions" refers to hybridisation conditions under which the oligo- or polynucleotides that are capable of hybridizing to the polynucleotides of the invention or parts thereof do not cross hybridise to unrelated polynucleotides. Appropriate stringent hybridisation conditions for each nucleic acid sequence may be established by a person skilled in the art on well-known parameters such as temperature, composition of the nucleic acid molecules, salt conditions etc.; see, for example, Sambrook et al., "Molecular Cloning, A Laboratory Manual"; CSH Press, Cold Spring Harbor, 1989 or Higgins and Hames (eds.), loc. cit. , see in particular the chapter "Hybridization Strategy" by Britten & Davidson, 3 to 15. Such conditions comprise, e.g. an overnight incubation at 65°C in 4x SSC (600 mM NaCl, 60 mM sodium citrate) followed by washing at 65°C in 0.1x SSC for one hour. Alternatively, hybridisation conditions can comprise: an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 μg/ml denatured, sheared salmon sperm DNA, followed by washing in e.g. 0.1-0.5x SSC at about 55-65°C for about 5 to 20 min. Said conditions for hybridisation are also known by a person skilled in the art as "highly stringent conditions for hybridization". Changes in the stringency of hybridisation are primarily accomplished through the manipulation of formamide concentration (lower percentages of formamide result in lowered stringency), salt conditions, or temperature. It is of note that variations in the above conditions may be accomplished through the inclusion and/or substitution of alternate blocking reagents. Typical blocking reagents include Denhardt's reagent, BLOTTO, heparin, denatured salmon sperm DNA, and commercially available proprietary formulations. The inclusion of specific blocking reagents may require modification of the hybridisation conditions described above, due to problems with compatibility. Such modifications can generally be effected by the skilled person without further ado. The embodiment recited herein above preferably refers to highly stringent conditions.

A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed).

These oligo- or polynucleotides may, for example, be useful as probes in Northern or Southern Blot analysis of RNA or DNA preparations, respectively, or can be used as oligonucleotide primers in PCR analysis dependent on their respective size. Also comprised are hybridising oligo- or polynucleotides which are useful for analysing DNA-Protein interactions via, e.g., electrophoretic mobility shift analysis (EMSA). Preferably, said hybridising oligo- or polynucleotides comprise at least 10, such as at least 11, such as at least 12, such as at least 13, such as at least 14, such as at least 15, such as at least 16, such as at least 17 nucleotides, but may also comprise at least 19, 25, 50, 100, 150, 200, 500 or more nucleotides, whereas oligonucleotides of a length lying between these values but not mentioned are explicitly encompassed as well. Preferably, the mutations of the present invention have a central location in said oligo- or polynucleotides. "Central" meaning most preferably that an equal number of nucleotides is located in the 5'- and 3' -direction adjacent to the position of the mutation. In a different preferred embodiment 60, 70, 80 or 90 percent of nucleotides are located in the 5'- or 3'-direction adjacent to the position of the mutation and the remaining nucleotides are located in the opposite direction.

The person skilled in the art is familiar with the preparation and the use of said probes (see, e.g., Sambrook and Russel "Molecular Cloning, A Laboratory Manual", Cold Spring Harbor Laboratory, N.Y. (2001)). Said nucleic acid molecules may be chemically synthesized or transcribed by an appropriate vector containing a chimeric gene which allows for the transcription of said nucleic acid molecule in the cell.

The present invention further relates to a composition comprising the nucleic acid molecule of the invention, or the vector of the invention or the host of the invention or the polypeptide of the invention. Also described herein is a composition comprising the oligo- or polynucleotide described above.

The term "composition", as used in accordance with the present invention, relates to a composition which comprises at least one mutant polypeptide of the invention. It may, optionally, comprise furthermore excipients, additives and/or adjuvants. Examples of additional components include surfactants, such as for example anionic, non-ionic, amphoteric and/or cationic surfactants. The composition may optionally comprise further molecules capable of altering the characteristics of the mutant polypeptide of the invention thereby, for example, reducing, stabilizing, delaying, modulating and/or activating their function. Furthermore, the composition may comprise a plurality of different mutant polypeptides of the invention.

Also described herein is a kit comprising the nucleic acid molecule, or the vector, or the host, or the polypeptide of the invention, or the oligo- or polynucleotide described above.

The various components of the kit may be packaged in one or more containers such as one or more vials. The containers or vials may, in addition to the components, comprise preservatives or buffers for storage.

Preferably, the nucleic acid molecule of the invention or the polypeptide of the invention or the oligo- or polynucleotides described above are in isolated form, i.e. they are an isolated nucleic acid molecule or an isolated polypeptide or isolated oligo- or polynucleotides. The term "isolated" as used herein refers to nucleic acid molecules, polypeptides or oligo- or polynucleotides removed from their original environment (e.g., the natural environment if it is naturally occurring, or a host cell if expressed exogenously), and thus is altered "by the hand of man" from its original environment. An isolated nucleic acid molecule, polypeptide or oligo-or polynucleotide generally is provided with fewer non-nucleic acid or non-proteinaceous components (e.g. lipids) than the amount of components present in a source sample.

Unless otherwise defined, all technical and scientific terms used herein have the same meaning as commonly understood by one of ordinary skill in the art to which this invention belongs. In case of conflict, the patent specification, including definitions, will prevail.

The present invention will be illustrated by the following experiment description and taking into consideration the appended figures.

The figures show:

Figure 1: Schematic representation of the screening procedure and the enzyme reaction catalysed by P450 BM3

Figure 2: SDS-PAGE comparison of Cyt P450 BM3 purified by different ion-exchange methods (lanes 2 to 4). Lane 5 is a non purified sample (cell homogenate) while lane 1 represents molecular weight markers (Fermentas).

Figure 3: Diagram of the improved enzymatic efficacies of the muteins expressed as Kcat/Km. A) with BCC as substrate, B) with BCC acid as substrate, and C) with DBCC as the substrate.

**Figure 4:** Sequence alignment of wild type P450 BM3 holoenzyme (SEQ ID NO: 2) and reductase domain (SEQ ID NO: 1); the reductase domain starts at position 472. Positions of mutations of the present invention within the reductase domain are indicated by bold characters and the preferred amino acid exchange is shown underneath the respective position.

## Examples

### Example 1: General methods and materials

#### 1.1 Library preparation and library screening using flow cytometry in double emulsions.

As a PCR template for P450 BM3 gene and library and library amplification pCWORI vector harbouring F87A/R471C variant of BM3 was used (Prof. Frances Arnold, Caltech, Pasadena, USA). All libraries were constructed in pALXtreme-1a vector (pET-28a(+) derivative where 63 % of the sequence have been deleted and *lacI* gene has been transferred to the genome of BL21-Gold (DE3) under the control of Q1 promoter (Dr. Alexander Schenk, Jacobs University Bremen, Bremen, Germany). For plasmid isolation, constructs were transformed in *E. coli* XL10-Gold (Invitrogen, Karlsruhe, Germany) and for protein expression in BL21-Gold (DE3) $lacI^{Q1}$ (Dr. Alexander Schenk). Cells were normally grown in $LB_{Kan}$ media (50 μg/ml) unless stated otherwise.

Substrates were synthesized as described below.

The assay in the emulsion was assembled as described below. Concentrated Screening master mix was prepared by mixing 25 μl isocitric acid (80 mM), 25 μl NADPH (10 mM), 12.5 μl isocitrate dehydrogenase (0.5 U/μl) and 5 μl of fluorescein (500 μM). Cell suspension (40 μl) in buffer (0.1 M Tris-HCl pH 8.0) was mixed with concentrated Screening mix (13 μl), vortexed shortly and emulsified (see below).

The assay in the 96-well microtiter plate format (MTP) was performed using Corning (Hagen, Germany) 96-well black flat bottom plates. Cells expressing P450 BM3 variants were grown directly in the MTP, centrifuged and resuspended in 100 μl of assay-mix (100 mM phosphate buffer pH 9.0, 54 μM PMB, 75 μM BCC Acid and 250 μM NADPH). Fluorescence was monitored using TECAN Safire (Crailsheim, Germany) MTP spectrofluorimeter (excitation: 400 nm, emission: 440 nm, gain: 55, z-position: 5000 μm, integration time: 40 μs and number of light flashes: 5).

The P450 BM3 gene was amplified in error prone PCR (epPCR) conditions using balanced dNTPs, addition of $Mn^{2+}$ ions, decreasing the amount of template DNA and increasing the number of cycles (Cadwell, R.C. and Joyce, G.F. (1992) Randomization of genes by PCR mutagenesis. PCR Methods Appl, 2, 28-33). All PCRs were done in 50 μl volume using thin-wall PCR tubes (Sarstedt, Germany) and Eppendorf Gradient Cycler (Darmstadt, Germany). Reactions consisted of 1X PCR buffer pH 9.2 (50 mM tris-HCl, 16 mM ammonium-sulphate, 1.75 mM magnesium-chloride and 0.1% Tween 20), dNTPs (0.2 mM each), forward and reverse primer (400 nM each, forward primer: 5'-A*C*C*A*T*G*G*G*C*A*G*C*ATGACAATTAAAGAAATGCCTCAGCCAAAAACG -3', reverse primer: 5'-G*G*C*T*T*T*G*T*T*A*G*C*TTACCCAGCCCACACGTCTTTTGCGTATC -3', (asterisks mark positions of phospho-

thioester bond), $MnCl_2$ (0.2 mM), *Taq* polymerase (5 U) and template DNA (25 ng). Cycling was done as follows: 1 cycle of initial denaturation (94°C, 2 min), 35 cycles consisting of denaturation (94°C, 30 sec), annealing (67°C, 30 sec), elongation (72°C, 2 min) and 1 cycle of final elongation (72°C, 5 min). After PCR sample was purified using PCR purification kit (Qiagen, Hilden, Germany) and quantified via Nano Drop (ND-1000, Nano Drop Technologies, Delaware, USA). Gene was cloned in pALXtreme-1a vector using Phosphorothioate based Ligase Independent Gene cloning system (PIGe) and transformed in XL10-Gold cells. Several tubes of transformed cells were pooled and grown together in liquid $LB_{Kan}$ media (4 ml, 37°C, 12 h, 250 rpm). Small aliquot (5 μl) of transformation mixture was plated on $LB_{Kan}$ agar plates to determine transformation efficiency and size of primary library. Plasmid was recovered using Plasmid Prep Kit (Qiagen, Hilden, Germany) and re-transformed to expression strain BL21-Gold (DE3) $lacI^{Q1}$.

Induction of P450 BM3 variants was done by inoculating cells in $LB_{Kan}$ media (4 ml) and growing them for 2 hours (37°C, 250 rpm). Then IPTG (0.5 mM) was added and expression was continued (30°C, 250 rpm) for an additional 3 hours. After this, cells were centrifuged (5900 x g, 3 min) and washed twice with ice cold PBS. Finally cells were re-suspended in activity buffer (0.1 mM tris pH 8.0) in concentration of $5 \times 10^6$ cell/μl. Prior to emulsification, cell suspension was passed trough a 5 μm filter. Cell suspension was mixed with concentrated screening master mix (25 μl isocitric acid (80 mM), 25 μl NADPH (10 mM), 12.5 μl isocitrate dehydrogenase (0.5 U/μl) and 5 μl of fluorescein (500 μM)).

Emulsification was done as described previously (Miller, O.J., Bernath, K., Agresti, J.J., Amitai, G., Kelly, B.T., Mastrobattista, E., Taly, V., Magdassi, S., Tawfik, D.S. and Griffiths, A.D. (2006) Directed evolution by in vitro compartmentalization. Nat Methods, 3, 561-570) with decreasing the final volume of emulsion to 525 μl and using a Miccra D-1 (ART, Müllheim, Germany) homogeniser. After preparation of primary emulsion, substrate was added (1 μl of 200 mM BCC Acid in DMSO) and secondary emulsion was prepared immediately after. Emulsion was incubated on room temperature (2 h), in dark. For visualization of integrity of secondary emulsion fluorescent microscopy was used (Keyence BZ-8000, Neu-Isenburg, Germany, mercury lamp excitation, blue (450±20 nm) and green (520±20 nm) filters for emission).

For analysis and sorting, the emulsion was diluted 100 times in sterile PBS. Sample was run with speed 5 μl/min trough Partec CyFlow Space flow cytometer (Partec, Muenster, Germany). Sheet fluid (0.9% NaCl, 0.01 Triton X-100 in Milli-Q water) was filtered and autoclaved before use. Detection was "triggered" on green fluorescence (excitation: 488 nm, emission: 530 nm) coming from fluorescein used as an internal control dye. Analysis speed was approx. 6000-7000 events/sec while sorting speed was 5-10 events/sec. For sorting ~0.1-0.01% of active population was chosen and sorted in approx. 50 ml volume.

The sorted sample was passed trough a 0.2 μm filter, recovered in 1 ml of $LB_{Kan}$ media with shaking (1400 rpm, 10 min) and then inoculated in $LB_{Kan}$ media (4 ml). For the next round of enrichment, this was used as a pre-culture sample. Enrichment/sorting was repeated 3 times.

After each round, 100 μl of sorted sample was plated on $LB_{Kan}$ agar plates with IPTG (5 μM) for activity assay and cell quantification. Cells were grown overnight (37°C, 16 h). Activity assay was done by picking colony from the agar plate directly in 50 μl Assay mix (100 mM phosphate buffer pH 9.0, 54 μM PMB, 75 μM substrate (BCC, BCC acid or DBCC, respectively) and 250 μM NADPH)) in black flat bottom 386-well MTP. Fluorescence (excitation: 400 nm, emission: 440 nm) was monitored using TECAN Safire (Crailsheim, Germany) MTP spectrofluorimeter in 60 min periods (gain: 80, z-position: 8500 μm, integration time: 40 μs and number of light flashes: 10).

Finally, after the flow cytometry enrichment steps, cells were grown on $LB_{Kan}$ agar plates (37°C, 16 h) and picked into 150 μl $LB_{Kan}$ media in flat bottom transparent 96-well MTP using sterile toothpicks. The suspension was grown (37°C, 900 rpm, 70% humidity, 16 h), diluted with glycerol (25% final) and stored on -80°C (Master plate). Replica of Master plate for activity testing was made in 150 μl $LB_{Kan}$ media with supplements (0.5 mM δ-aminolevulinic acid, 0.5 mM thiamine, 1 x trace elements and 5 μM IPTG) in flat bottom black 96-well MTP and grown (30°C, 700 rpm, 70% humidity) for another 12 h. Plates were centrifuged (3200 x g, 10 min, 4°C) and the assay was done as described above.

The most active clones (including the starting clone M0) were selected from MTP screening and inoculated in 4 ml $LB_{Kan}$ media. Four milliliters of pre-culture was transferred to 250 ml $TB_{Kan}$ media (in 1 l flask) with supplements (0.5 mM δ-aminolevulinic acid, 0.5 mM thiamine and 1 x trace elements), grown (37°C, 250 rpm) for 2 hours and then induced by addition of IPTG (0.5 mM). Protein was expressed (30°C, 250 rpm) for 8 hours. After expression, cell suspension was kept on ice. Expressed cells were centrifuged (3200 x g, 10 min, 4°C), re-suspended in 15 ml lysis buffer (0.01 M tris pH 7.8) and passed trough French press (3 times, 1500 bars). Cell lysate was cleared by centrifugation (21000 x g, 10 min, 4°C) and filtering (0.45 μm). Protein was purified using ion-exchange chromatography as follows: a sample (10 ml) was loaded on DEAE-ion exchange column equilibrated in 100 mM tris-HCl pH 7.8 (buffer A). The flow was kept constant (5 ml/min). Detection was absorbance set up for total proteins (280 nm) and for heme proteins (417 nm). The unbound sample was washed out with 2 CV of buffer A. A gradient of buffer B (100 mM tris-HCl pH 7.8 with 2 M NaCl) was adjusted to 5 % and loosely bound proteins were washed out (2 CV). Then a linear gradient of 5-20 % was set up in 5 CV. Cyt P450 BM3 was eluted in this region. After 20 % B gradient was adjusted to 100 % B in 2 CV and washing of the column (100 % B) was continued for another 2 CV. At the end, column was washed with buffer A, then with Milli-Q water and kept in 20 % ethanol. The peak corresponding to P450 BM3 was pooled (10 ml) and the

concentration of protein was determined using CO binding method (Omura, T. and Sato, R. (1964) The Carbon Monoxide-Binding Pigment Of Liver Microsomes. I. Evidence For Its Hemoprotein Nature. J Biol Chem, 239, 2370-2378).The purity of the samples was confirmed by SDS-PAGE.

Kinetic characterization was done in black flat bottom 96-well MTP in a final volume of 120 μl. Enzyme fractions were diluted 10-15 times in PBS and kept on ice. Assay was assembled as follows: 100 μl 0.1 M phosphate buffer pH 9.0, 10 μl enzyme solution and 2 μl of substrate. After incubation with shaking (750 rpm, 5 min), 8 μl of NADPH (10 mM) was added. Fluorescence (excitation: 400 nm, emission: 440 nm) was monitored for 10 minutes (1 min interval, gain: 80, z-position: 5500 μm, integration time: 40 μs and number of light flashes: 10). Slope (AU/min) was calculated in the first 5 minutes of the reaction (linear range) using Microsoft Excel. Concentration of the product was calculated form the standard curve constructed for 3-carboxy coumarin (10-325 nM). For determination of standard deviation, 5 repetitions were done for each substrate concentration. For $K_m$ and $k_{cat}$ determination, data was plotted and fitted using Origin 7.0 (OriginLab Corporation, Northampton, USA).

Sequencing of selected variants was done with MWG (Ebersberg, Germany). The sequence was analyzed using Vector NTI (Invitrogen, Karlsruhe, Germany).

### 1.2 Enzyme expression and purification

All variants are expressed using BL21-Gold (DE3) $lacI^{Q1}$ expression strain and pALXtreme vector. Pre-culture was inoculated from glycerol stock directly into 4 mL $LB_{Kan}$ media and grown (37°C, 250 rpm) for 16 h. Aliquot of pre-culture (500 μL) was transferred to 250 mL $LB_{Kan}$ supplemented media (0.5 mM aminolevulinic acid, 0.5 mM thiamine, 1X trace elements and 5 μM IPTG). Variants were expressed (30°C, 250 rpm) for 14-16 hours.

After the expression phase, the cell suspension was centrifuged (3200 x g, 10 min, 4°C). The pellet was re-suspended in lysis buffer (15 mL of 10 mM tris-HCl pH 7.8) and lysed using French press (3 passes, 1500 bar). Cell homogenate was cleared out by filtration (21000 x g, 10 min, 4°C) and filtration (0.45 μm Millipore filter).

P450 BM3 has previously been purified using DEAE ion-exchange chromatography with step elution of salt as described in Schwaneberg et. al 1999, Fig.2 lane 2 and 3. For the purification of the mutants of the present invention we developed a slightly modified the protocol in a way that a gradient elution was used. Lane 4 in fig. 2 shows the result of this modification of this protocol using a gradient elution thep instead of a block gradient for elution. In detail, the filtered crude extract of recombinant E. coli was loaded on DEAE-ion exchange column equilibrated in 100 mM tris-HCl pH 7.8 (buffer A). The flow was kept constant (5 ml/min). Detection was accomplished by measuring the absorbance set up for total proteins (280 nm) and for heme proteins (417 nm). Unbound samples were washed out with 2 column volumes (CV) of buffer A. A gradient of buffer B (100 mM tris-HCl pH 7.8 with 2 M NaCl) was adjusted to 5 % and loosely bound proteins were washed out (2 CV). Then a linear gradient 5-20 % B was set up in 5 CV. Cyt P450 BM3 was eluted in this region, well separated from contaminating proteins. After 20 % B the gradient was adjusted to 100 % B in 2 CV and washing of the column (100 % B) was continued for another 2 CV. At the end, column was washed with buffer A, then with Milli-Q water and kept in 20 % ethanol.

### Example 2: Selection of the Parent Mutants

The wild type sequence of Cyt P450 BM3 has been deposited in GenBank/EMBL databank with the accession number J04832 or AAA87602.1, respectively..

The P450 BM-3 single mutant F87A was used as starting template. This mutation was disclosed elsewhere (e.g. EP 1196603 B1, EP 1196545 B1, EP 1196605 B1, US 7531335).

Other parent enzymes, designated Dm1 (R47F/F87A/R471 C) and Dm2#4 (F87A/M354S/R471C), comprise additional mutation sites which were previously described (Wong, T.S., Arnold, F.H., and Schwaneberg, U., 2004, Laboratory evolution of cytochrome P450 BM-3 monooxygenase for organic cosolvents. Biotechnology and Bioengineering 85, 3: 351-358) and show a significantly improved enzymatic efficiency as compared to the wild type.

The position 354 was exchanged from Met to Ser and this mutation has already been shown to affect enzyme activity towards different substrates (Nazor, J., Dannenmann, S., Adjei, R.O., Fordjour, Y.B., Ghampson, I.T., Blanusa, M., Roccatano, D. and Schwaneberg, U. (2008) Laboratory evolution of P450 BM3 for mediated electron transfer yielding an activity-improved and reductase-independent variant. Protein Eng Des Sel, 21, 29-35).

As is shown in Tab. 1 to 3 and was disclosed previously, these mutations in the parent enzymes have a dramatic effect on the catalytic efficiency (Kcat/Km) with all three substrates used for the kinetic characterization.

### Example 3: Enzyme expression and purification

All variants were expressed using BL21-Gold (DE3) $lacI^{Q1}$ expression strain and pALXtreme vector. Pre-culture was inoculated from glycerol stock directly into 4 mL $LB_{Kan}$ media and grown (37°C, 250 rpm) for 16 h. Aliquot of pre-

culture (500 μL) was transferred to 250 mL $LB_{Kan}$ supplemented media (0.5 mM aminolevulinic acid, 0.5 mM thiamine, 1X trace elements and 5 μM IPTG). Variants were expressed (30°C, 250 rpm) for 14-16 hours.

After the expression phase, the cell suspension was centrifuged (3200 x g, 10 min, 4°C). Pellet was re-suspended in lysis buffer (15 mL of 10 mM tris-HCl pH 7.8) and lysed using French press (3 passes, 1500 bar). Cell homogenate was cleared out by filtration (21000 x g, 10 min, 4°C) and filtration (0.45 μm Millipore filter).

Cyt P450 BM3 has previously been purified using DEAE ion-exchange chromatography with step elution of salt. We slightly modified the protocol in a way that gradient elution was used. First, loosely bound proteins were washed out with 5 % buffer B in 2 CV. Cyt P450 BM3 is was eluted in ~10 ml of elution buffer, at linear gradient of buffer B (5-20 % B), well separated from other contaminating proteins.

Purity of the protein was estimated to 90-95 % using SDS-PAGE (Figure 32). The result was confirmed by comparing concentrations of total proteins (determined with BCA method) and concentration of Cyt P450 BM3 (determined with CO binding method). After chromatography the protein purity was sufficient for kinetic characterization. Sequencing of selected variants was done with MWG (Ebersberg, Germany). The sequence was analyzed using Vector NTI (Invitrogen, Karlsruhe, Germany).

**Example 4: Determination of the kinetic parameters of the mutants and comparison with the respective parent and the wild-type enzyme**

Kinetic characterization was done in black flat bottom 96-well MTP (Corning) in final volume of 120 μl. Enzyme fractions were diluted 5 times in PBS and kept on ice. Assay was assembled as follows: 100 μl 0.1 M phosphate buffer pH 9.0, 10 μl enzyme solution and 2 μl of substrate dilution (0.24-125 μM, 10 concentrations). After incubation with shaking (750 rpm, 5 min) 8 μl of NADPH (10 mM) was added. Fluorescence (excitation: 400 nm, emission: 440 nm) was monitored for 10 minutes (1 min interval, gain: 80, z-position: 5500 μm, integration time: 40 μs and number of light flashes: 10). Slope (AU/min) was calculated in first 5 minutes of the reaction (linear range) using Microsoft Excel. Concentration of the product was calculated form the standard curve constructed for 3-carboxy coumarin (10-325 nM). For determination of standard deviation 3 repetitions were done for each substrate concentration. For $K_m$ and $k_{cat}$ determination data was plotted and fitted using Origin 7.0 (OriginLab Corporation, Northampton, USA).

**4.1 Substrates for screening for monooxygenase activity**

All substrates used for the kinetic characterization of the mutants comprise a coumarine "core" which is derivatized at position 7 and 3. After the conversion by P450 monooxygenase 3-carboxy coumarin, or its derivates, are released. This leads to an increase in specific fluorescence (excitation 370-400 nm, emission 440-460 nm).

**The BCC** substrate has a 7-hydroxy group derivatized with a benzyl group (bound with an ether bond). Hydroxylation at the αC atom of the benzyl group leads to fluorophore release. The carboxy group in position 3 is esterified with a methyl group so this substrates has no charge. **BCC acid** has a very similar structure to the previously mentioned BCC except that it possesses a negative charge due to chemically de-esterified carboxy group in position 3. This substrate is of interest since P450 BM3 rarely has activity towards drug-like molecules containing charge. **DBCC** has two benzyl groups bound in position 7 and 3 with an ether and an ester bond, respectively. This makes this substrate the bulkiest one within the three substrates used in the present invention.

4.1.1 Substrate preparation

The substrate 7-benzoxy-3-carboxy coumarin was synthesized starting from 3-carboxy coumarin methyl ester. In detail, synthesis was as described in the following.

Synthesis and characterization of 12-(4-methyl umbelliferone)-dodecanoic acid and methyl ester

**Step 1** - protection of carboxylic group of fatty acid by esterification. For this step, 1.275 g (4.57 mmol) of 12-bromo-dodecanoic acid was dissolved in 20 ml dry methanol. Next, 100 μl of concentrated $H_2SO_4$ was added. The reaction was incubated at 75°C for 3 hours. The process was monitored by TLC using petrol ether: ethyl acetate (2:1) as a developing solution. For visualization phosphomolybdic acid system was used.

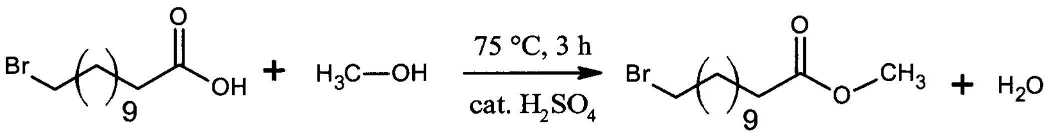


After the reaction was completed, the sample was evaporated using a Rotavap at 40°C for 10-15 minutes. The

pellet was dissolved in $CH_2Cl_2$ and extracted twice with 10 ml of saturated $KHCO_3$, once with distilled water and once with brine. The organic phase was separated, dried with $MgSO_4$, filtered and evaporated using Rotavap.

**Step 2** - Conversion of 4-MU into sodium salt. For this step, 0.80 g (4.54 mmol) of 4-MU was dissolved in 15 ml dry methanol. 0.18 g (4.50 mmol) of NaOH was added. The reaction was mixed with magnetic stirrer until NaOH completely dissolved. At this point, solution changed color to bright yellow. Methanol was evaporated using Rotavap.

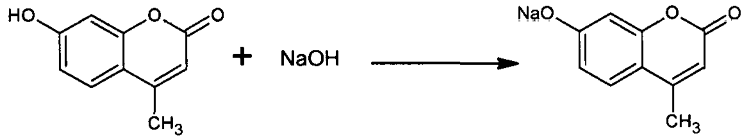

**Step 3** - Attaching of fluorescent probe (4-MU) to fatty acid. The previously prepared sodium salt of 4-MU was dissolved in 15 ml DMSO and dried shortly (10 min) on 120°C using an oil bath. Then methyl ester of 12-bromo-dodecanoic acid was added (dissolved in 15 ml DMSO). The mixture was stirred at 160°C for 3 hours. Progress was monitored by TLC.

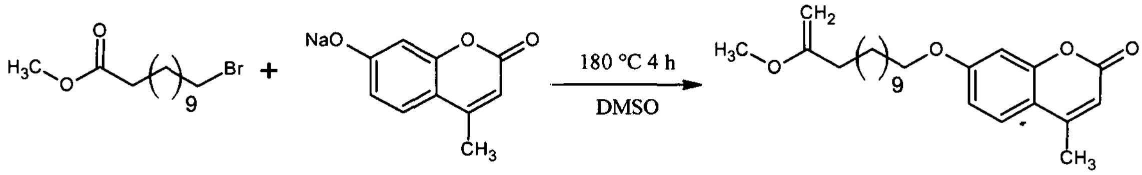


After the reaction the mixture was poured into 200 ml of ice cold distilled water and a white precipitate formed immediately. After 10-15 minutes the mixture was centrifuged (4000 rpm, 5 min). The pellet was dissolved in $CH_2Cl_2$, extracted twice with saturated $NaHCO_3$ and twice with distilled water. The organic layer was dried with $MgSO_4$, filtered and evaporated using Rotavap.

**Step 4:** De-esterification of the substrate - release of carboxyl group. The pellet from the previous step was dissolved in 25 ml potassium-metoxide (prepared by dissolving 0.8 g (20.46 mmol) of KOH in 5 ml of water and then filling it up to 50 ml with methanol). The reaction was incubated for 1 hour at 80°C. The sample was cooled down to room temperature and poured in ~100 ml ice cold water (pH adjusted to ~1 with HCl). The formed precipitate was filtered and dried over night under the vacuum.

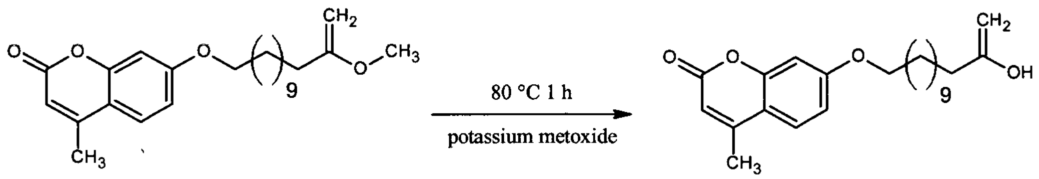


For characterization by NMR, approx. 10 mg of both samples, ester and acid, were dissolved in 1 ml of $CDCl_3$. $^{13}C$ and $^{1}H$ NMR spectra were recorded using a 400 MHz NMR (JOEL ECX 400, Peabody, USA). For fluorescent spectra characterization a stock solution of substrate was prepared in DMSO (15 mM). Dilutions of stock solution were made in buffer and 3D fluorescent spectra were recorded using TECAN Safire (Switzerland).

**Conversion of 12-(4-MU)-dodecanoic acid and methyl ester**

To test the possibility of conversion of novel coumarin/fatty acid compounds by Cyt P450 BM3 the soluble purified enzyme was used. Activity was tested with Cyt P450 BM3 wild-type (Wt) and selected variants (F87A, F87A/R47Y, F87A/R47Y/M354S and Y51 F).

The assay was assembled as follows: 230 μl of buffer (50 mM phosphate, 50 mM tris-HCl pH 8.0 0.25 KCl), 5 μl substrate (15 mM in DMSO) and 10 μl soluble enzyme. Reaction was incubated for 5 minutes at room temperature. Conversion was initiated by addition of 20 μl NADPH (5 mM). Fluorescence was monitored using TECAN Safire (ex. 310 and 380 nm, em. 390 and 440 nm, respectively) in black flat bottom 96-well MTPs (Greiner Bio-One). After reaction, 3 μl of each sample was analyzed by TLC (petrol ether: ethyl acetate = 1: 1). Visualization was by UV light (254 and 366 nm).

**Synthesis and characterization of 7-benzoxy-4-MU**

First, 0.5 g (2.84 mmol) of 4-MU was dissolved in 20 ml DMSO with stirring. 600 µl (3.51 mmol) of benzyl bromide was added together with catalytic amount of NaOH and mixture was refluxed at 100°C for 2 hours. After the reaction mixture was poured into ice cold water (~200 ml) and left over night. Precipitate was separated by filtration and dried in vacuum. Dry pellet was re-dissolved in $CH_2Cl_2$. Chromatography was done on silica gel column using petrol ether: ethyl acetate (1:1) for elution. Fractions with target compound are pooled, solvent evaporated on Rotavap and pellet dried in vacuum, overnight.

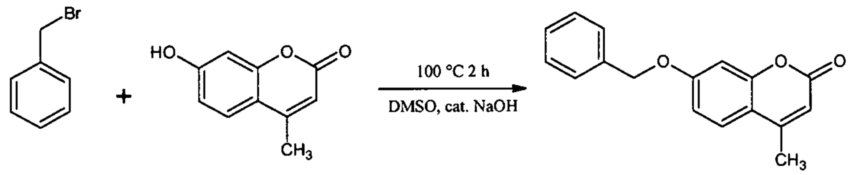


For NMR characterization 5 mg of purified compound was dissolved in 1 ml $CDCl_3$. $^{13}C$ and NMR spectra were recorded using a 400 MHz NMR (JOEL ECX 400, Peabody, USA).

**Conversion of 7-benzoxy-4-MU**

To test the conversion of coumarin/benzyl compound (crude) with Cyt P450 BM3, reaction was assembled as follows: 100 µl buffer (50 mM phosphate, 50 mM tris-HCl pH 8.0, 0.25 mM KCl), 2.5 µl substrate (15 mM in DMSO) and 10 µl enzyme. After 5 minutes incubation on room temperature reaction was initiated with addition of 10 µl NADPH (10 mM). Conversion was done 1 hour at room temperature. Reaction products were analyzed by TLC using petrol ether: ethyl acetate (1:1) as a developer. Visualization was done under UV light (366 nm). Activity was tested for both, Cyt P450 BM3 wild-type (Wt) and selected variants (F87A, F87A/R47Y, F87A/R47Y/M354S and Y51 F).
For kinetic testing assay was assembled as described in the paragraph above. Fluorescence was monitored using TECAN Safire (ex. 380 nm, em. 440 nm, gain 50) in 60 minutes period (5 minutes interval).
To test applicability of NADPH-recycling system, assay was assembled as described in the paragraph above. Additionally, 5 µl of isocitric acid (80 mM) and 10 µL of isocitrate dehydrogenase (0.01 U/µL) were added in reaction mix. The reaction was initiated with addition of 2.5 µl NADPH (5 mM). Fluorescence was monitored using TECAN Safire (ex. 380 nm, em. 440 nm, gain 50) in 60 minutes period (5 minutes interval).
The influence of the substrate concentration on the conversion reaction was tested in the following experiment. The reaction mix was assembled as follows: 100 µl buffer (50mM phosphate, 50mM tris-HCl pH 8.0, 0.25 KCl), 2.5µl substrate (different concentrations in DMSO), 10µl enzyme (Wt, 11µM), 5µl isocitric acid (80mM), 10µl isocitric dehydrogenase (0.01U/µl). The reaction mix was incubated 5 minutes at room temperature. The reaction was initiated by addition of 2.5µl of NADPH (5mM). Concentration of the substrate was ranging from 2.9mM to 23µM (in serial dilution by a factor of two). Fluorescence was monitored using TECAN Safire (ex. 380 nm, em. 440 nm, gain 50) in 160 minutes period (10 minutes interval). $V_0$ was calculated for first 30 minutes of reaction (linear part).

**Synthesis and characterization of substrates for Cyt P450 BM3 using 3-carboxy coumarin (3-CC) as a fluorescent probe**

**Step 1:** Synthesis of methyl ester of 3-carboxy coumarine.

8.4 g (60.82 mmol) of 2,4-dihydroxybenzaldehyde was dissolved in 45 ml of anhydrous methanol. Solution was stirred and 8.7 g (65.85 mmol) of dimethyl malonate was added. Solution was brought to reflux temperature. 450 mg (5.16 mmol) of moprholine and 150 mg (2.49 mmol) of acetic acid were added to 2 ml of methanol and stirred until precipitate fully dissolved. This solution was then added to refluxed reaction mixture and reflux was continued for another 3 hours. After cooling, the product was filtered and re-crystallized from boiling methanol (~300 ml).

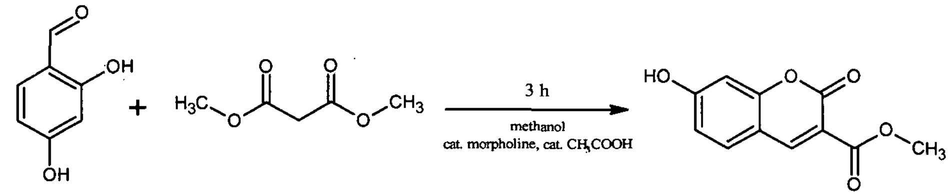


**Step 2:** Preparation of sodium salt of 3-CC.

For this step, 1.5 g (6.81 mmol) of 3-CC methyl ester was re-suspended in 50 ml of toluene, with stirring, and heated at 120°C until 5 ml of toluene evaporated (30-60 minutes). After cooling the mixture to room temperature 0.5 g (10.84 mmol) of NaH was added. Mixture was heated at 120°C and stirred until toluene evaporated (1-2 hours). The obtained salt was dried in vacuum overnight.

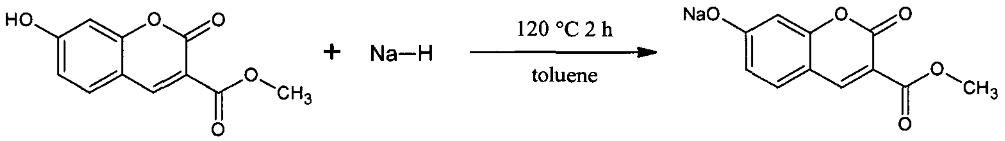


**Step 3:** Attaching benzyl group to 3-CC methyl ester.

3 g (12.39 mmol) of prepared 3-CC methyl ester sodium salt was dissolved in 200 ml of DMF (dried with molecular sieves). Mixture was heated to 120°C. During heating, 2.138 g (12.5 mmol) of benzyl bromide was added. Mixture was kept on 120°C for 2 hours with stirring. Then, an additional 1 g (5.85 mmol) of benzyl bromide was added and reaction continued at 120°C for 4-6 hours. At the end, one more batch (0.7 g, 4.09 mmol) of benzyl bromide was added. Reaction was continued for an additional hour. At the end, mixture was cooled to room temperature for a few hours and poured to 400 ml of ice cold water. After precipitate formed (30-60 min), the suspension was filtered and rinsed with water. The precipitate was dried and re-dissolved in $CH_2Cl_2$. Organic phase was extracted twice with water, filtered and evaporated on Rotavap. The precipitate was dried overnight in vacuum.

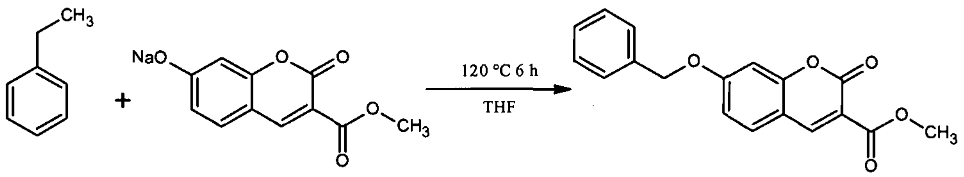


Target compounds were isolated after chromatography on silica gel. Sample was loaded in $CH_2Cl_2$. Elution was done by adding small amount of ethyl acetate into $CH_2Cl_2$ (1:20). Two main fractions were pooled according to TLC. Re-chromatography of un-pure Fraction II was done on silica gel using $CH_2Cl_2$: ethyl acetate (20:1) for elution. Purity was monitored on TLC using the same solvent system.

**Step 4:** De-esterification of methyl ester group.

50 mg (161.13 μmol) of 7-benzoxy-3-carboxy coumarin methyl ester was dissolved in 4 ml THF (kept at 4°C). At the same time, 0.07 g (1.67 mmol) of LiOH was dissolved in 4 ml of distilled water (kept at 4°C). When both components were cooled to 4°C they were mixed, stirred 1 hour on ice and left overnight in the fridge (with constant stirring). The following day, 1.66 ml of 3M HCl was added to reaction mix and left to warm up to room temperature. Finally, 3.22 ml of brine was added and organic phases separated. Reaction mix was extracted three times with 20 ml ethyl acetate. All organic phases are pooled, evaporated on Rotavap and precipitate was dried overnight under vacuum. Purity was monitored by TLC.

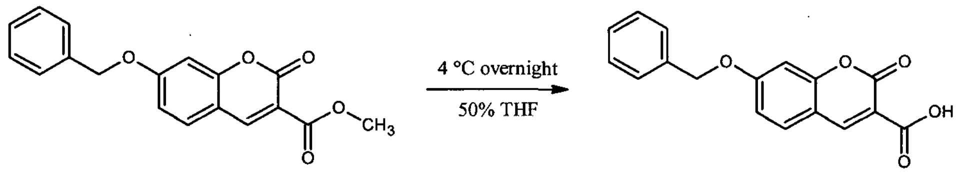


For characterization by NMR, approx. 5 mg of both samples (Fraction 12 and Fraction 24) were dissolved in 1 ml of $CDCl_3$. $^{13}C$ and $^{1}H$ NMR spectra were recorded using a 400 MHz NMR (JOEL ECX 400, Peabody, USA).

**Conversion of 3-carboxy coumarine based compounds using Cyt P450 BM3**

Conversion of substrates based on 3-carboxy coumarine was done with purified Cyt P450 BM3. Tested variants included Wt and mutants F87A, F87A/R47F, F87A/R47Y, F87A/R47F/M354S and Y51 F. The assay was assembled in black flat bottom 384-well MTPs (Greiner Bio-One) as follows: 50 μl buffer (100 mM phosphate buffer pH 9.0), 1 μl substrate (15 mM BCC and BCC Acid in DMSO, 10 mM DBCC in DMSO) and 5 μl enzyme (10 mg/ml). Reaction mix was incubated 5 minutes on room temperature. Reaction was initiated by addition of 4 μl NADPH (10 mM). Fluorescence was monitored in 1 min intervals for 40 minutes using TECAN Safire (TECAN Group, gain 70, z-position 7800). After the reaction 3 μl of each reaction mix was loaded and analyzed on TLC. Visualization was done by UV light (366 nm). Reverse phase HPLC was performed to analyze reaction products of conversion of BCC and BCC Acid by the Cyt P450 BM3 F87A/R47F/M354S variant.

HPLC was performed on ÄKTA Purifier (GE Healthcare) connected to SOURCE 5RPC ST 4.6/450 column (GE Healthcare). Buffer A was 10 mM phosphate buffer pH 2.8 and buffer B was 90 % acetonitrile in 10 mM phosphate buffer pH 2.8. Flow was kept constant at 1 ml/min and detection was on 210 nm and 350 nm (specific for the coumarin structures). Sample (100 μl) was loaded in buffer A. Next, unbound sample was washed out with 2 CV of buffer A and linear gradient of buffer B (0-100 % in 10 CV) was applied. All target compounds were eluted in this region. Column was washed with 3 CV of buffer B (100 %). Four samples were prepared for each substrate and they included:

Sample 1 (100 μl) - blank: buffer + DMSO + NADPH + enzyme
Sample 2 (100 μl) - standard substrate: buffer + substrate
Sample 3 (100 μl) - standard product: buffer + 3-carboxy coumarin
Sample 4 (100 μl) - reaction mix: buffer + substrate in DMSO + NADPH + enzyme

The reaction mix was incubated 2 hours at room temperature before analysis. Fractions containing unknown reaction products were collected and analyzed by TLC and by 3D spectral fluorimetry. 3D spectra were recorded using TECAN Safire (TECAN Group) and black flat bottom 96-well MTP (Greiner Bio-One).
For optimization of different excitation/emission wavelengths an assay was set up as follows: 50 μl buffer (100 mM phosphate buffer pH 7.5), 1 μl substrate (dilution in DMSO) and 5 μl enzyme (Cyt P450 BM3 139-3). Reaction mix was incubated on room temperature for 5 minutes. The reaction was initiated by addition of 4 μl NADPH (10 mM). The assay was done in black flat bottom 384-well MTPs (Greiner Bio-One). Fluorescence was monitored using TECAN Safire on three excitation (375, 400 and 405 nm) and three emission (455, 440 and 455 nm) wavelengths, respectively. Substrate concentrations were 167, 83, 42, 21, 10 and 5.2 μM, respectively.
To test the effect of different permeabilizers (organic solvents and polymixin B sulphate - PMB) on conversion of BCC an experiment was set up as follows. *E. coli* DH5α harboring pCWORI vector with Cyt P450 BM3 Wt gene was expressed in $TB_{Amp}$ media overnight (50 ml, 0.5 mM IPTG, 37°C, 250 rpm). After expression cells were centrifuged (4000 rpm, 10 min, 4°C) and washed twice with PBS. At the end, the cell pellet was re-suspended in 10 ml of activity buffer (100 mM phosphate buffer pH 7.5) and kept on ice. This concentrated cell suspension was used in assays. Assay was carried out as described in the paragraph above using only 167 μM substrate and adding 5 μl of cell suspension instead of purified enzyme. Organic solvents (ethanol, acetone, isopropanol and toluene) were added directly to the reaction mix in a final concentration of 10 % (vol/vol). Fluorescence (ex.400 nm, em. 440 nm) was recorded for 60 minutes (1 min interval).
After testing different permeabilizers the effect of different concentrations of PMB on activity was tested. DH5α expressing Cyt P450 BM3 Wt has been used. Assay was assembled as described in the paragraph above. Different amounts (1-5 μl in 1 μl steps) of PMB stock solution (3.6 mM) have been added. Final volume of the reaction mix was kept constant by decreasing the volume of activity buffer.

To test the difference in permeabilization potential between PMB and PMBN an experiment was set up as follows. DH5α cells expressing Cyt P450 BM3 139-3 variant have been used. The assay was assembled as described previously. Five and one μl of each permeabilizer stock (3.6 mM PMB or PMBN) was added in the reaction mix. Fluorescence was monitored for 60 minutes (1 min interval).

Dilutions of the substrates were made in DMSO and kept at 4°C (in the dark).

Alternatively, the activity of P450 monooxygenases can be measured using substrates known in the art such as for example p-nitrophenoxydodecanoic acid (12-pNCA) as described in Schwaneberg, U., Schmidt-Dannert, C., Schmitt, J., Schmid, R.D., 1999, A continuous spectrophotometric assay for P450 BM-3, a fatty acid hydroxylating enzyme and its mutant F87A, Anal. Biochem. 269: 359-366).

All three substrates used herein are based on the same reporter molecule but all three have different properties (charge, bulky side groups etc). This was utilized for the directed evolution method for the discovery of new residues in Cyt P450 BM3 structure connected with specific activity and offers new options to improve its activity towards drug like molecules, charged molecules, bulky molecules etc..

**Table 2:** Structure and names of novel coumarine based substrates for screening Cyt P450 BM3 activity in MTP and double emulsions

| Structure | IUPAC name | Common name | Short name |
|---|---|---|---|
| 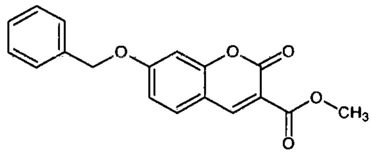 | Methyl 7-(benzyloxy)-2-oxo-2*H*-chromene-3-carboxylate | 7-benzoxy-3-carboxy coumarin methyl ester | BCC |
| 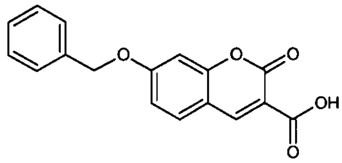 | 7-(benzyloxy)-2-oxo-2*H*-chromene-3-carboxylic acid | 7-benzoxy-3-carboxy coumarin | BCC Acid |
| 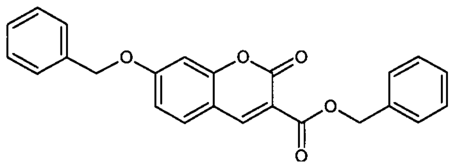 | Benzyl 7-(benzyloxy)-2-oxo-2*H*-chromene-3-carboxylate | 7-benzoxy-3-carboxy coumarin benzyl ester | DBCC |

**Results of kinetic characterization with 3 substrates**

The results of the Michaelis Menten assays with the wild type P450/BM3, the three parent muteins and the muteins of the invention applying the three different substrates are shown in Tables 3 to 5. The enzymatic activity is expressed by $K_{cat}/K_M$ [eg/min μM]. The ratio of improvement of enzymatic efficacy as compared to the respective starting mutation is shown in the columns next to the column comprising the measured $K_{cat}/K_M$ values.

**Table 3:** BCC as substrate

| Variant | Mutations | $k_{cat}/K_m$ eq/min μM | Mut/Wt | Mut/F8 A | Mut/R471 | Mut/M354 |
|---|---|---|---|---|---|---|
| WT | - | 0.0006 | **1.0** | 0.0 | 0.0 | 0.0 |
| F87A | F87A/R471C | 0.0247 | 41.2 | **1.0** | 0.2 | 0.6 |
| A3 | F87A/R471C/**M812T** | 0.0921 | 153.5 | 3.7 | 0.8 | 2.2 |
| Dm1 | R47F/F87A/R471C | 0.1142 | 190.3 | 4.6 | **1.0** | 2.7 |
| E1 | R47F/F87A/R471C/**A730V** | 0.4701 | 783.6 | 19.0 | 4.1 | 11.2 |
| E9 | R47F/F87A/R471C/**D648G** | 0.5805 | 967.5 | 23.5 | 5.1 | 13.8 |
| H3 | R47F/F87A/R471C/**Q512R** | 0.3384 | 563.9 | 13.7 | 3.0 | 8.1 |
| M1 | L29S/R47Y/F87A/Q189R/ R471C/**Y857N** | 0.2341 | 390.2 | 9.5 | 2.1 | 5.6 |

(continued)

| Variant | Mutations | $k_{cat}/K_m$ eq/min μM | Mut/Wt | Mut/F8 A | Mut/R471 | Mut/M354 |
|---|---|---|---|---|---|---|
| M2 | E13G/R47L/F87A/R471C/ **L1030S** | 0.3990 | 665.0 | 16.2 | 3.5 | 9.5 |
| **D**m2#4 | F87A/M354S/R471C | 0.0420 | 70.0 | 1.7 | 0.4 | **1.0** |
| D7 | F87A/F162L/M354S/Y429C/ R471C | 0.2652 | 442.0 | 10.7 | 2.3 | 6.3 |
| E5 | F87A/F162L/M354S/R471C/ **L798S** | 0.2167 | 361.1 | 8.8 | 1.9 | 5.2 |
| M3 | E64G/F87A/R223H/M354S/ R471C/**T883H/P884R/N951 D** | 0.5721 | 953.5 | 23.2 | 5.0 | 13.6 |

**Table 4:** BCC acid as substrate

| Variant | Mutations | $k_{cat}/K_m$ eq/min μM | Mut/Wt | Mut/F87A | Mut/R471 | Mut/M354 |
|---|---|---|---|---|---|---|
| WT | | 0.0003 | **1.0** | 0.1 | 0.0 | 0.0 |
| F87A | F87A/R471C | 0.0044 | 13.0 | **1.0** | 0.3 | 0.5 |
| A3 | F87A/R471C/**M812T** | 0.0192 | 56.4 | 4.4 | 1.2 | 2.0 |
| Dm1 | R47F/F87A/R471C | 0.0155 | 45.7 | 3.5 | **1.0** | 1.6 |
| E1 | R47F/F87A/R471C/**A730V** | 0.0290 | 85.4 | 6.6 | 1.9 | 3.0 |
| E9 | R47F/F87A/R471C/**D648G** | 0.0442 | 129.9 | 10.0 | 2.8 | 4.6 |
| H3 | R47F/F87A/R471C/**Q512R** | 0.0362 | 106.4 | 8.2 | 2.3 | 3.7 |
| M1 | L29S/R47Y/F87A/Q189R/ R471C/**Y857N** | 0.0281 | 82.6 | 6.4 | 1.8 | 2.9 |
| M2 | E13G/R47L/F87A/R471C/ **L1030S** | 0.0419 | 123.2 | 9.5 | 2.7 | 4.3 |
| Dm2# 4 | F87A/M354S/R471C | 0.0097 | 28.6 | 2.2 | 0.6 | 1.0 |
| D7 | F87A/F162L/M354S/Y429C/ R471C | 0.0728 | 214.0 | 16.5 | 4.7 | 7.3 |
| E5 | F87A/F162L/M354S/R471C/ **L798S** | 0.0446 | 131.1 | 10.1 | 2.9 | 4.5 |
| M3 | E64G/F87A/R223H/M354S/ R471C/T883H/P884R/N951 D | 0.0547 | 160.8 | 12.4 | 3.5 | 5.5 |

**Table 5:** DBCC as substrate

| Variant | Mutations | kcat/Km eq/min μM | Mut/Wt | Mut/F87A | Mut/R47 | Mut/M354 |
|---|---|---|---|---|---|---|
| WT | | 0.0037 | **1.0** | 0.2 | 0.0 | 0.1 |
| F87A | F87A/R471C | 0.0150 | 4.0 | **1.0** | 0.1 | 0.5 |
| A3 | F87A/R471C/**M812T** | 0.2022 | 54.6 | 13.5 | 1.7 | 6.3 |
| Dm1 | R47F/F87A/R471C | 0.1163 | 31.4 | 7.8 | **1.0** | 3.6 |
| E1 | R47F/F87A/R471C/**A730V** | 0.2933 | 79.3 | 19.6 | 2.5 | 9.1 |
| E9 | R47F/F87A/R471C/**D648G** | 0.5278 | 142.7 | 35.2 | 4.5 | 16.4 |
| H3 | R47F/F87A/R471C/**Q512R** | 0.3953 | 106.8 | 26.4 | 3.4 | 12.3 |

(continued)

| Variant | Mutations | kcat/Km eq/min µM | Mut/Wt | Mut/F87A | Mut/R47 | Mut/M354 |
|---|---|---|---|---|---|---|
| M1 | L29S/R47Y/F87A/Q189R/R47 1C/ **Y857N** | 0.3263 | 88.2 | 21.8 | 2.8 | 10.2 |
| M2 | E13G/R47L/F87A/R471C/ **L1030S** | 0.0455 | 12.3 | 3.0 | 0.4 | 1.4 |
| Dm2#4 | F87A/M354S/R471C | 0.0321 | 8.7 | 2.1 | 0.3 | **1.0** |
| D7 | F87A/F162L/M354S/Y429C/ R471C | 0.2234 | 60.4 | 14.9 | 1.9 | 6.8 |
| E5 | F87A/F162L/M354S/R471C/ **L798S** | 0.2319 | 62.7 | 15.5 | 2.0 | 7.0 |
| M3 | E64G/F87A/R223H/M354S/ R471C/T883H/P884R/N951D | 0.1676 | 45.3 | 11.2 | 1.4 | 5.1 |

## Claims

1. A nucleic acid molecule encoding a polypeptide having cytochrome P450 monooxygenase activity, wherein said polypeptide comprises a reductase domain that deviates by at least one and less than 70 mutations from

    (a) the reductase domain of cytochrome P450 BM3, wherein the reductase domain of cytochrome P450 BM3 is represented by SEQ ID NO:1; or
    (b) a reductase domain having at least 95% sequence identity to SEQ ID NO:1; and
    wherein at least one of said at least one mutations in the reductase domain occurs at a position in SEQ ID NO:1 corresponding to position 812 in SEQ ID NO:2 and wherein said mutation results in an increased cytochrome P450 monooxygenase activity as compared to the parent sequence comprising the reductase domain of SEQ ID NO:1,
    wherein said cytochrome P450 monooxygenase activity is selected from the group consisting of monooxygenation of saturated and/or unsaturated fatty acids, alcohols, and/or amides; conversion of long chain alcohols and/or amines, conversion of lauric, myristic, and/or palmitic acids to omega-1, omega-2, and/or omega-3 hydroxy analogues and hydroxylation of coumarine based substrates.

2. The nucleic acid molecule of claim 1, wherein
met at a position in SEQ ID NO:1 corresponding to position 812 in SEQ ID NO:2 is replaced by thr, ser, cys, asn, gln, asp or glu.

3. The nucleic acid molecule of claim 2, wherein
met at a position in SEQ ID NO:1 corresponding to position 812 in SEQ ID NO:2 is replaced by thr.

4. The nucleic acid molecule according to any one of claims 1 to 3, wherein the polypeptide having cytochrome P450 monooxygenase activity deviates by at least one further mutation from cytochrome P450 BM3, wherein cytochrome P450 BM3 is represented by SEQ ID NO:2.

5. The nucleic acid molecule according to claim 4, wherein at least one of said at least one further mutations occurs at a position corresponding to position 87, 471, 47, 29, 189, 13, 162, 354, 429, 64 and/or 223 in SEQ ID NO:2.

6. The nucleic acid molecule of claim 5, wherein
phe at a position corresponding to position 87 in SEQ ID NO:2 is replaced by ala, gly, val, leu, ile, met, trp or pro;
arg at a position corresponding to position 471 in SEQ ID NO:2 is replaced by cys, ser, tyr, thr, asn, gln, asp or glu;
arg at a position corresponding to position 47 in SEQ ID NO:2 is replaced by phe, tyr, leu, val, gly, ala, ile, met, trp or pro;
leu at a position corresponding to position 29 in SEQ ID NO:2 is replaced by ser, tyr, thr, cys, asn, gln, asp or glu;
gln at a position corresponding to position 189 in SEQ ID NO:2 is replaced by arg, lys or his; glu at a position corresponding to position 13 in SEQ ID NO:2 is replaced by gly, val, ala, leu, ile, met, phe, trp or pro;
phe at a position corresponding to position 162 in SEQ ID NO:2 is replaced by leu, val, gly, ala, ile, met, trp or pro;
met at a position corresponding to position 354 in SEQ ID NO:2 is replaced by ser, asn, gln, tyr, thr, cys, asp or glu;
tyr at a position corresponding to position 429 in SEQ ID NO:2 is replaced by cys, ser, asn, gln, thr, asp or glu;
glu at a position corresponding to position 64 in SEQ ID NO:2 is replaced by gly, val, ala, leu, ile, met, phe, trp or pro; and/or
arg at a position corresponding to position 223 in SEQ ID NO:2 is replaced by his, lys, phe, tyr or trp.

7. The nucleic acid molecule of claim 6, wherein
phe at a position corresponding to position 87 in SEQ ID NO:2 is replaced by ala;
arg at a position corresponding to position 471 in SEQ ID NO:2 is replaced by cys;
arg at a position corresponding to position 47 in SEQ ID NO:2 is replaced by phe, tyr or leu; leu at a position corresponding to position 29 in SEQ ID NO:2 is replaced by ser;
gln at a position corresponding to position 189 in SEQ ID NO:2 is replaced by arg;
glu at a position corresponding to position 13 in SEQ ID NO:2 is replaced by gly;
phe at a position corresponding to position 162 in SEQ ID NO:2 is replaced by leu;
met at a position corresponding to position 354 in SEQ ID NO:2 is replaced by ser;
tyr at a position corresponding to position 429 in SEQ ID NO:2 is replaced by cys;
glu at a position corresponding to position 64 in SEQ ID NO:2 is replaced by gly; and/or
arg at a position corresponding to position 223 in SEQ ID NO:2 is replaced by his.

8. The nucleic acid molecule according to any one of claims 1 to 7, wherein the polypeptide deviates from the polypeptide of SEQ ID NO:2 by the amino acid substitution pattern F87A, R471C and M812T.

9. A vector comprising the nucleic acid molecule of any one of claims 1 to 8.

10. A host transformed with and comprising the vector of claim 9, wherein the host is selected from the group consisting of bacteria, fungi, yeast, plant cells or animal cells and wherein the host is not a human embryo.

11. A method of producing a polypeptide encoded by the nucleic acid molecule according to any one of claims 1 to 8 comprising culturing the host of claim 10 under suitable conditions and isolating the polypeptide produced.

12. A polypeptide encoded by the nucleic acid molecule according to any one of claims 1 to 8.

13. Use of the polypeptide of claim 12 in synthetic chemical transformation, *in vitro* biotransformation or fine chemical synthesis.

14. A composition comprising the nucleic acid molecule of any one of claims 1 to 8 or the vector of claim 9 or the host of claim 10 or the polypeptide of claim 12.

## Patentansprüche

1. Nukleinsäuremolekül, das ein Polypeptid kodiert, das Zytochrom P450-Monooxygenase-Aktivität hat, wobei das Polypeptid eine Reduktasedomäne umfasst, die sich in mindestens einer und weniger als 70 Mutationen unterscheidet von

(a) der Reduktasedomäne von Zytochrom P450 BM3, wobei die Reduktasedomäne von Zytochrom P450 BM3 in SEQ ID NO:1 dargestellt ist; oder
(b) einer Reduktasedomäne, die mindestens 95% Sequenzidentität zu SEQ ID NO:1 hat; und
wobei mindestens eine der mindestens einen Mutation in der Reduktasedomäne an einer Position in SEQ ID NO:1 vorkommt, die der Position 812 in SEQ ID NO:2 entspricht und wobei die Mutation zu einer erhöhten Zytochrom P450 Monooxygenase-Aktivität im Vergleich zu der Ausgangssequenz führt, die die Reduktasedomäne der SEQ ID NO:1 umfasst,
wobei die Zytochrom P450 Monooxygenase-Aktivität ausgewählt ist aus Monooxygenierung von gesättigten und/oder ungesättigten Fettsäuren, Alkoholen, und/oder Amiden; Umwandlung von langkettigen Alkoholen und/oder Aminen, Umwandlung von Laurinsäuren, Myristinsäuren und/oder Palmitinsäuren in Omega-1-, Omega-2-, und/oder Omega-3-Hydroxyl-Analoga und Hydroxylierung von auf Kumarin basierenden Stoffen.

**2.** Nukleinsäuremolekül nach Anspruch 1, wobei Met an einer Position in SEQ ID NO:1, die der Position 812 in SEQ ID NO:2 entspricht, durch Thr, Ser, Cys, Asn, Gln, Asp oder Glu ersetzt ist.

**3.** Nukleinsäuremolekül nach Anspruch 2, wobei Met an einer Position in SEQ ID NO:1, die der Position 812 in SEQ ID NO:2 entspricht, durch Thr ersetzt ist.

**4.** Nukleinsäuremolekül nach einem der Ansprüche 1 bis 3, wobei das Polypeptid, das Zytochrom P450 Monooxygenase-Aktivität hat, sich in mindestens einer weiteren Mutation von Zytochrom P450 BM3 unterscheidet, wobei Zytochrom P450 BM3 in SEQ ID NO:2 dargestellt ist.

**5.** Nukleinsäuremolekül nach Anspruch 4, wobei mindestens eine der mindestens einen weiteren Mutation an einer Position vorkommt, die der Position 87, 471, 47, 29, 189, 13, 162, 354, 429, 64 und/oder 223 in SEQ ID NO:2 entspricht.

**6.** Nukleinsäuremolekül nach Anspruch 5, wobei

Phe an einer Position, die der Position 87 in SEQ ID NO:2 entspricht, durch Ala, Gly, Val, Leu, Ile, Met, Trp oder Pro ersetzt ist;
Arg an einer Position, die der Position 471 in SEQ ID NO:2 entspricht, durch Cys, Ser, Tyr, Thr, Asn, Gln, Asp oder Glu ersetzt ist;
Arg an einer Position, die der Position 47 in SEQ ID NO:2 entspricht, durch Phe, Tyr, Leu, Val, Gly, Ala, Ile, Met, Trp oder Pro ersetzt ist;
Leu an einer Position, die der Position 29 in SEQ ID NO:2 entspricht, durch Ser, Tyr, Thr, Cys, Asn, Gln, Asp oder Glu ersetzt ist;
Gln an einer Position, die der Position 189 in SEQ ID NO:2 entspricht, durch Arg, Lys oder His ersetzt ist;
Glu an einer Position, die der Position 13 in SEQ ID NO:2 entspricht, durch Gly, Val, Ala, Leu, Ile, Met, Phe, Trp oder Pro ersetzt ist;
Phe an einer Position, die der Position 162 in SEQ ID NO:2 entspricht, durch Leu, Val, Gly, Ala, Ile, Met, Trp oder Pro ersetzt ist;
Met an einer Position, die der Position 354 in SEQ ID NO:2 entspricht, durch Ser, Asn, Gln, Tyr, Thr, Cys, Asp oder Glu ersetzt ist;
Tyr an einer Position, die der Position 429 in SEQ ID NO:2 entspricht, durch Cys, Ser, Asn, Gln, Thr, Asp oder Glu ersetzt ist;
Glu an einer Position, die der Position 64 in SEQ ID NO:2 entspricht, durch Gly, Val, Ala, Leu, Ile, Met, Phe, Trp oder Pro ersetzt ist; und/oder
Arg an einer Position, die der Position 223 in SEQ ID NO:2 entspricht, durch His, Lys, Phe, Tyr oder Trp ersetzt ist.

**7.** Nukleinsäuremolekül nach Anspruch 6, wobei

Phe an einer Position, die der Position 87 in SEQ ID NO:2 entspricht, durch Ala ersetzt ist;
Arg an einer Position, die der Position 471 in SEQ ID NO:2 entspricht, durch Cys ersetzt ist;
Arg an einer Position, die der Position 47 in SEQ ID NO:2 entspricht, durch Phe, Tyr oder Leu ersetzt ist;
Leu an einer Position, die der Position 29 in SEQ ID NO:2 entspricht, durch Ser ersetzt ist;
Gln an einer Position, die der Position 189 in SEQ ID NO:2 entspricht, durch Arg ersetzt ist;

Glu an einer Position, die der Position 13 in SEQ ID NO:2 entspricht, durch Gly ersetzt ist;
Phe an einer Position, die der Position 162 in SEQ ID NO:2 entspricht, durch Leu ersetzt ist;
Met an einer Position, die der Position 354 in SEQ ID NO:2 entspricht, durch Ser ersetzt ist;
Tyr an einer Position, die der Position 429 in SEQ ID NO:2 entspricht, durch Cys ersetzt ist;
Glu an einer Position, die der Position 64 in SEQ ID NO:2 entspricht, durch Gly ersetzt ist; und/oder
Arg an einer Position, die der Position 223 in SEQ ID NO:2 entspricht, durch His ersetzt ist.

**8.** Nukleinsäuremolekül nach einem der Ansprüche 1 bis 7, wobei sich das Polypeptid von dem Polypeptid der SEQ ID NO:2 in dem Aminosäure-Austauschmuster F87A, R471C und M812T unterscheidet.

**9.** Vektor, umfassend das Nukleinsäuremolekül nach einem der Ansprüche 1 bis 8.

**10.** Wirt, der mit dem Vektor nach Anspruch 9 transformiert ist und diesen umfasst, wobei der Wirt ausgewählt ist aus Bakterien, Pilzen, Hefen, Pflanzenzellen und Tierzellen und wobei der Wirt kein menschlicher Embryo ist.

**11.** Verfahren zur Herstellung eines Polypeptids, das durch das Nukleinsäuremolekül nach einem der Ansprüche 1 bis 8 kodiert wird, umfassend Züchten des Wirts nach Anspruch 10 unter geeigneten Bedingungen und Isolieren des hergestellten Polypeptids.

**12.** Polypeptid, das durch das Nukleinsäuremolekül nach einem der Ansprüche 1 bis 8 kodiert wird.

**13.** Verwendung des Polypeptids nach Anspruch 12 in synthetisch-chemischer Transformation, *in vitro* Biotransformation oder Feinchemikaliensynthese.

**14.** Zusammensetzung, umfassend das Nukleinsäuremolekül nach einem der Ansprüche 1 bis 8 oder den Vektor nach Anspruch 9 oder den Wirt nach Anspruch 10 oder das Polypeptid nach Anspruch 12.

## Revendications

**1.** Molécule d'acide nucléique codant pour un polypeptide doté d'une activité cytochrome P450 monooxygénase, ledit polypeptide comprenant un domaine réductase qui diffère par au moins une et par moins de 70 mutations de :

(a) le domaine réductase du cytochrome P450 BM3, le domaine réductase du cytochrome P450 BM3 étant représenté par SEQ ID NO : 1 ; ou
(b) un domaine réductase présentant au moins 95 % d'identité de séquence avec SEQ ID NO : 1 ; et
dans laquelle au moins l'une desdites au moins une mutation dans le domaine réductase survient au niveau d'une position dans SEQ ID NO : 1 correspondant à la position 812 dans SEQ ID NO : 2 et dans laquelle ladite mutation produit une augmentation de l'activité cytochrome P450 monooxygénase comparativement à la séquence parente comprenant le domaine réductase de SEQ ID NO : 1,
dans laquelle ladite activité cytochrome P450 monooxygénase est choisie dans le groupe constitué de la monooxygénation d'acides gras saturés et/ou insaturés, d'alcools, et/ou d'amides ; la conversion d'alcools et/ou d'amines à chaîne longue, la conversion des acides laurique, myristique, et/ou palmitique en analogues hydroxylés oméga-1, oméga-2, et/ou oméga-3 et l'hydroxylation de substrats à base de coumarine.

**2.** Molécule d'acide nucléique selon la revendication 1, dans laquelle met au niveau d'une position dans SEQ ID NO : 1 correspondant à la position 812 dans SEQ ID NO : 2 est remplacé par thr, ser, cys, asn, gln, asp ou glu.

**3.** Molécule d'acide nucléique selon la revendication 2, dans laquelle met au niveau d'une position dans SEQ ID NO : 1 correspondant à la position 812 dans SEQ ID NO : 2 est remplacé par thr.

**4.** Molécule d'acide nucléique selon l'une quelconque des revendications 1 à 3, dans laquelle le polypeptide doté d'une activité cytochrome P450 monooxygénase diffère du cytochrome P450 BM3 par au moins une autre mutation, le cytochrome P450 BM3 étant représenté par SEQ ID NO : 2.

**5.** Molécule d'acide nucléique selon la revendication 4, dans laquelle au moins l'une desdites au moins une autre mutation survient au niveau d'une position correspondant à la position 87, 471, 47, 29, 189, 13, 162, 354, 429, 64 et/ou 223 dans SEQ ID NO : 2.

6. Molécule d'acide nucléique selon la revendication 5, dans laquelle phe au niveau d'une position correspondant à la position 87 dans SEQ ID NO : 2 est remplacé par ala, gly, val, leu, ile, met, trp ou pro ;
arg au niveau d'une position correspondant à la position 471 dans SEQ ID NO : 2 est remplacé par cys, ser, tyr, thr, asn, gln, asp ou glu ; arg au niveau d'une position correspondant à la position 47 dans SEQ ID NO : 2 est remplacé par phe, tyr, leu, val, gly, ala, ile, met, trp ou pro ;
leu au niveau d'une position correspondant à la position 29 dans SEQ ID NO : 2 est remplacé par ser, tyr, thr, cys, asn, gln, asp ou glu ;
gln au niveau d'une position correspondant à la position 189 dans SEQ ID NO : 2 est remplacé par arg, lys ou his ;
glu au niveau d'une position correspondant à la position 13 dans SEQ ID NO : 2 est remplacé par gly, val, ala, leu, ile, met, phe, trp ou pro ; phe au niveau d'une position correspondant à la position 162 dans SEQ ID NO : 2 est remplacé par leu, val, gly, ala, ile, met, trp ou pro ;
met au niveau d'une position correspondant à la position 354 dans SEQ ID NO : 2 est remplacé par ser, asn, gln, tyr, thr, cys, asp ou glu ;
tyr au niveau d'une position correspondant à la position 429 dans SEQ ID NO : 2 est remplacé par cys, ser, asn, gln, thr, asp ou glu ;
glu au niveau d'une position correspondant à la position 64 dans SEQ ID NO : 2 est remplacé par gly, val, ala, leu, ile, met, phe, trp ou pro ; et/ou
arg au niveau d'une position correspondant à la position 223 dans SEQ ID NO : 2 est remplacé par his, lys, phe, tyr ou trp.

7. Molécule d'acide nucléique selon la revendication 6, dans laquelle phe au niveau d'une position correspondant à la position 87 dans SEQ ID NO : 2 est remplacé par ala ;
arg au niveau d'une position correspondant à la position 471 dans SEQ ID NO : 2 est remplacé par cys ;
arg au niveau d'une position correspondant à la position 47 dans SEQ ID NO : 2 est remplacé par phe, tyr ou leu ;
leu au niveau d'une position correspondant à la position 29 dans SEQ ID NO : 2 est remplacé par ser ;
gln au niveau d'une position correspondant à la position 189 dans SEQ ID NO : 2 est remplacé par arg ;
glu au niveau d'une position correspondant à la position 13 dans SEQ ID NO : 2 est remplacé par gly ;
phe au niveau d'une position correspondant à la position 162 dans SEQ ID NO : 2 est remplacé par leu ;
met au niveau d'une position correspondant à la position 354 dans SEQ ID NO : 2 est remplacé par ser ;
tyr au niveau d'une position correspondant à la position 429 dans SEQ ID NO : 2 est remplacé par cys ;
glu au niveau d'une position correspondant à la position 64 dans SEQ ID NO : 2 est remplacé par gly ; et/ou
arg au niveau d'une position correspondant à la position 223 dans SEQ ID NO : 2 est remplacé par his.

8. Molécule d'acide nucléique selon l'une quelconque des revendications 1 à 7, dans laquelle le polypeptide diffère du polypeptide de SEQ ID NO : 2 par le profil de substitution d'acide aminé F87A, R471C et M812T.

9. Vecteur comprenant la molécule d'acide nucléique selon l'une quelconque des revendications 1 à 8.

10. Hôte transformé avec et comprenant le vecteur selon la revendication 9, l'hôte étant choisi dans le groupe constitué de bactéries, de champignons, de levures, de cellules végétales ou de cellules animales et l'hôte n'étant pas un embryon humain.

11. Méthode de production d'un polypeptide codé par la molécule d'acide nucléique selon l'une quelconque des revendications 1 à 8 comprenant la culture de l'hôte selon la revendication 10 dans des conditions appropriées et l'isolement du polypeptide produit.

12. Polypeptide codé par la molécule d'acide nucléique selon l'une quelconque des revendications 1 à 8.

13. Utilisation du polypeptide selon la revendication 12 dans une transformation chimique synthétique, une biotransformation *in vitro* ou une synthèse chimique fine.

14. Composition comprenant la molécule d'acide nucléique selon l'une quelconque des revendications 1 à 8 ou le vecteur selon la revendication 9 ou l'hôte selon la revendication 10 ou le polypeptide selon la revendication 12.

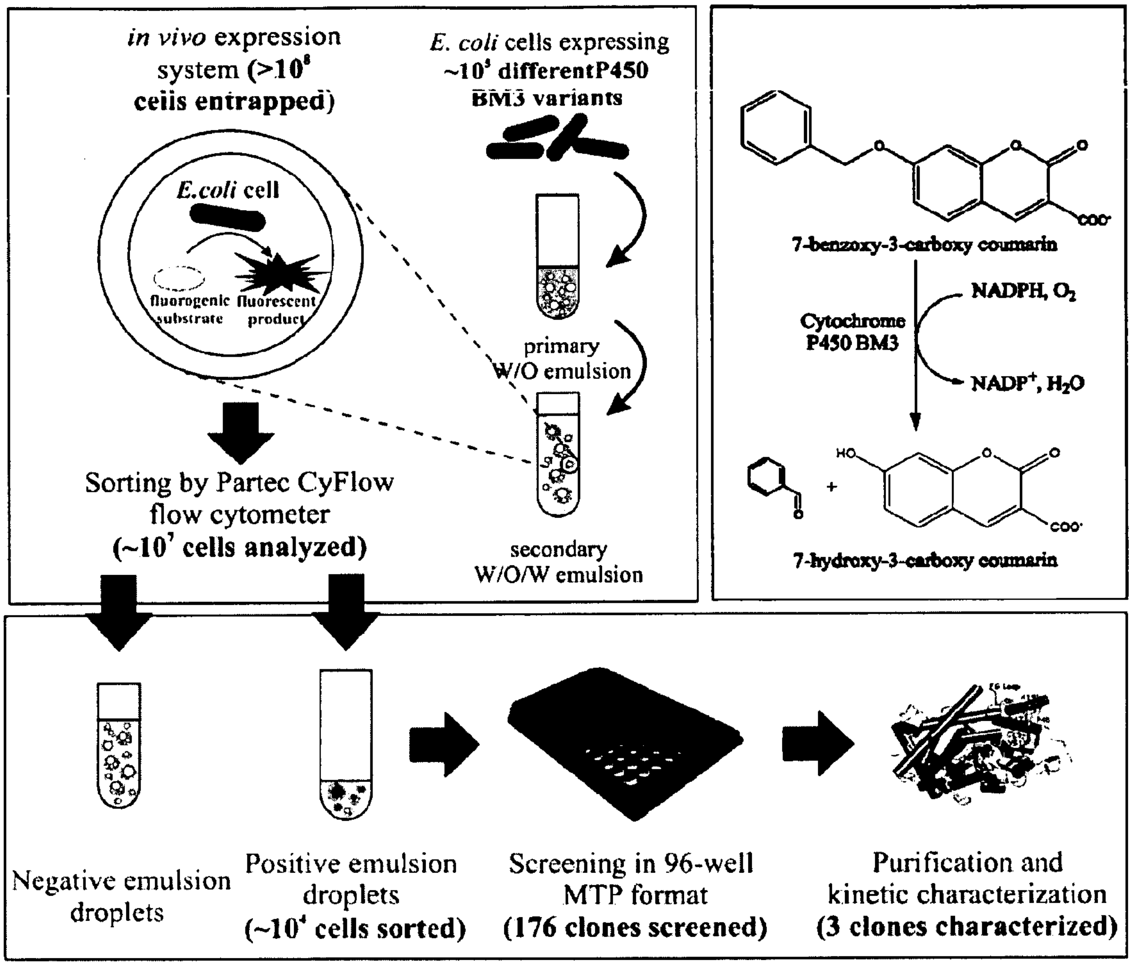


**Figure 1**

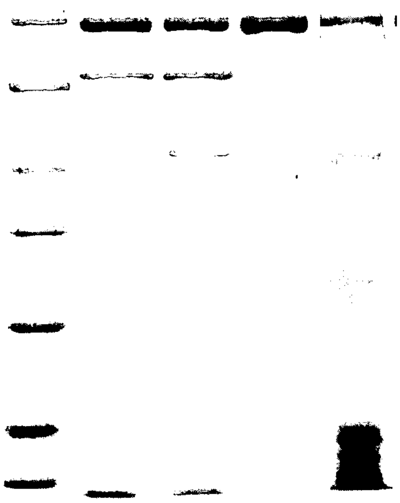

**Figure 2**

A)

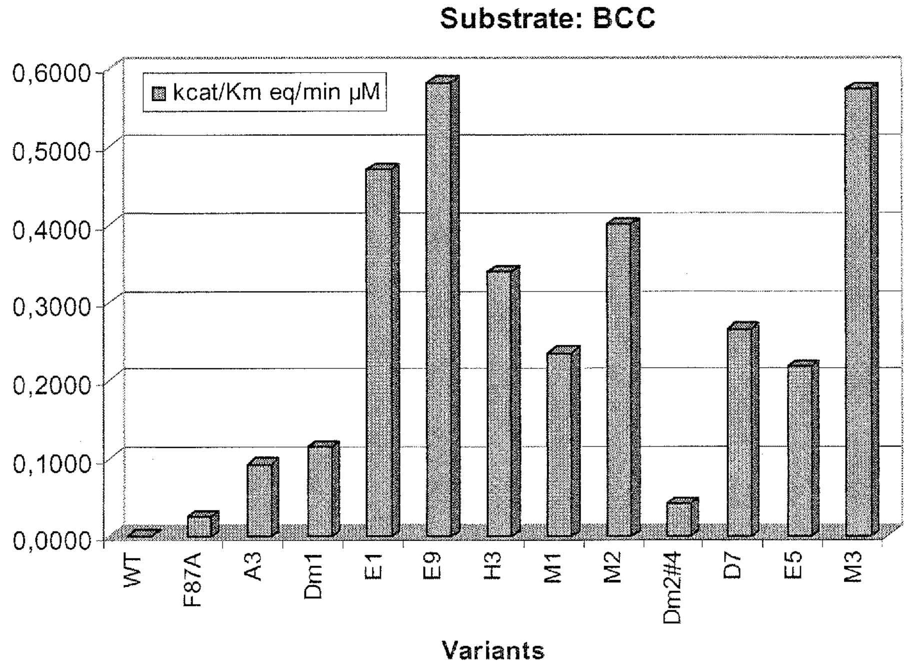
Substrate: BCC
kcat/Km eq/min µM
0,6000
0,5000
0,4000
0,3000
0,2000
0,1000
0,0000
WT
F87A
A3
Dm1
E1
E9
H3
M1
M2
Dm2#4
D7
E5
M3
Variants

B)

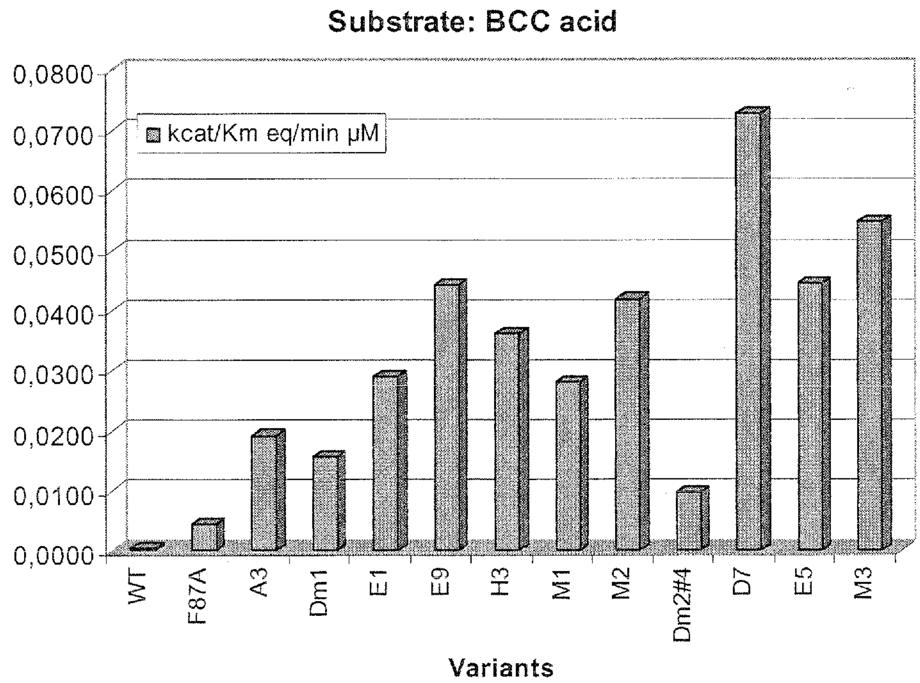
Substrate: BCC acid
kcat/Km eq/min µM
0,0800
0,0700
0,0600
0,0500
0,0400
0,0300
0,0200
0,0100
0,0000
WT
F87A
A3
Dm1
E1
E9
H3
M1
M2
Dm2#4
D7
E5
M3
Variants

Figure 3

C)

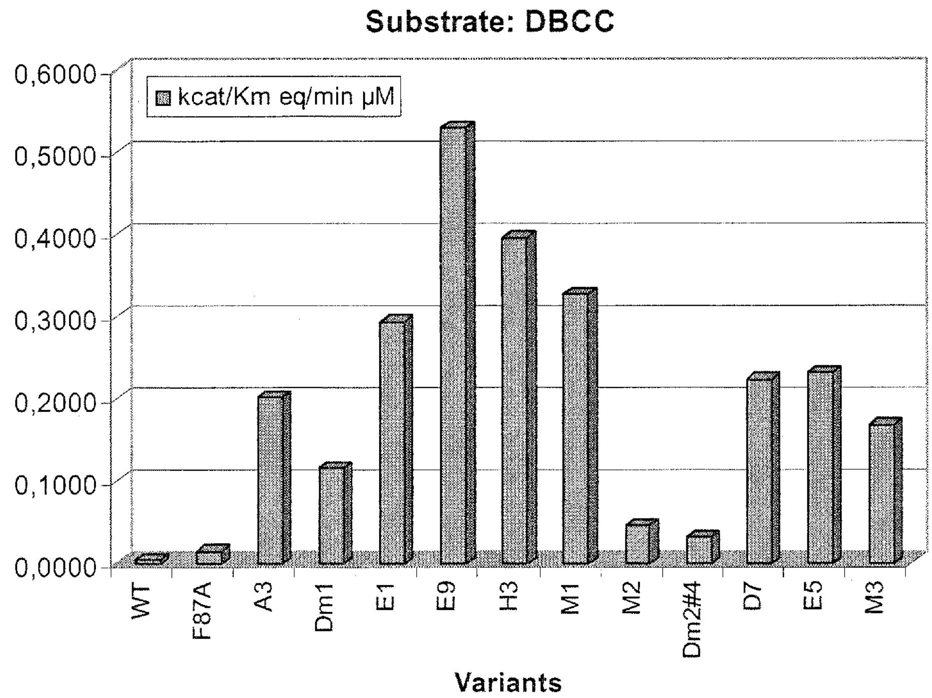


Figure 3 continued

```
TIKEMPQPKT FGELKNLPLL NTDKPVQALM KIADELGEIF KFEAPGRVTR YLSSQRLIKE ACDESRFDKN    70

LSQALKFVRD FAGDGLFTSW THEKNWKKAH NILLPSFSQQ AMKGYHAMMV DIAVQLVQKW ERLNADEHIE   140

VPEDMTRLTL DTIGLCGFNY RFNSFYRDQP HPFITSMVRA LDEAMNKLQR ANPDDPAYDE NKRQFQEDIK   210

VMNDLVDKII ADRKASGEQS DDLLTHMLNG KDPETGEPLD DENIRYQIIT FLIAGHETTS GLLSFALYFL   280

VKNPHVLQKA AEEAARVLVD PVPSYKQVKQ LKYVGMVLNE ALRLWPTAPA FSLYAKEDTV LGGEYPLEKG   350

DELMVLIPQL HRDKTIWGDD VEEFRPERFE NPSAIPQHAF KPFGNGQRAC IGQQFALHEA TLVLGMMLKH   420

FDFEDHTNYE LDIKETLTLK PEGFVVKAKS KKIPLGGIPS PSTEQSAKKV RKKAENAHNT PLLVLYGSNM   490
                                                        KKAENAHNT PLLVLYGSNM

GTAEGTARDL ADIAMSKGFA PQVATLDSHA GNLPREGAVL IVTASYNGHP PDNAKQFVDW LDQASADEVK   560
GTAEGTARDL ADIAMSKGFA PQVATLDSHA GNLPREGAVL IVTASYNGHP PDNAKQFVDW LDQASADEVK
                       R
GVRYSVFGCG DKNWATTYQK VPAFIDETLA AKGAENIADR GEADASDDFE GTYEEWREHM WSDVAAYFNL   630
GVRYSVFGCG DKNWATTYQK VPAFIDETLA AKGAENIADR GEADASDDFE GTYEEWREHM WSDVAAYFNL

DIENSEDNKS TLSLQFVDSA ADMPLAKMHG AFSTNVVASK ELQQPGSARS TRHLEIELPK EASYQEGDHL   700
DIENSEDNKS TLSLQFVDSA ADMPLAKMHG AFSTNVVASK ELQQPGSARS TRHLEIELPK EASYQEGDHL
                  G
GVIPRNYEGI VNRVTARFGL DASQQIRLEA EEEKLAHLPL AKTVSVEELL QYVELQDPVT RTQLRAMAAK   770
GVIPRNYEGI VNRVTARFGL DASQQIRLEA EEEKLAHLPL AKTVSVEELL QYVELQDPVT RTQLRAMAAK
                                V
TVCPPHKVEL EALLEKQAYK EQVLAKRLTM LELLEKYPAC EMKFSEFIAL LPSIRPRYYS ISSSPRVDEK   840
TVCPPHKVEL EALLEKQAYK EQVLAKRLTM LELLEKYPAC EMKFSEFIAL LPSIRPRYYS ISSSPRVDEK
                             S             T
QASITVSVVS GEAWSGYGEY KGIASNYLAE LQEGDTITCF ISTPQSEFTL PKDPETPLIM VGPGTGVAPF   910
QASITVSVVS GEAWSGYGEY KGIASNYLAE LQEGDTITCF ISTPQSEFTL PKDPETPLIM VGPGTGVAPF
                  N                           HR
RGFVQARKQL KEQGQSLGEA HLYFGCRSPH EDYLYQEELE NAQSEGIITL HTAFSRMPNQ PKTYVQHVME   980
RGFVQARKQL KEQGQSLGEA HLYFGCRSPH EDYLYQEELE NAQSEGIITL HTAFSRMPNQ PKTYVQHVME
                                            D
QDGKKLIELL DQGAHFYICG DGSQMAPAVE ATLMKSYADV HQVSEADARL WLQQLEEKGR YAKDVWAG   1048
QDGKKLIELL DQGAHFYICG DGSQMAPAVE ATLMKSYADV HQVSEADARL WLQQLEEKGR YAKDVWAG
```

**Figure 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7691616 B
- US 776768 A
- US 7704715 B
- EP 1196603 B1
- EP 1196545 B1
- EP 1196605 B1
- US 7531335 B
- US 7524664 B
- US 7226768 B
- US 20090264311 A1
- WO 2003008563 A2
- WO 2008115844 A2
- WO 2003008563 A
- WO 2007129050 A
- US 20090186415 A1
- WO 03014341 A
- WO 2001042455 A
- WO 0177368 A1
- EP 10009185 A


### Non-patent literature cited in the description


- **PORTER, T.D. ; COON, M.J.** Cytochrome P450: multiplicity of isoforms, substrates, and catalytic and regulatory mechanisms. *J. Biol. Chem.,* 1991, vol. 266, 13469-13472
- **BODDUPALLI, S. S. et al.** Fatty acid monooxygenation by cytochrome P-450 BM-3. *J. Biol. Chem.,* 1990, vol. 265, 4233-4239
- **RUETTINGER et al.** Coding nucleotide, 5'-Regulatory, and Deduced Amino Acid Sequences of P-450 BM-3, a Single Peptide Cytochrome P-450:NADPH-P-450 reductase from Bacillus megaterium. *J. Biol. Chem.,* 1989, vol. 264 (19), 10987-10995
- **RAVICHANDRAN, K.G. ; BODDUPALLI, S.S. ; HASERMANN, C.A. ; PETERSON, J.A. ; DEISENHOFER, J.** Crystal structure of hemoprotein domain of P450 BM-3, a prototype for microsomal P450's. *Science,* 1993, vol. 261, 731-736
- **SEVRIOUKOVA, I.F. ; LI, H. ; ZHANG, H. ; PETERSON, J.A. ; POULOS, T.L.** Structure of a cytochrome P450-redox partner electron-transfer complex. *Proc Natl Acad Sci USA,* 1999, vol. 96, 1863-1868
- **WONG, T.S. ; ARNOLD, F.H. ; SCHWANEBERG, U.** Laboratory evolution of cytochrome P450 BM-3 monooxygenase for organic co-solvents. *Biotechnology and Bioengineering 85,* 2004, vol. 3, 351-358
- **NOBLE, M.A. ; MILES, C.S. ; CHAPMAN, S.K. ; LYSEK, D.A. ; MACKAY, A.C. ; REID, G.A. ; HANZLIK, R.P. ; MUNRO, A.W.** Roles of key active-site residues in flavocytochrome P450 BM3. *Biochem J.,* 1999, vol. 339, 371-379
- **WHITEHOUSE et al.** A highly active single-mutation variant of P450 BM3 (CYP102A1). *Chem. Biochem.,* 2009, vol. 10 (10), 1654-6
- **GIRVAN, HM.** Novel haem co-ordination variants of flavocytochrome P450 BM3. *Biochem. J.,* 2009, vol. 417 (1), 65-76
- **BRAASCH ; COREY.** *Chem Biol,* 2001, vol. 8, 1
- **SCHWANEBERG, U. ; SCHMIDT-DANNERT, C. ; SCHMITT, J. ; SCHMID, R.D.** A continuous spectrophotometric assay for P450 BM-3, a fatty acid hydroxylating enzyme and its mutant F87A. *Anal. Biochem,* 1999, vol. 269, 359-366
- **LI, Q.S. ; SCHWANEBERG, U. ; FISCHER, P. ; SCHMID, R.D.** Directed evolution of the fatty-acid hydroxylase P450 BM-3 into an indole-hydroxylating catalyst. *Chemistry,* 2000, vol. 6, 1531-1536
- **MIYAZAKI, K. ; ARNOLD, F.H.** Exploring nonnatural evolutionary pathways by saturation mutagenesis: rapid improvement of protein function. *J. Mol. Evol.,* 1999, vol. 49, 716-720
- **WONG TS ; TEE KL ; HAUER B ; SCHWANEBERG U.** Sequence saturation mutagenesis (SeSaM): a novel method for directed evolution. *Nucleic Acids Res. 10,* 2004, vol. 32 (3), e26
- **CHEN, K. ; ARNOLD, F.H.** Tuning the activity of an enzyme for unusual environments: sequential ramdom mutagenesis of subtilisin E for catalysis in dimethylformamide. *Proc. Natl. Acad. Sci. USA,* 1993, vol. 90, 5618-5622
- **MCCULLUM E.O. ; WILLIAMS B.A. ; ZHANG J. ; CHAPUT J.C.** Random mutagenesis by error-prone PCR. *Methods Mol Biol.,* 2010, vol. 634, 103-9
- **SACHIS et al.** Improved PCR method for the creation of saturation mutagenesis libraries in directed evolution: application to difficult-to-amplify templates. *Appl. Microbiol. Biotechnol.,* 2008, vol. 81 (2), 387-97

- **STEMMER, W.** Rapid evolution of a protein in vitro by DNA shuffling. *Nature,* 1994, vol. 370 (6488), 389-391
- **TEE KL ; SCHWANEBERG U.** Directed evolution of oxygenases: screening systems, success stories and challenges. *Comb Chem High Throughput Screen,* 2007, vol. 10, 197-217
- **MILLER, O.J. et al.** Directed evolution by in vitro compartmentalization. *Nat Methods,* 2006, vol. 3 (7), 561-70
- **STEPHEN F. ALTSCHUL ; THOMAS L. MADDEN ; ALEJANDRO A. SCHÄFFER ; JINGHUI ZHANG ; ZHENG ZHANG ; WEBB MILLER ; DAVID J. LIPMAN.** Gapped BLAST and PSI-BLAST: a new generation of protein database search programs. *Nucleic Acids Res.,* 1997, vol. 25, 3389-3402
- *Thompson Nucl. Acids Res.,* 1994, vol. 2, 4673-4680
- **PEARSON ; LIPMAN.** *Proc. Natl. Acad. Sci.,* 1988, vol. 85, 2444
- **HENIKOFF.** *Proc. Natl. Acad. Sci.,* 1989, vol. 89, 10915
- **GUENGERICH, F.P.** rate-limiting steps in Cytochrome P450 catalysis. *Biol. Chem.,* vol. 383, 1553-1564
- **GUENGERICH, F.P.** rate-limiting steps in cytochrome P450 catalysis. *Biol. Chem.,* 2002, vol. 383, 1553-1564
- **BERHARDT, R.** Cytochromes P450 as versatile biocatalysts. *J. Biotechnol.,* 2006, vol. 124, 129-145
- **T. MANIATIS ; E. F. FRITSCH ; J. SAMBROOK.** Molecular Cloning: A Laboratory Manual. Cold Spring Harbor Laboratory, Cold Spring Harbor, 1989
- **T. J. SILHAVY ; M. L. BERMAN ; L. W. ENQUIST.** Experiments with Gene Fusions. Cold Spring Harbor Laboratory, Cold Spring Harbor, 1984
- **AUSUBEL, F. M. et al.** Current Protocols in Molecular Biology. Greene Publishing Assoc. and Wiley Interscience, 1987
- Cloning Vectors. Elsevier, 1985
- Current Protocols in Molecular Biology. Wiley Interscience, 1997
- **T. MANIATIS ; E. F. FRITSCH ; J. SAMBROOK.** Molecular Cloning: A Laboratory Manual. Cold Spring Harbor Laboratory, 1989
- **COOPER, F. G.** Biochemische Arbeitsmethoden [Methods in Biochemistry. Verlag Walter de Gruyter
- **SCOPES, R.** Protein Purification. Springer Verlag
- **HARLOW, E. ; LANE, D.** Antibodies: A Laboratory Manual. Cold Spring Harbor (N.Y.) Press, 1988
- **SAMBROOK ; RUSSELL.** Molecular Cloning, A Laboratory Manual. Cold Spring Harbor Laboratory, 2001
- **AUSUBEL.** Current Protocols in Molecular Biology. Green Publishing Associates and Wiley Interscience, 1989
- Nucleic acid hybridization, a practical approach. IRL Press Oxford, 1985
- **SAMBROOK et al.** Molecular Cloning, A Laboratory Manual. CSH Press, Cold Spring Harbor, 1989
- **SAMBROOK ; RUSSE.** Molecular Cloning, A Laboratory Manual. Cold Spring Harbor Laboratory, 2001
- **CADWELL, R.C. ; JOYCE, G.F.** Randomization of genes by PCR mutagenesis. *PCR Methods Appl,* 1992, vol. 2, 28-33
- **MILLER, O.J. ; BERNATH, K. ; AGRESTI, J.J. ; AMITAI, G. ; KELLY, B.T. ; MASTROBATTISTA, E. ; TALY, V. ; MAGDASSI, S. ; TAWFIK, D.S. ; GRIFFITHS, A.D.** Directed evolution by in vitro compartmentalization. *Nat Methods,* 2006, vol. 3, 561-570
- **OMURA, T. ; SATO, R.** The Carbon Monoxide-Binding Pigment Of Liver Microsomes. I. Evidence For Its Hemoprotein Nature. *J Biol Chem,* 1964, vol. 239, 2370-2378
- **WONG, T.S. ; ARNOLD, F.H. ; SCHWANEBERG, U.** Laboratory evolution of cytochrome P450 BM-3 monooxygenase for organic cosolvents. *Biotechnology and Bioengineering,* 2004, vol. 85 (3), 351-358
- **NAZOR, J. ; DANNENMANN, S. ; ADJEI, R.O. ; FORDJOUR, Y.B. ; GHAMPSON, I.T. ; BLANUSA, M. ; ROCCATANO, D. ; SCHWANEBERG, U.** Laboratory evolution of P450 BM3 for mediated electron transfer yielding an activity-improved and reductase-independent variant. *Protein Eng Des Sel,* 2008, vol. 21, 29-35
- **SCHWANEBERG, U. ; SCHMIDT-DANNERT, C. ; SCHMITT, J. ; SCHMID, R.D.** A continuous spectrophotometric assay for P450 BM-3, a fatty acid hydroxylating enzyme and its mutant F87A. *Anal. Biochem.,* 1999, vol. 269, 359-366